# EUROPEAN PATENT SPECIFICATION

(11) **EP 1 140 291 B1**
(45) Date of publication and mention of the grant of the patent: **23.11.2005**
(21) Application number: 99966523.5
(22) Date of filing: 21.12.1999
(51) Int. Cl.: A61P 35/00, A61K 31/335, A61K 31/35, A61K 31/475

(54) **COMBINATION CHEMOTHERAPY COMPRISING A MITOTIC INHIBITOR AND A MEK INHIBITOR**
CHEMOTHERIAPIE MIT EINEM ANTIMITOTISCHEN MITTEL UND EINEM MEK INHIBITOR
CHIMIOTHERAPIE UTILISANT UN INHIBITEUR DE LA MITOSE AVEC UN INHIBITEUR MEK

(30) Priority: 22.12.1998 US 113291 P; 10.11.1999 US 164788 P
(43) Date of publication of application: 10.10.2001
(73) Proprietor: Warner-Lambert Company LLC, Morris Plains, NJ 07950 (US)
(72) Inventor: GOWAN, Richard, Carleton, Brighton, MI 48114 (US); SEBOLT-LEOPOLD, Judith, Ann Arbor, MI 48103 (US)
(74) Representative: Tesch, Rudolf
(86) International application number: PCT/US1999/030485
(87) International publication number: WO 2000/037141

(56) References cited:
- WO-A-95/19970
- WO-A-97/32604
- WO-A-98/37881
- WO-A-98/42830
- US-A- 5 959 097
- US-A- 6 002 008
- US-A- 6 040 321
- WANG ET AL: "Effect of bryostatin 1 on taxol-induced apoptosis and cytotoxicity in human leukemia cells (U937)" BIOCHEMICAL PHARMACOLOGY, vol. 56, no. 5, 1998, pages 635-644, XP000909271
- LIEU ET AL: "Role of mitogen-activated protein kinase in Taxol-induced apoptosis in human leukemic U937 cells" CELL GROWTH & DIFFERENTIATION, vol. 9, no. 9, September 1998 (1998-09), pages 767-776, XP000909267
- DE SOUZA ET AL: "Enhancement of paclitaxel activity against hormone-refractory prostate cancer cells in vitro and in vivo by quinacrine" BRITISH JOURNAL OF CANCER, vol. 75, no. 11, June 1997 (1997-06), pages 1593-1600, XP000909266

## Description

### FIELD OF THE INVENTION

This invention relates to a treatment for cancer in a patient in need of such treatment, comprising the step of administering to the patient a mitotic inhibitor and the step of administering to the patient a MEK inhibitor. The invention also relates to compositions or packaged units comprising a mitotic inhibitor and a MEK inhibitor.

### BACKGROUND OF THE INVENTION

Cancer chemotherapy can entail the use of a combination of agents, generally as a means to reduce the toxic effects of the individual agents when used alone, and in some instances because the combination has greater efficacy than when either agent is used alone.

Mitotic inhibitors are antineoplastic agents that adversely affect the microtubular network in cells that is essential for mitotic and interphase cellular function. Mitotic inhibitors generally bind to free tubulin in cells, promoting the assembly of tubulin into stable microtubules, and simultaneously inhibiting their disassembly. Thus stabilized, microtubules cannot function normally, which in turn results in the inhibition of interphase and mitotic functions in the cell.

Several mitotic inhibitors are now used clinically to treat a variety of cancers. For example, paclitaxel, a natural product, is an antimicrotubule agent that not only promotes the assembly of microtubules from tubulin dimers but also stabilizes microtubules by preventing depolymerization. In addition, paclitaxel induces abnormal arrays or bundles of microtubules throughout the cell cycle and multiple asters of microtubules during mitosis. Paclitaxel is indicated primarily for ovarian carcinoma and breast cancer, although it is useful in treating other cancers such as lung cancer. Use of paclitaxel is generally accompanied by undesirable side effects, including hypersensitivity reactions, hypotension, bradycardia, hypertension, nausea and vomiting, and injection-site reactions. Docetaxel, another mitotic inhibitor, acts much like paclitaxel in its ability to bind to microtubules. Other mitotic inhibitors include the vinca alkaloids, such as vinblastine, vincristine and vinorelbine, as well as derivatives of such compounds such as vinflunine.

MEK inhibitors are compounds which inhibit one or more of the family of mammalian enzymes known as MAP kinase kinases, which phosphorylate the MAP kinase subfamily of enzymes (mitogen-associated protein kinase enzymes) referred to as MAP kinases or ERKs (extracellular signal-regulating enzymes such as ERK1 and ERK 2). These enzymes regulate phosphorylation of other enzymes and proteins within the mammalian body. MEK 1 and MEK 2, as well as ERK1 and ERK 2, are dual specificity kinases that are present in all cell types and play a critical role in the regulation of cell proliferation and differentiation in response to mitogens and a wide variety of growth factors and cytokines. Upon activation, these enzymes control a cascade that can phosphorylate a large number of substrates, including transcription factors, the EGF receptor, phospholipase A2, tyrosine hydroxylase, and cytoskeletal proteins. One selective MEK inhibitor has been shown to be useful to treat a number of proliferative disorders, including psoriasis, restenosis, and cancer, as described in US Patent No. 5,525,625. A whole series of MEK inhibitors have been described as useful to prevent and treat septic shock, see WO 98/37881.

The prior art fails to teach or suggest that any such selective MEK inhibitors can be combined with mitotic inhibitors according to this invention.

### SUMMARY OF THE INVENTION

This invention features a treatment for a proliferative disease, including (a) the step of administering to a patient in need of such treatment a MEK inhibitor and (b) the step of administering to said patient a mitotic inhibitor, wherein the amount of the MEK inhibitor and the amount of the mitotic inhibitor are such that the combination of the agents is an effective antiproliferative therapy. The administration of a mitotic inhibitor may be before, during, or after the administration of the MEK inhibitor. Simultaneous administration may be by the same (both actives by either local or systemic injection) or different routes (e.g., oral administration of a MEK inhibitor and intravenous administration of the mitotic inhibitor). The invention also encompasses the use of additional pharmaceutical agents, such as a second MEK inhibitor, an inhibitor of farnesyl transferase (a ras inhibitor), a RAF inhibitor, a second mitotic inhibitor, an anti-angiogenesis agent, a steroid, or other anti-cancer agents, as well as adjuvants, enhancers, or other pharmaceutically active and pharmaceutically acceptable materials. Therefore, the invention provides a treatment for cancer by administering at least one (e.g., one, two, or three) MEK inhibitors and at least one (e.g., one or two) mitotic inhibitors to the patient. In one aspect, the amounts of each active may vary independently from each other over time. For example, a patient may receive a first MEK inhibitor with a mitotic agent for a period of time, and then the first MEK inhibitor may be replaced by a second MEK inhibitor.

The invention also features compositions, packaged units, and kits which include at least one MEK inhibitor and at least one mitotic inhibitor. For example, the invention encompasses: (a) a single formulation (whether tablet, solution, or suspension, for example) that includes both a mitotic inhibitor and a MEK inhibitor; (b) a blister pack containing separate formulations of each active, such as a tablet or capsule form of a MEK inhibitor and a capsule or ampoule of a solution of a mitotic inhibitor; and (c) a kit with separate formulations of each active packaged together in a box with instructions for combination administration.

Selective MEK 1 or MEK 2 inhibitors are those compounds which inhibit the MEK 1 or MEK 2 enzymes without substantially inhibiting other enzymes such as MKK3, ERK, PKC, Cdk2A, phosphorylase kinase, EGF and PDGF receptor kinases, and C-src. In general, a selective MEK 1 or MEK 2 inhibitor has an IC₅₀ for MEK 1 or MEK 2 that is at least one-fiftieth (1/50) that of its IC₅₀ for one of the above-named other enzymes. Preferably, a selective inhibitor has an IC₅₀ that is at least 1/100, more preferably 1/500, and even more preferably 1/1000, 1/5000 or less than that of its IC₅₀ for one or more of the above-named enzymes.

In a preferred embodiment, the combination to be used according to this invention comprises the mitotic inhibitor paclitaxel. In another preferred embodiment, the mitotic inhibitor administered is selected from paclitaxel, docetaxel, vincristine, vinblastine, vinorelbine, and vinflunine.

According to the invention, the mitotic inhibitor is administered in combination with a selective MEK inhibitor which is a phenyl amine derivative of Formula I.

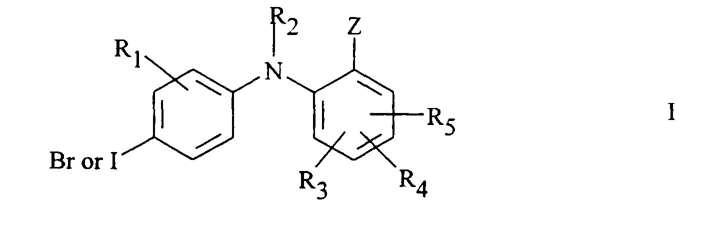

In Formula (I), R₁ is hydrogen, hydroxy, C₁-C₈ alkyl, C₁-C₈ alkoxy, halo, trifluoromethyl, or CN. R₂ is hydrogen. R₃, R₄, and R₅ are independently selected from hydrogen, hydroxy, halo, trifluoromethyl, C₁-C₈ alkyl, C₁-C₈ alkoxy, nitro, CN, and -(O or NH)ₘ-(CH₂)ₙ-R₉. R₉ is hydrogen, hydroxy, COOH, or NR₁₀R₁₁; n is 0-4; m is 0 or 1.
Each of R₁₀ and R₁₁ is independently selected from hydrogen and C₁-C₈ alkyl, or taken together with the nitrogen to which they are attached can complete a 3-10 member cyclic ring optionally containing 1, 2, or 3 additional heteroatoms selected from O, S, NH, or N-(C₁-C₈ alkyl). Z is COOR₇, tetrazolyl, CONR₆R₇, CONHNR₁₀R₁₁, or CH₂OR₇. R₆ and R₇ independently are hydrogen, C₁-C₈ alkyl, C₂-C₈ alkenyl, C₂-C₈ alkynyl, (CO)-C₁-C₈ alkyl, aryl, heteroaryl, C₃-C₁₀ cycloalkyl, or C₃-C₁₀ (cycloalkyl optionally containing one, two, or three heteroatoms selected from O, S, NH, or N alkyl); or R₆ and R₇ together with the nitrogen to which they are attached complete a 3-10 member cyclic ring optionally containing 1, 2, or 3 additional heteroatoms selected from O, S, NH, or N alkyl. In formula (I), any of the foregoing alkyl, alkenyl, aryl, heteroaryl, heterocyclic, and alkynyl groups can be unsubstituted or substituted by halo, hydroxy, C₁-C₆ alkoxy, amino, nitro, C₁-C₄ alkylamino, di(C₁-C₄)alkylamino, C₃-C₆ cycloalkyl, phenyl, phenoxy, C₃-C₅ heteroaryl or heterocyclic radical, or C₃-C₅ heteroaryloxy or heterocyclic radical-oxy. The invention also provides a pharmaceutically acceptable salt of each of the disclosed MEK inhibitors.

In a more preferred embodiment, the MEK inhibitor is selected from a compound in Formula (I) Compound Table below.

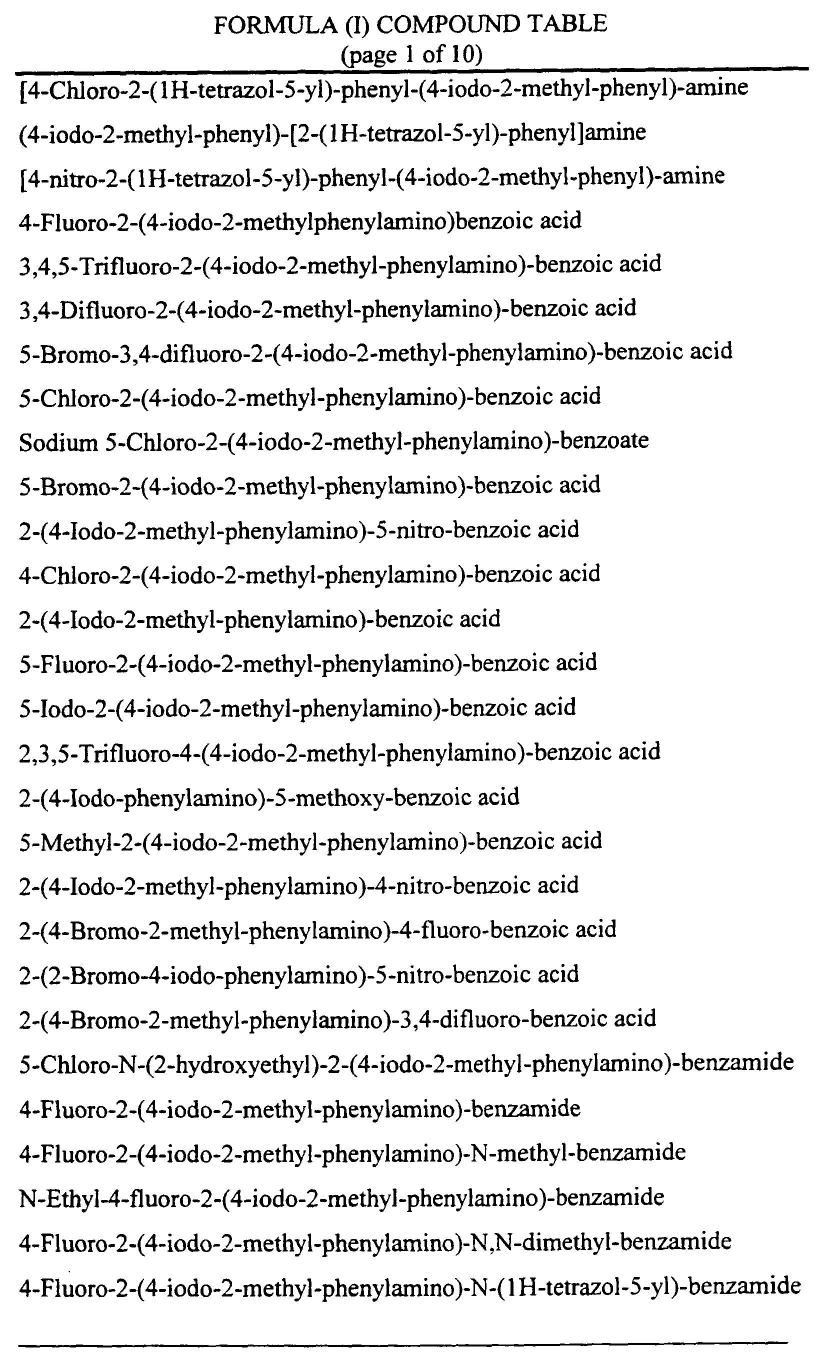

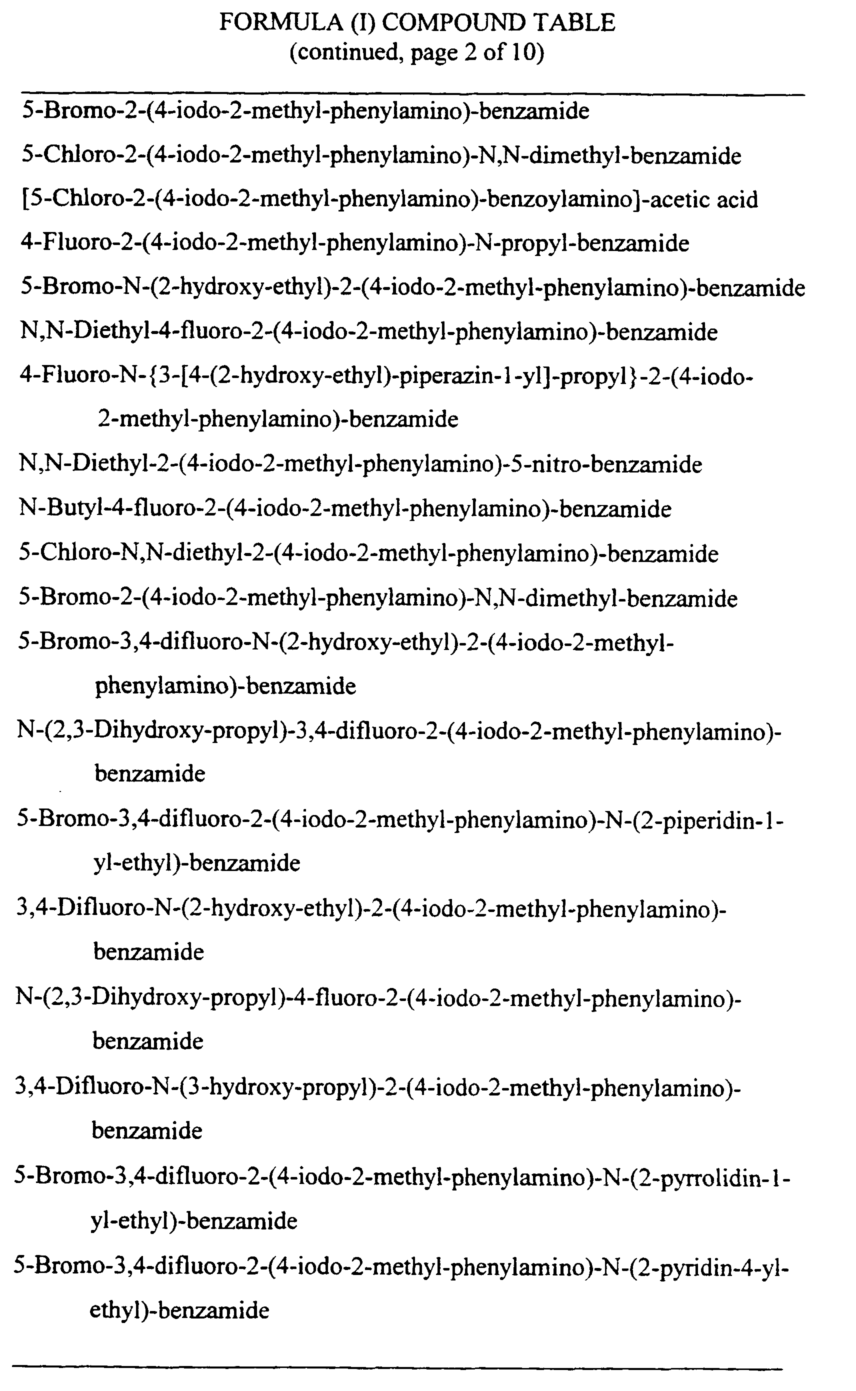

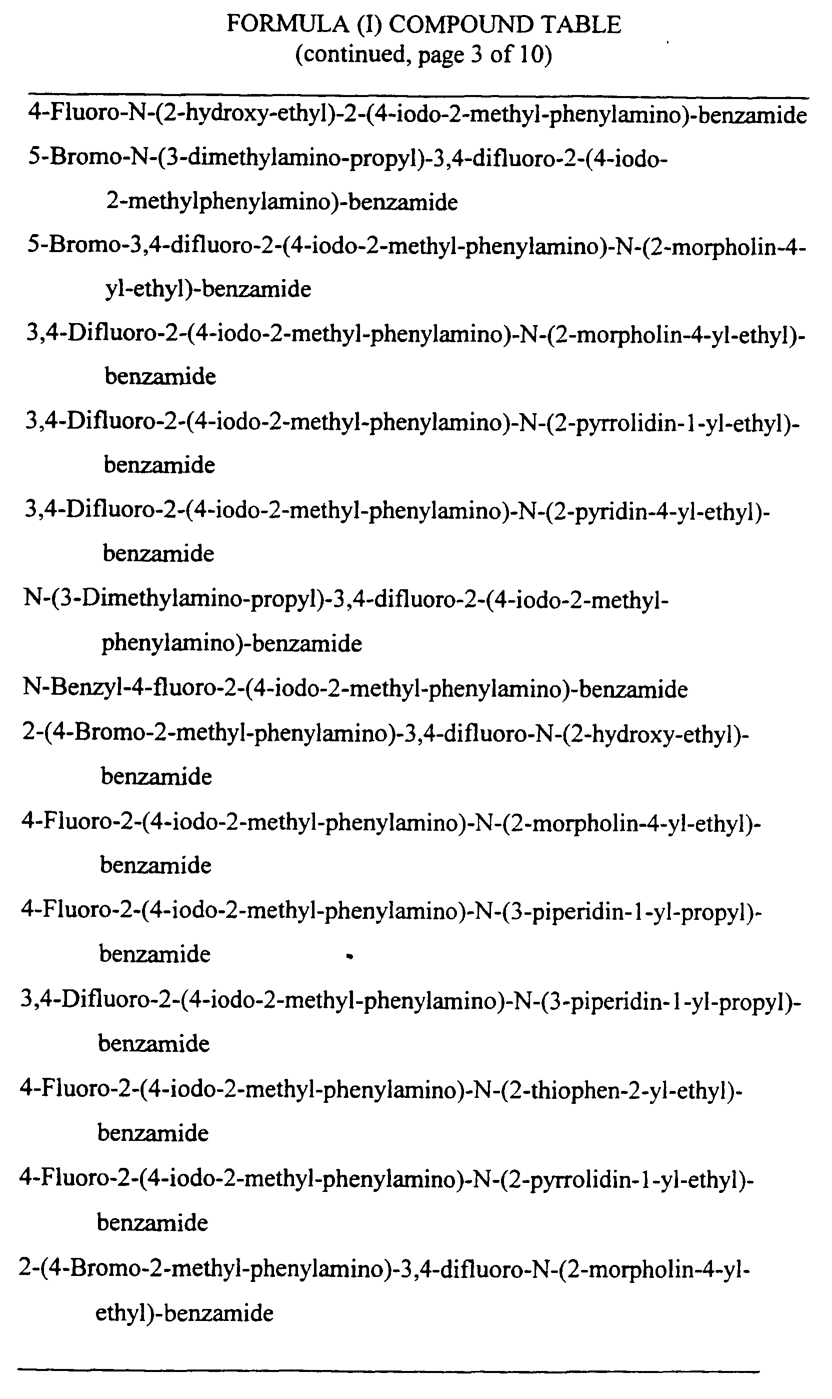

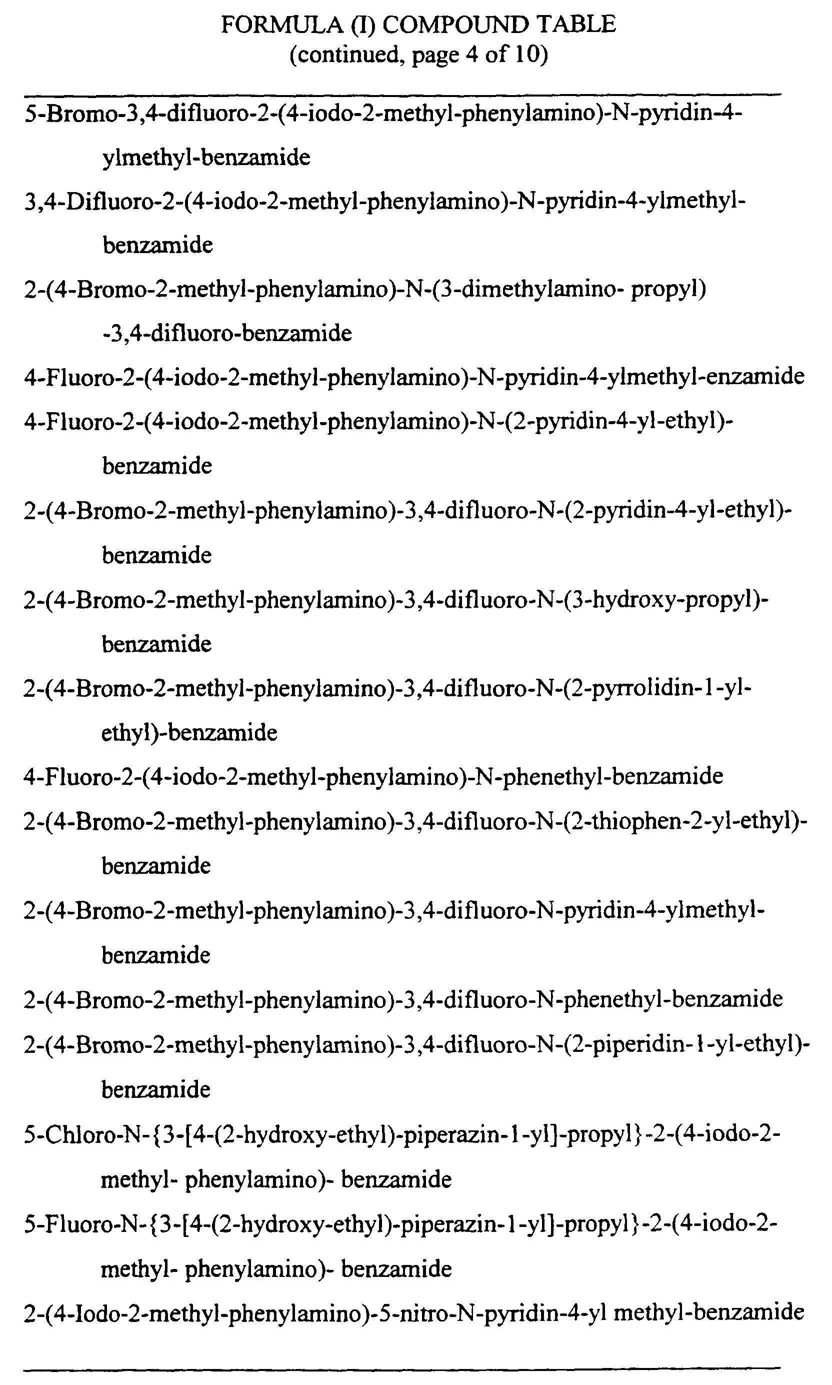

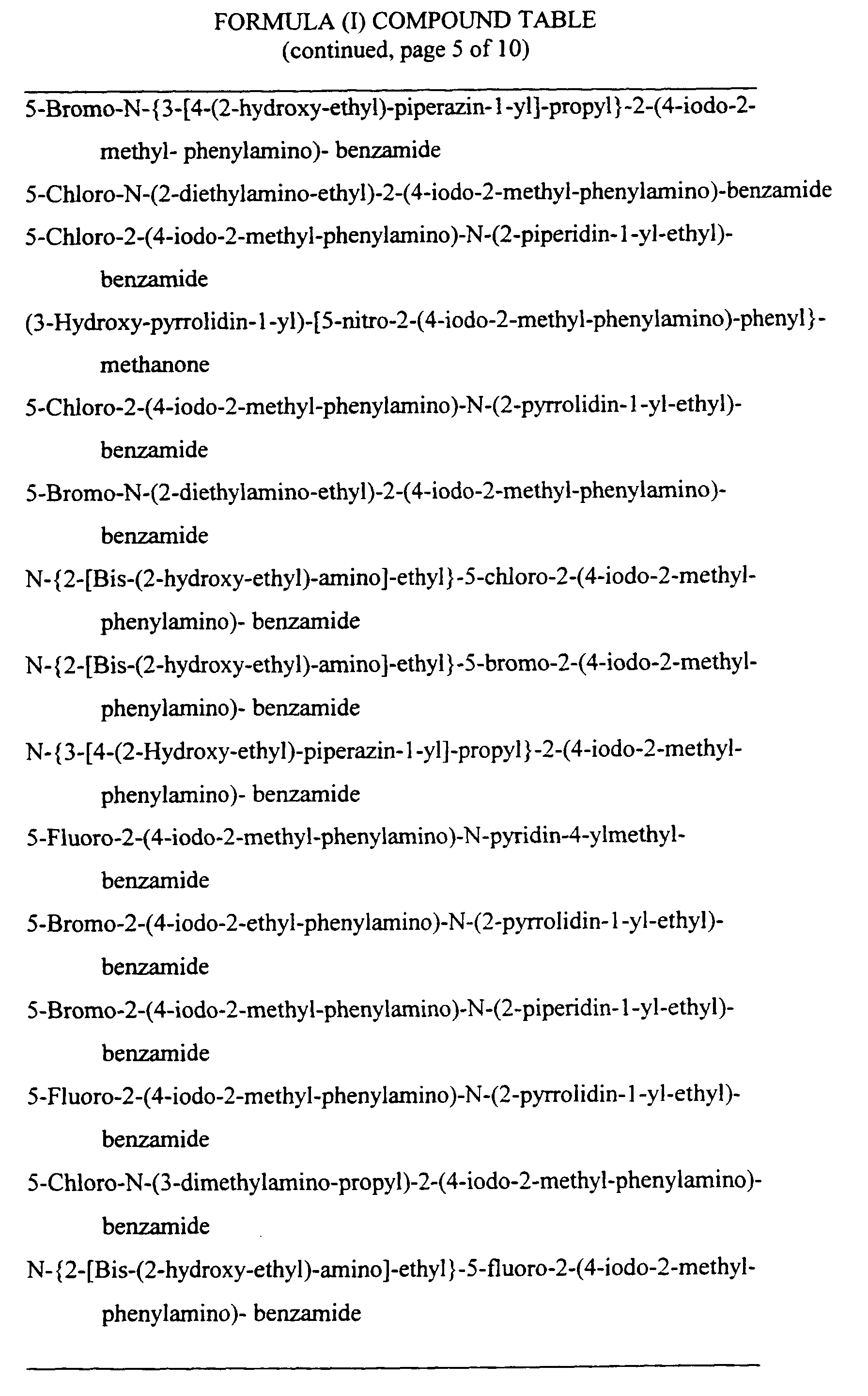

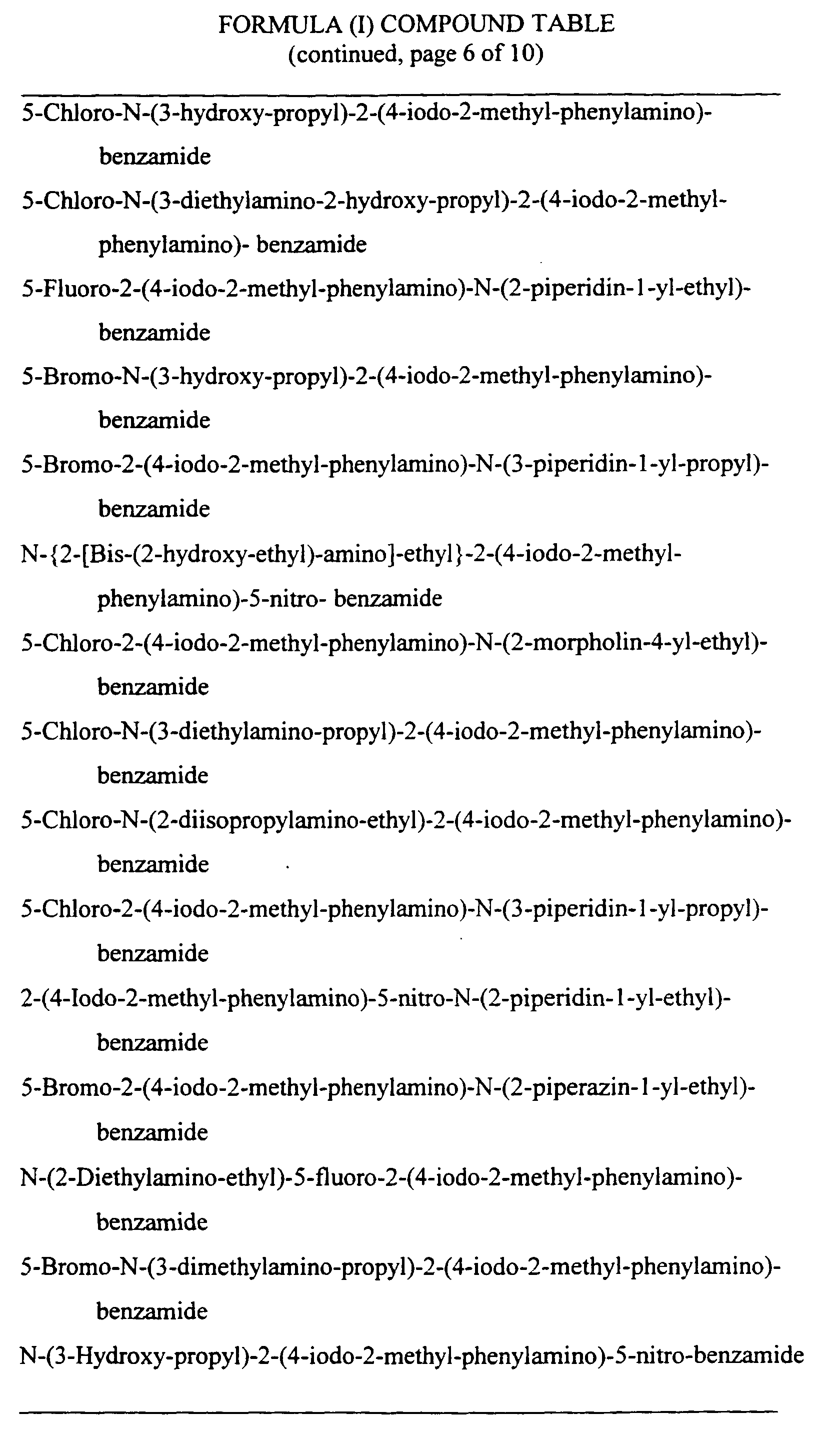

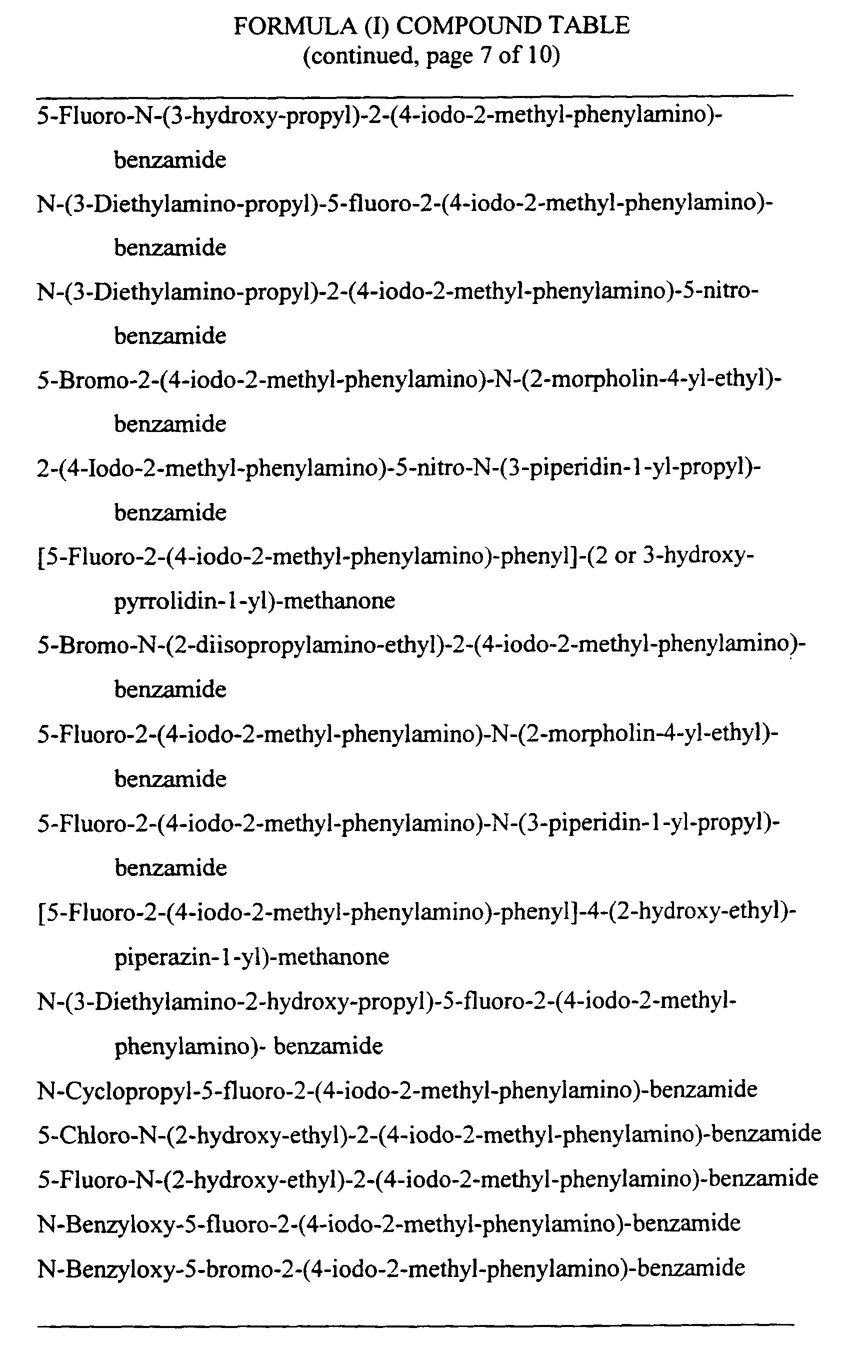

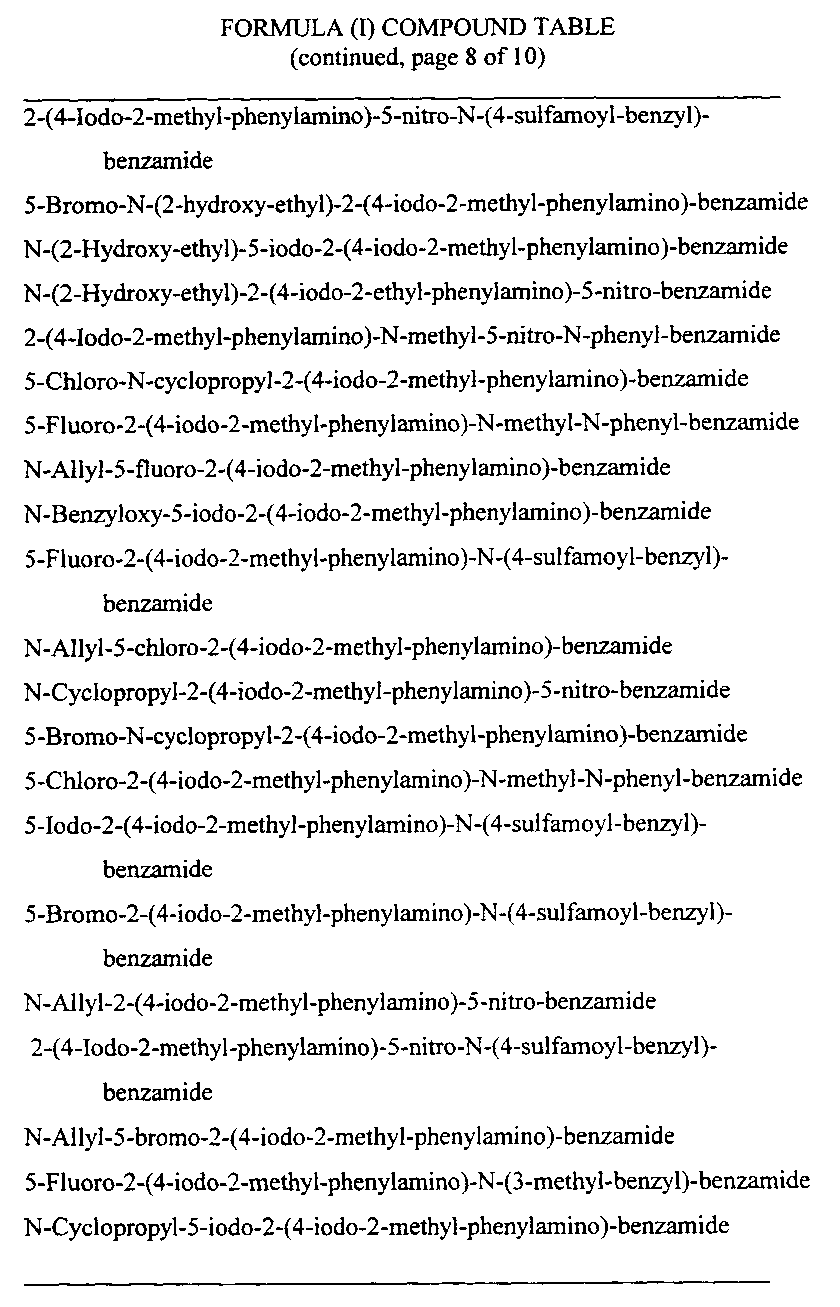

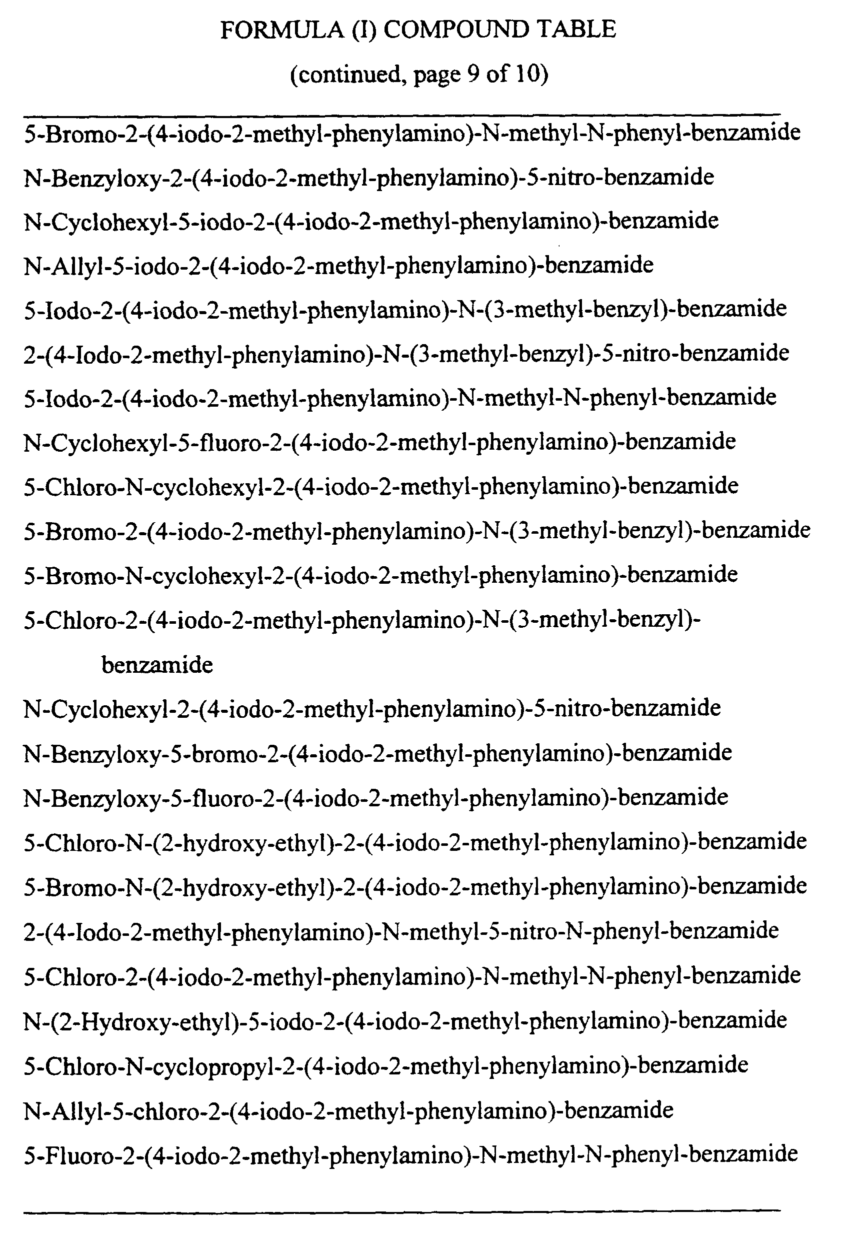

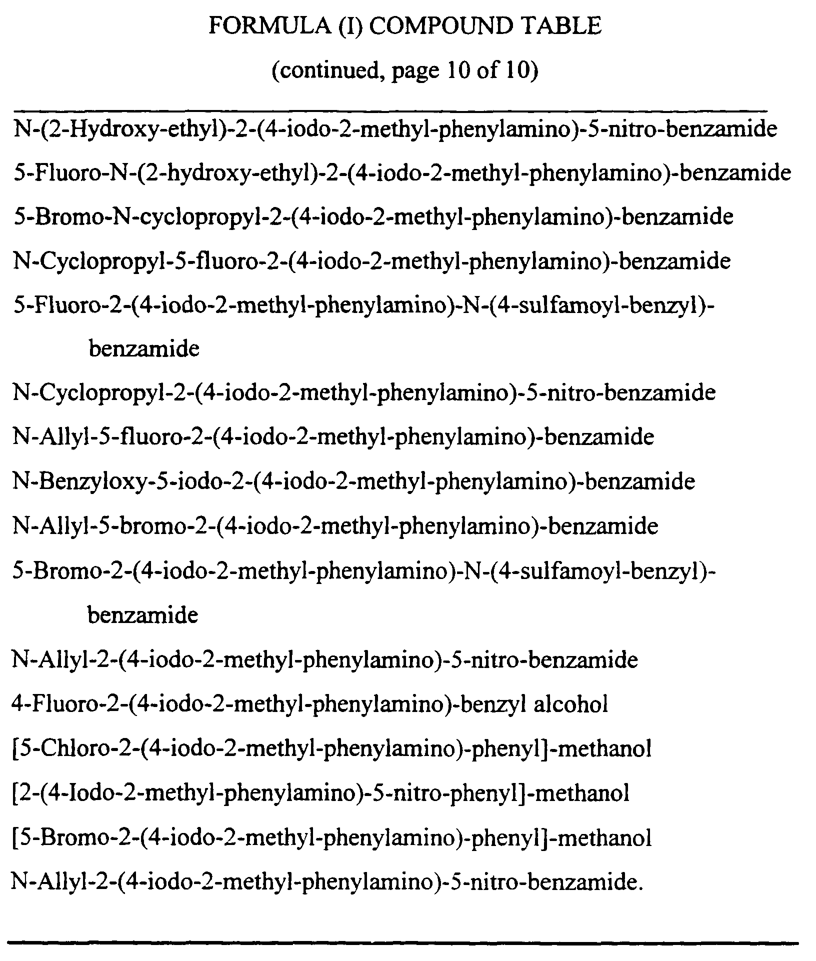

In another preferred embodiment, the MEK inhibitor is a compound of Formula II

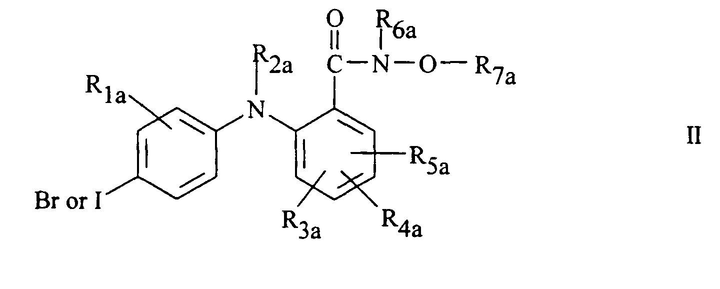

In Formula (II), R₁ₐ is hydrogen, hydroxy, C₁-C₈ alkyl, C₁-C₈ alkoxy, halo, trifluoromethyl, or CN. R₂ₐ is hydrogen. Each of R₃ₐ, R₄ₐ, and R₅ₐ is independently selected from hydrogen, hydroxy, halo, trifluoromethyl, C₁-C₈ alkyl, C₁-C₈ alkoxy, nitro, CN, and (O or NH)ₘ-(CH₂)ₙ-R₉ₐ- R₉ₐ is hydrogen, hydroxy, CO₂H or NR₁₀ₐR₁₁ₐ; n is 0-4; and m is 0 or 1. Each of R₁₀ₐ and R₁₁ₐ is independently hydrogen or C₁-C₈ alkyl, or taken together with the nitrogen to which they are attached can complete a 3- to 10-member cyclic ring optionally containing one, two, or three additional heteroatoms selected from O, S, NH, or N-(C₁-C₈ alkyl). R₆ₐ is hydrogen, C₁-C₈ alkyl, (CO)-(C₁-C₈ alkyl), aryl, aralkyl, or C₃-C₁₀ cycloalkyl. R₇ₐ is hydrogen, C₁-C₈ alkyl, C₂-C₈ alkenyl, C₂-C₈ alkynyl, C₃-C₁₀ (cycloalkyl or cycloalkyl optionally containing a heteroatom selected from O, S, or NR₉ₐ). In Formula (II), any of the foregoingany of the foregoing alkyl, alkenyl, aryl, heteroaryl, heterocyclic, and alkynyl groups can be unsubstituted or substituted by halo, hydroxy, C₁-C₆ alkoxy, amino, nitro, C₁-C₄ alkylamino, di(C₁-C₄)alkylamino, C₃-C₆ cycloalkyl, phenyl, phenoxy, C₃-C₅ heteroaryl or heterocyclic radical, or C₃-C₅ heteroaryloxy or heterocyclic radical-oxy; or R₆ₐ and R₇ₐ taken together with the N to which they are attached can complete a 5- to 10-membered cyclic ring, optionally containing one, two, or three additional heteroatoms selected from O, S, or NR₁₀ₐR₁₁ₐ. The invention also encompasses pharmaceutically acceptable salts of each of the disclosed compounds.

In a more preferred embodiment the MEK inhibitor is a compound selected from Formula (II) Compound Table below.

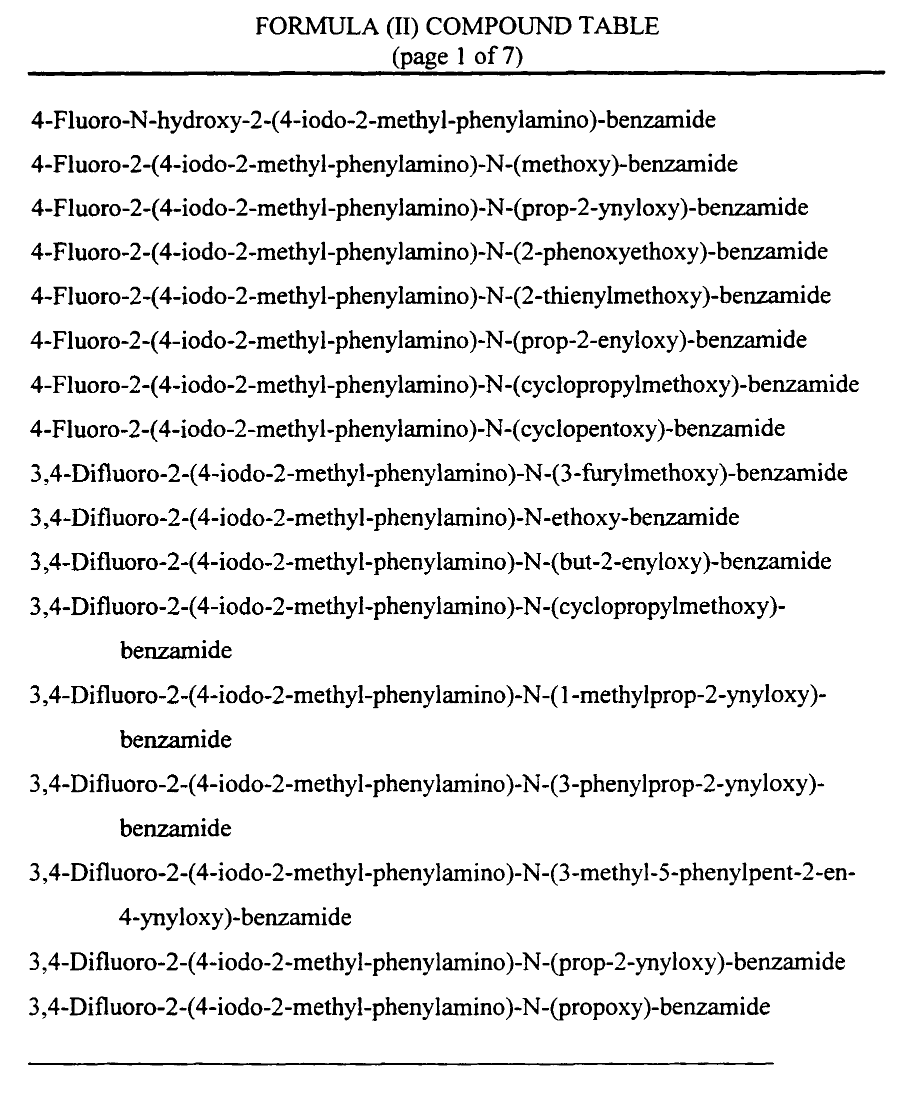

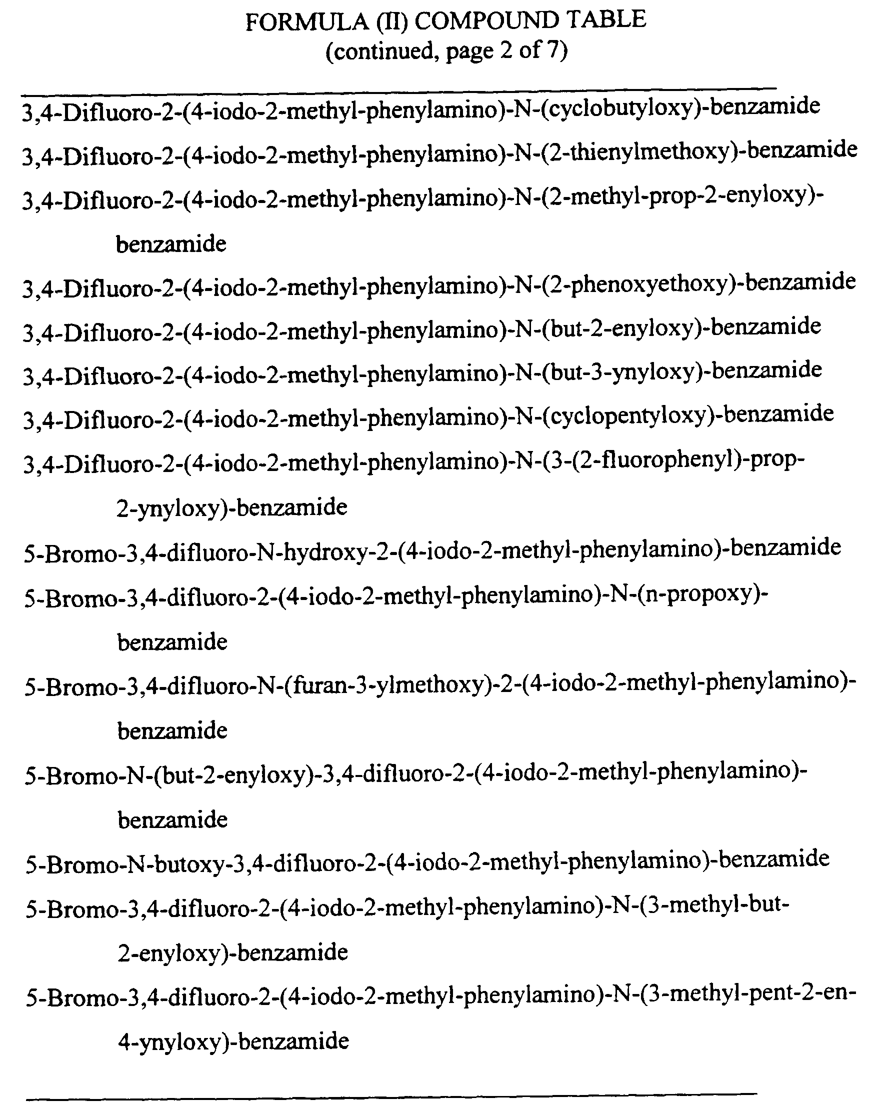

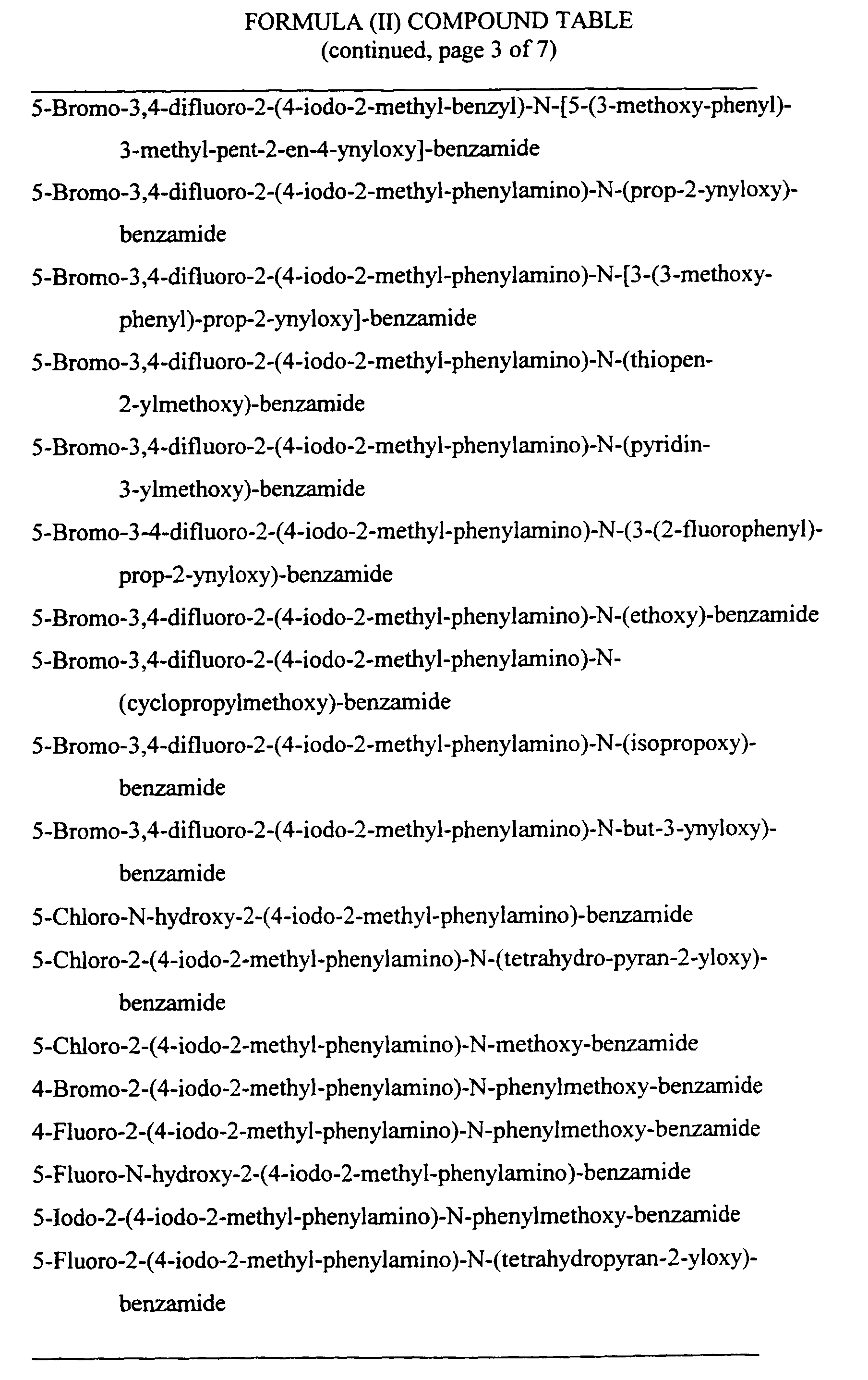

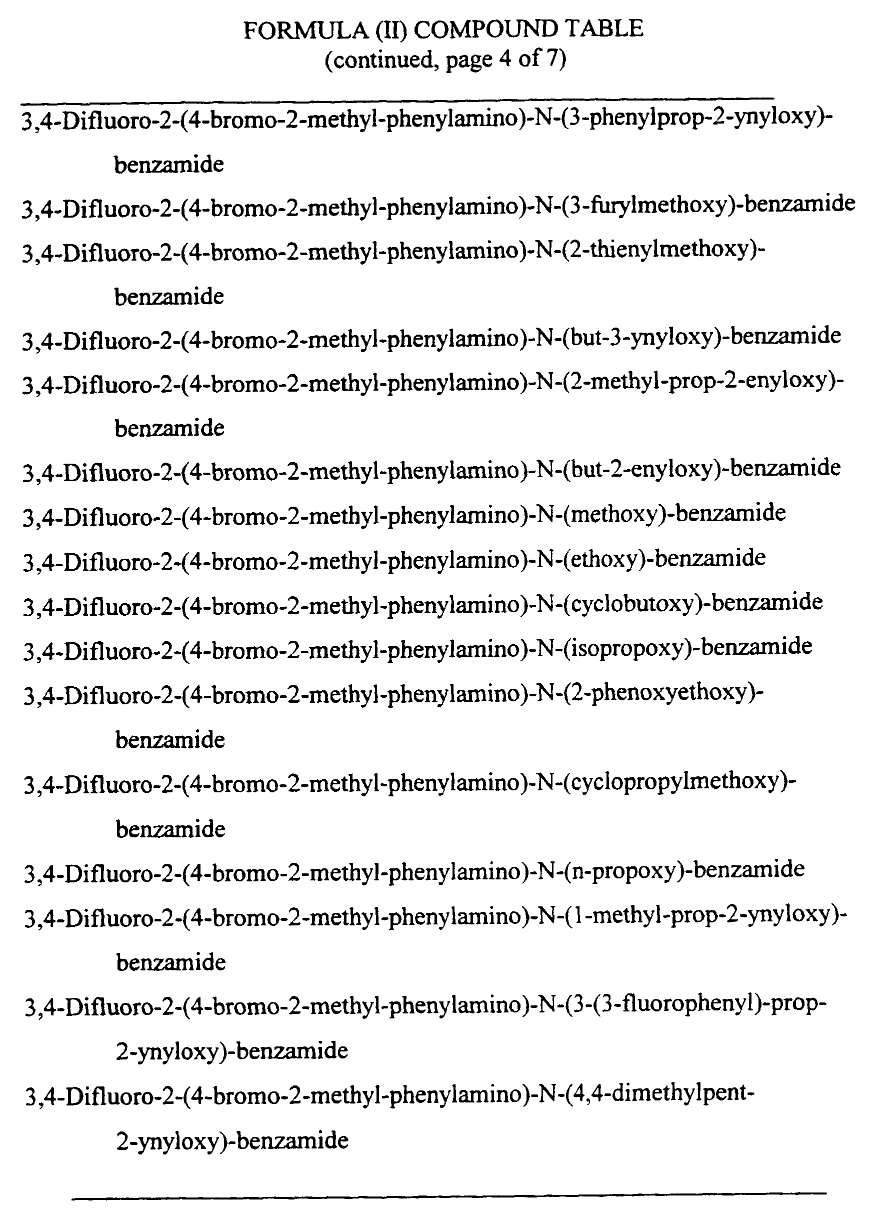

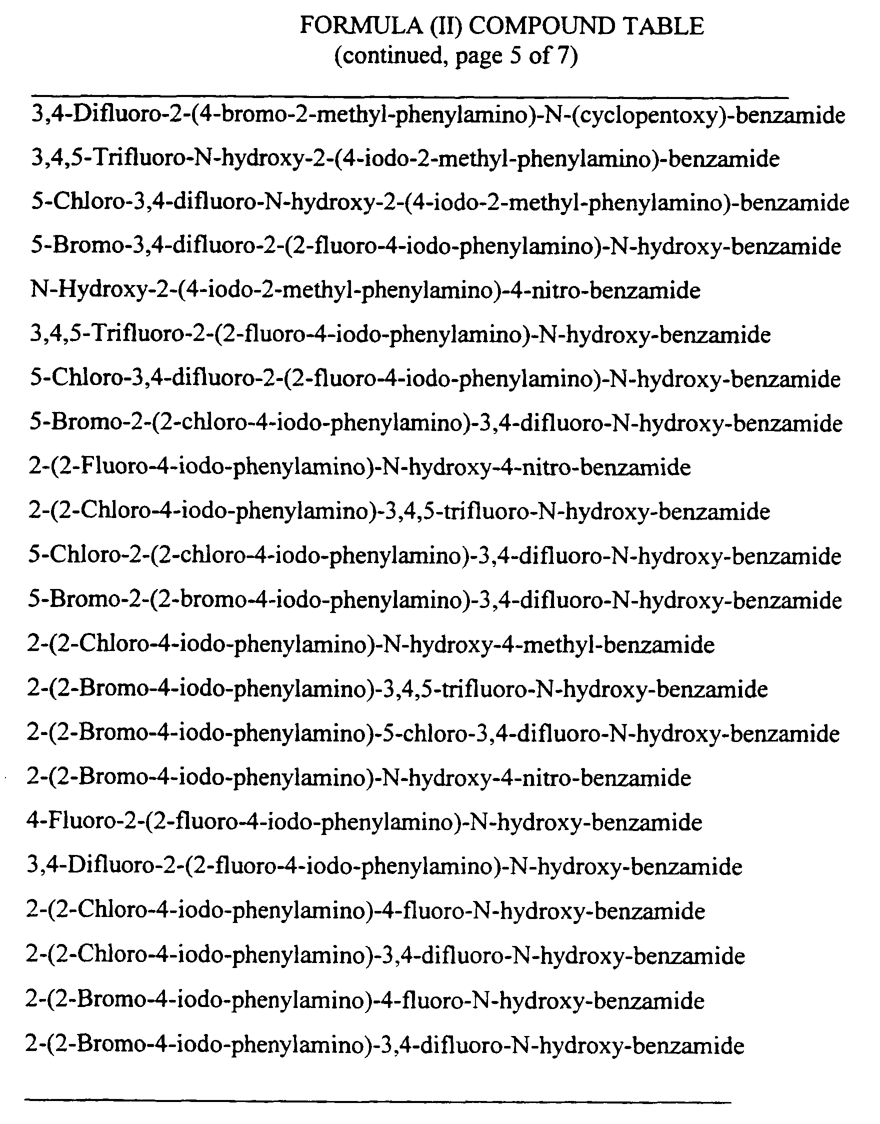

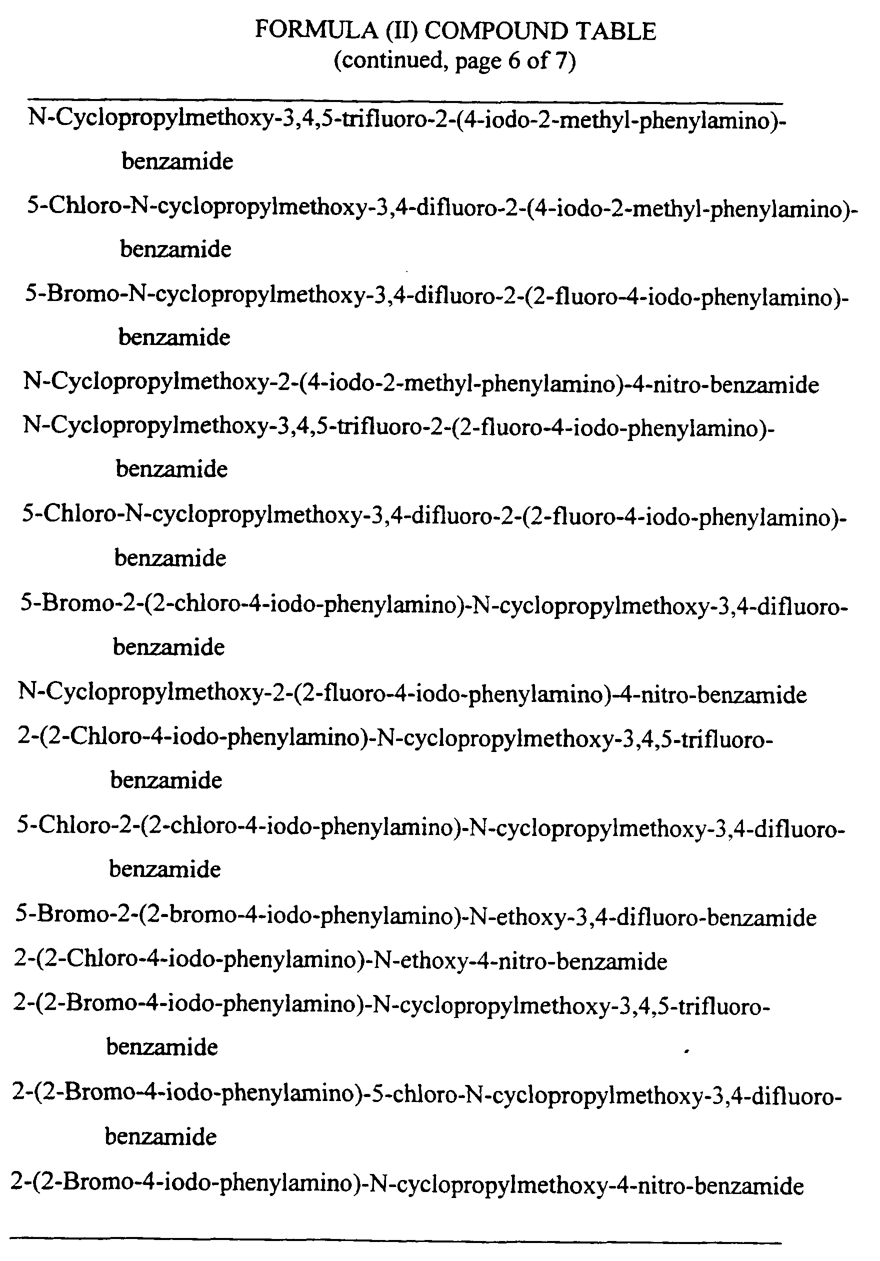

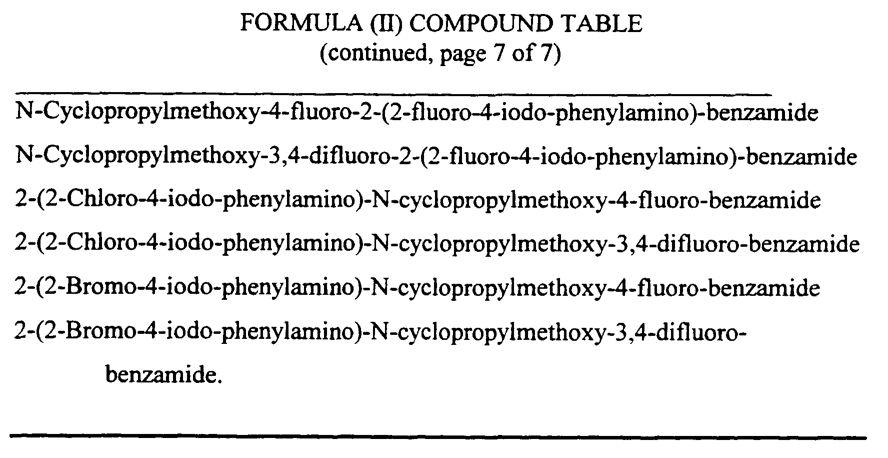

In a further preferred embodiment of this invention, a mitotic inhibitor is administered to a patient suffering from cancer and in need of treatment in combination with a selective MEK inhibitor selected from: 2-(2-Chloro-4-iodophenylamino)-N-cyclopropylmethoxy-3,4-difluorobenzamide (PD184352); 2-(2-Methyl-4-iodophenylamino)-N-hydroxy-4-fluorobenzamide (PD 170611); 2-(2-Methyl-4-iodophenylamino)-N-hydroxy-3,4-difluoro-5-bromobenzamide (PD171984); 2-(2-Methyl-4-iodophenylamino)-N-cyclopropylmethoxy-3,4-difluoro-5-bromobenzamide (PD177168); 2-(2-Methyl-4-iodophenylamino)-N-cyclobutylmethoxy-3,4-difluoro-5-bromobenzamide (PD 180841); 2-(2-Chloro-4-iodophenylamino)-N-cyclopropylmethoxy-3,4-difluoro-5-bromobenzamide (PD 184161); 2-(2-Chloro-4-iodophenylamino)-N-hydroxy-3,4-difluoro-5-bromobenzamide (PD 184386); 2-(2-Chloro-4-iodophenylamino)-N-cyclobutylmethoxy-3,4-difluorobenzamide (PD 185625); 2-(2-Chloro-4-iodophenylamino)-N-hydroxy-4-fluorobenzamide (PD 185848); 2-(2-Methyl-4-iodophenylamino)-N-hydroxy-3,4-difluorobenzamide (PD 188563); 2-(2-Methyl-4-iodophenylamino)-N-cyclopropylmethoxy-3,4,5-trifluorobenzamide (PD 198306); and 2-(2-Chloro-4-iodophenylamino)-N-cyclopropylmethoxy-4-fluorobenzamide (PD 203311); and the benzoic acid derivatives thereof. For example, the benzoic acid derivative of PD 198306 is 2-(2-Methyl-4-iodophenylamino)-3,4,5-trifluorobenzoic acid.

Additional preferred compounds include 2-(2-chloro-4-iodophenylamino)-5-chloro-N-cyclopropylmethoxy -3,4-difluorobenzamide (PD 297189), 2-(4-iodophenylamino)-N-cyclopropylmethoxy-5-chloro-3,4-difluorobenzamide (PD 297190), 2-(4-iodophenylamino)-5-chloro-3,4-difluorobenzoic acid (PD 296771), 2-(2-chloro-4-iodophenylamino)-5-chloro-3,4-difluorobenzoic acid (PD 296770), 5-chloro-3,4-difluoro-2-(4-iodo-2-methylphenylamino)-benzoic acid (PD 296767); and 5-chloro-N-cyclopropylmethoxy -3,4-difluoro-2-(4-iodo-2-methylphenylamino)-benzamide.

The most preferred embodiment of this invention is a combination of paclitaxel and the MEK inhibitor 2-(2-chloro-4-iodophenylamino)-N-cyclopropylmethoxy-3,4-difluorobenzamide (PD184352).

Other features and advantages of the invention are apparent from the figures, description, examples, and claims below.

### BRIEF DESCRIPTION OF THE FIGURES

FIG. 1 shows the effect on apoptosis in colon 26 carcinoma cells of paclitaxel (Taxol®, paclitaxel injection, Bristol-Meyers Squibb) alone, of 2-(2-chloro-4-iodophenylamino)-N-cyclopropylmethoxy-3,4-difluorobenzamide (PD184352) alone, and of the combination of the two agents.
FIG. 2 shows a second experiment measuring the effect on apoptosis in colon 26 carcinoma cells of Taxol alone and of 2-(2-chloro-4-iodophenylamino)-N-cyclopropylmethoxy-3,4-difluorobenzamide (PD 184352) alone, and the combination of the two agents.
FIG. 3 shows the effect on apoptosis in HT-29 colon carcinoma cells treated with Taxol alone, with 2-(2-chloro-4-iodophenylamino)-N-cyclopropylmethoxy-3,4-difluorobenzamide (PD184352) alone, and the combination of the two agents.

### DETAILED DESCRIPTION OF THE INVENTION

This invention provides a treatment of cancer in a patient which comprises administering to a patient suffering from cancer and in need of treatment an antitumor effective amount of a mitotic inhibitor in combination with an antitumor effective amount of a selective MEK inhibitor. Preferred mitotic inhibitors to be used according to this invention include paclitaxel, docletaxel, vincristine, vinblastine, vinorelbine, and the fluorinated derivative of vinorelbine, vinflunine. The invention is practiced by administering a phenyl amine MEK inhibitor of Formula I or Formula II in combination with a mitotic inhibitor, especially paclitaxel. Such MEK phenyl amine compounds are specific MEK 1 and MEK 2 inhibitors, meaning that they inhibit these enzymes without inhibiting other enzymes to a great extent.

The mammals to be treated according to this invention are patients, both humans and animals such as horses and dogs, who have developed a cancer and who are in need of treatment. Those skilled in the medical art are readily able to identify individual patients who are afflicted with cancer and who are in need of treatment. Typical cancers to be treated according to this invention are colon cancer, pancreatic cancer, breast cancer, ovarian cancer, lung cancer and other cancers susceptible to treatment with mitotic inhibitors such as paclitaxel and/or MEK inhibitors.

As noted above, the MEK inhibitors can be formulated for administration by the oral or parenteral routes. They can also be administered transdermally, as skin patches or lotions, or as suppositories. While the MEK inhibitors can be formulated with paclitaxel, for instance in solution for intravenous injection or infusion, the active agents will more typically be formulated individually in their normal preparations, and will be administered individually, but generally at about the same time, or together in a course of treatment. For example, paclitaxel is available commercially in sterile nonpyrogenic solutions containing polyoxyethylated castor oil and dehydrated alcohol. The product is available in packages of 30 mg/5 mL and 100 mg/16.7 mL. The MEK inhibitor and paclitaxel can be formulated individually and packaged together, in a kit for example, for convenience in usage. Alternatively, the agents can be formulated together in a single formulation, in which case the paclitaxel will be present at concentrations ranging from about 1 to about 1000 parts by weight relative to the MEK inhibitor, and the MEK inhibitor will be present at concentrations of about 1000 to about 1 part by weight relative to the paclitaxel. Generally, the agents will be administered at about equal doses, or as otherwise approved by health regulatory agencies.

Further examples of combinations provided by this invention include: (a) vincristine administered in combination with 2-(2-methyl-4-iodophenylamino)-N-hydroxy-3,4-difluoro-5-bromobenzamide; (b) the mitotic inhibitor docetaxel (Taxotere® Rhone Poulenc Rorer) administered in combination with the selective MEK inhibitor 2-(2-chloro-4-iodophenylamino)-N-hydroxy-3,4-difluoro-5-bromobenzamide; (c) the mitotic inhibitor vinflunine, the fluoro derivative of vinorelbine, administered in combination with the selective MEK inhibitor is 2-(2-methyl-4-iodophenylamino)-N-hydroxy-4-fluorobenzamide.

Compounds of the combinations of the present are MEK inhibitors, which also can be used individually to treat septic shock. A MEK inhibitor is a compound that shows MEK inhibition when tested in the assays titled "Enzyme Assays" in United States Patent Number 5,525,625, column 6, beginning at line 35. An example of a MEK inhibitor is 2-(2-amino-3-methoxyphenyl)-4-oxo-4H-[1]benzopyran. Specifically, a compound is a MEK inhibitor if a compound shows activity in the assay titled "Cascade Assay for Inhibitors of the MAP Kinase Pathway," column 6, line 36 to column 7, line 4 of the United States Patent Number 5,525,625 and/or shows activity in the assay titled "In Vitro MEK Assay" at column 7, lines 4 to 27 of the above-referenced patent.

Other features and advantages of the invention are apparent from the description, examples, and claims below.

### A. Terms

Some of the terms used herein are defined below and by their usage throughout this disclosure.

The term "patient" means all animals including humans. Examples of patients include humans, cows, dogs, cats, goats, sheep, horses, and pigs.

As used herein, the term "aryl" means a cyclic, bicyclic, or tricyclic aromatic ring moiety having from five to twelve carbon atoms. Examples of typical aryl groups include phenyl, naphthyl, and fluorenyl. The aryl may be substituted by one, two, or three groups selected from fluoro, chloro, bromo, iodo, alkyl, hydroxy, alkoxy, nitro, amino, alkylamino, or dialkylamino. Typical substituted aryl groups include 3-fluorophenyl, 3,5-dimethoxyphenyl, 4-nitronaphthyl, 2-methyl-4-chloro-7-aminofluorenyl, and the like.

The term "aryloxy" means an aryl group bonded through an oxygen atom, for example phenoxy, 3-bromophenoxy, naphthyloxy, and 4-methyl-1-fluorenyloxy.

"Heteroaryl" means a cyclic, bicyclic, or tricyclic aromatic ring moiety having from four to eleven carbon atoms and one, two, or three heteroatoms selected from O, S, or N. Examples include furyl, thienyl, pyrrolyl, pyrazolyl, imidazolyl, triazolyl, thiazolyl, oxazolyl, xanthenyl, pyronyl, indolyl, pyrimidyl, naphthyridyl, pyridyl, benzinnidazolyl, and triazinyl. The heteroaryl groups can be unsubstituted or substituted by one, two, or three groups selected from fluoro, chloro, bromo, iodo, alkyl, hydroxy, alkoxy, nitro, amino, alkylamino, or dialkylamino. Examples of substituted heteroaryl groups include chloropyranyl, methylthienyl, fluoropyridyl, amino-1,4-benzisoxazinyl, nitroisoquinolinyl, and hydroxyindolyl.

The heteroaryl groups can be bonded through oxygen to make heteroaryloxy groups, for example thienyloxy, isothiazolyloxy, benzofuranyloxy, pyridyloxy, and 4-methylisoquinolinyloxy.

The term "alkyl" means straight and branched chain aliphatic groups. Typical alkyl groups include methyl, ethyl, isopropyl, tert.-butyl, 2,3-dimethylhexyl, and 1,1-dimethylpentyl. The alkyl groups can be unsubstituted or substituted by halo, hydroxy, alkoxy, amino, alkylamino, dialkylamino, cycloalkyl, aryl, aryloxy, heteroaryl, or heteroaryloxy, as those terms are defined herein. Typical substituted alkyl groups include chloromethyl, 3-hydroxypropyl, 2-dimethylaminobutyl, and 2-(hydroxymethylamino)ethyl. Examples of aryl and aryloxy substituted alkyl groups include phenylmethyl, 2-phenylethyl, 3-chlorophenylmethyl, 1,1-dimethyl-3-(2-nitrophenoxy)butyl, and 3,4,5-trifluoronaphthylmethyl. Examples of alkyl groups substituted by a heteroaryl or heteroaryloxy group include thienylmethyl, 2-furylethyl, 6-furyloxyoctyl, 4-methylquinolyloxymethyl, and 6-isothiazolylhexyl. Cycloalkyl substituted alkyl groups include cyclopropylmethyl, 2-cyclohexyethyl, piperidyl-2-methyl, 2-(piperidin-1-yl)-ethyl, 3-(morpholin-4-yl)propyl.

"Alkenyl" means a straight or branched carbon chain having one or more double bonds. Examples include but-2-enyl, 2-methyl-prop-2-enyl, 1,1-dimethyl-hex-4-enyl, 3-ethyl-4-methyl-pent-2-enyl, and 3-isopropyl-pent-4-enyl. The alkenyl groups can be substituted with halo, hydroxy, alkoxy, amino, alkylamino, dialkylamino, aryl, aryloxy, heteroaryl, or heteroyloxy, for example 2-bromoethenyl, 3-hydroxy-2-butenyl, 1-aminoethenyl, 3-phenylprop-2-enyl, 6-thienyl-hex-2-enyl, 2-furyloxy-but-2-enyl, and 4-naphthyloxy-hex-2-enyl.

"Alkynyl" means a straight or branched carbon chain having at least one triple bond. Typical alkynyl groups include prop-2-ynyl, 2-methyl-hex-5-ynyl, 3,4-dimethyl-hex-5-ynyl, and 2-ethyl-but-3-ynyl. The alkynyl groups can be substituted as the alkyl and alkenyl groups, for example, by aryl, aryloxy, heteroaryl, or heteroaryloxy, for example 4-(2-fluorophenyl)-but-3-ynyl, 3-methyl-5-thienylpent-4-ynyl, 3-phenoxy-hex-4-ynyl, and 2-furyloxy-3-methyl-hex-4-ynyl.

The alkenyl and alkynyl groups can have one or more double bonds or triple bonds, respectively, or a combination of double and triple bonds. For example, typical groups having both double and triple bonds include hex-2-en-4-ynyl, 3-methyl-5-phenylpent-2-en-4-ynyl, and 3-thienyloxy-hex-3-en-5-ynyl.

The term "cycloalkyl" means a nonaromatic ring or fused rings. Examples include cyclopropyl, cyclobutyl, cyclopenyl, cyclooctyl, bicycloheptyl, adamantyl, and cyclohexyl. The ring can optionally contain one, two, or three heteroatoms selected from O, S, or N. Such groups include tetrahydrofuryl, tetrahydropyrrolyl, octahydrobenzofuranyl, morpholinyl, piperazinyl, pyrrolidinyl, piperidinyl, octahydroindolyl, and octahydrobenzothiofuranyl. The cycloalkyl groups can be substituted with the same substituents as an alkyl and alkenyl groups, for example, halo, hydroxy, aryl, and heteroaryloxy. Examples include 3-hydroxycyclohexyl, 2-aminocyclopropyl, 2-phenylpyrrolidinyl, and 3-thienylmorpholine-1-yl.

### B. Administration and Formulation

The MEK inhibitors of the present treatment can be administered to a patient as part of a pharmaceutically acceptable composition. The compositions can be administered to humans and animals either orally, rectally, parenterally (intravenously, intramuscularly,or subcutaneously), intracisternally, intravaginally, intraperitoneally, intravesically, locally (powders, ointments, or drops), or as a buccal or nasal spray.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles include water, ethanol, polyols (propyleneglycol, polyethyleneglycol, glycerol, and the like), suitable mixtures thereof, vegetable oils (such as olive oil), and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, carboxymethylcellulose, alignates, gelatin, polyvinylpyrrolidone, sucrose, and acacia, (c) humectants, as for example, glycerol, (d) disintegrating agents, as for example, agar-agar, calcium carbonate, potato or tapioca starch, alginic acid, certain complex silicates, and sodium carbonate, (e) solution retarders, as for example paraffin, (f) absorption accelerators, as for example, quaternary ammonium compounds, (g) wetting agents, as for example, cetyl alcohol and glycerol monostearate, (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid compositions of a similar type may also be employed as fillers in soft and hard-filled gelatin capsules using such excipients as lactose or milk sugar as well as high molecular weight polyethyleneglycols, and the like.

Solid dosage forms such as tablets, dragees, capsules, pills, and granules can be prepared with coatings and shells, such as enteric coatings and others well-known in the art. They may contain opacifying agents, and can also be of such composition that they release the active compound or compounds in a certain part of the intestinal tract in a delayed manner. Examples of embedding compositions which can be used are polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil and sesame oil, glycerol, tetrahydrofurfuryl alcohol, polyethyleneglycols, and fatty acid esters of sorbitan or mixtures of these substances, and the like.

Besides such inert diluents, the composition can also include adjuvants, such as wetting agents, emulsifying and suspending agents, sweetening, flavoring, and perfuming agents.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administrations are preferably suppositories which can be prepared by mixing the compounds of the present invention with suitable non-irritating excipients or carriers such as cocoa butter, polyethyleneglycol, or a suppository wax, which are solid at ordinary temperatures but liquid at body temperature and therefore, melt in the rectum or vaginal cavity and release the active component.

Dosage forms for topical administration of a compound of this invention include ointments, powders, sprays, and inhalants. The active component is admixed under sterile conditions with a physiologically acceptable carrier and any preservatives, buffers, or propellants as may be required. Ophthalamic formulations, eye ointments, powders, and solutions are also contemplated as being within the scope of this invention.

The compounds of the present treatment can be administered to a patient at dosage levels in the range of 0.1 to 1000 mg per day. For a normal human adult having a body weight of about 70 kg, a dosage in the range of 0.01 to 100 mg per kg of body weight per day is preferable. The specific dosage used, however, can vary. For example, the dosage can depend on a numbers of factors including the requirements of the patient, the severity of the condition being treated, and the pharmacological activity of the compound being used. The determination of optimum dosages for a particular patient is well-known to those skilled in the art.

The compounds of the present treatment can be administered as pharmaceutically acceptable salts. The term "pharmaceutically acceptable salts" as used herein refers to those carboxylate salts, amino acid addition salts of the compounds of the present invention which are, within the scope of sound medical judgment, suitable for contact with the tissues of patients without undue toxicity, irritation, allergic response, and the like, commensurate with a reasonable benefit/risk ratio, and effective for their intended use, as well as the zwitterionic forms, where possible, of the compounds of the invention.

The term "salts" refers to the relatively non-toxic, inorganic and organic acid addition salts of compounds of the present invention. These salts can be prepared *in situ* during the final isolation and purification of the compounds or by separately reacting the purified compound in its free base form with a suitable organic or inorganic acid and isolating the salt thus formed. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate and laurylsulphonate salts, and the like. These may include cations based on the alkali and alkaline earth metals, such as sodium, lithium, potassium, calcium, magnesium and the like, as well as nontoxic ammonium, quaternary ammonium, and amine cations including, but not limited to ammonium, tetramethylammonium, tetraethylammonium, methylamine, dimethylamine, trimethylamine, triethylamine, ethylamine, and the like. (See, for example, S.M. Berge, et al., "Pharmaceutical Salts," *J. Pharm. Sci.,* 1977;66:1-19)

In addition, the compounds of the present treatment can exist in unsolvated as well as solvated forms with pharmaceutically acceptable solvents such as water, ethanol, and the like. In general, the solvated forms are considered equivalent to the unsolvated forms for the purposes of the present invention.

Some of the compounds of the present treatment can exist in different stereoisometric forms by virtue of the presence of chiral centers. It is contemplated that all stereoisometric forms of the compounds as well as mixtures thereof, including racemic mixtures, form part of this invention.

### C. Synthesis

The examples presented below are intended to illustrate particular embodiments of the invention. After the priority date of the present disclosure, related syntheses and MEK inhibition data were also published in WO 99/01421 and WO 99/01426.

The 2-(4-bromo and 4-iodo phenylamino)-benzoic acid derivatives of Formula I can be prepared from commercially available starting materials utilizing synthetic methodologies well-known to those skilled in organic chemistry. A typical synthesis is carried out by reacting a 4-bromo or 4-iodo aniline with a benzoic acid having a leaving group at the 2-position to give a 2-(phenylamino)-benzoic acid. This process is depicted in Scheme 1. 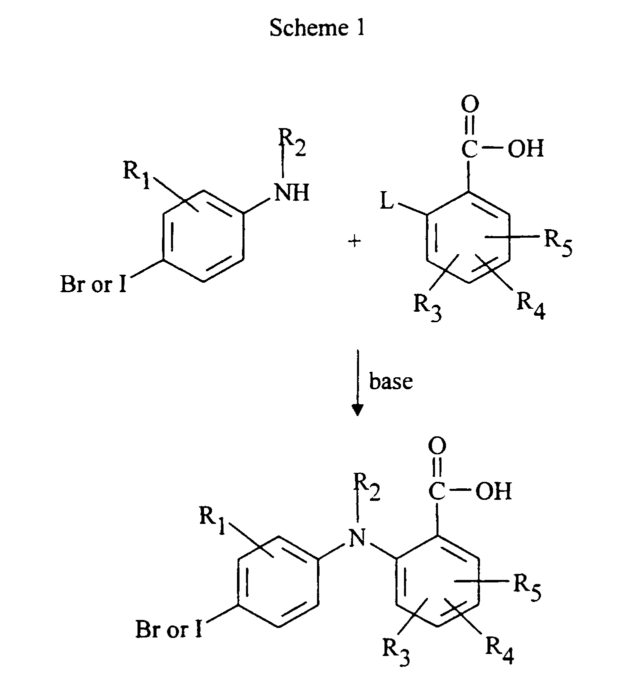 where L is a leaving group, for example halo such as fluoro.

The reaction of aniline and the benzoic acid derivative generally is accomplished by mixing the benzoic acid with an equimolar quantity or excess of the aniline in an unreactive organic solvent such as tetrahydrofuran or toluene, in the presence of a base such as lithium diisopropylamide, n-butyl lithium, sodium hydride, triethylamine, and Hunig's base. The reaction generally is carried out at a temperature of about -78°C to about 100°C, and normally is complete within about 2 hours to about 4 days. The product can be isolated by removing the solvent, for example by evaporation under reduced pressure, and further purified, if desired, by standard methods such as chromatography, crystallization, or distillation.

The 2-(phenylamino)-benzoic acid (e.g., Formula I, where R₇ is hydrogen) can be reacted with an organic or inorganic base such as pyridine, triethylamine, calcium carbonate, or sodium hydroxide to produce a pharmaceutically acceptable salt. The free acids can also be reacted with an alcohol of the formula HOR₇ (where R₇ is other than hydrogen, for example methyl) to produce the corresponding ester. Reaction of the benzoic acid with an alcohol can be carried out in the presence of a coupling agent. Typical coupling reagents include 2-ethoxy-1-ethoxycarbonyl-1,2-dihydroquinoline (EEDQ), 1,3-dicyclohexylcarbodiimide (DCC), bromo-tris(pyrrolidino)- phosphonium hexafluorophosphate (PyBrOP), and (benzotriazolyloxy) tripyrrolidino phosphonium hexafluorophosphate (PyBOP). The phenylamino benzoic acid and alcohol derivative normally are mixed in approximately equimolar quantities in an unreactive organic solvent such as dichloromethane, tetrahydrofuran, chloroform, or xylene, and an equimolar quantity of the coupling reagent is added. A base such as triethylamine or diisopropylethylamine can be added to act as an acid scavenger if desired. The coupling reaction generally is complete after about 10 minutes to 2 hours, and the product is readily isolated by removing the reaction solvent, for instance by evaporation under reduced pressure, and purifying the product by standard methods such as chromatography or crystallizations from solvents such as acetone, diethyl ether, or ethanol.

The benzamides of the invention, Formula I where Z is CONR₆R₇, are readily prepared by reacting the foregoing benzoic acids with an amine of the formula HNR₆R₇. The reaction is carried out by reacting approximately equimolar quantities of the benzoic acid and amine in an unreactive organic solvent in the presence of a coupling reagent. Typical solvents are chloroform, dichloromethane, tetrahydrofuran, benzene, toluene, and xylene. Typical coupling reagents include DCC, EEDQ, PyBrOP, and PyBOP. The reaction is generally complete after about 10 minutes to about 2 hours when carried out at a temperature of about 0°C to about 60°C. The product amide is readily isolated by removing the reaction solvent, for instance by evaporation, and further purification can be accomplished by normal methods such as chromatography, crystallization, or distillation. The hydrazides (z = CONHNR₁₀R₁₁) are similarly prepared by coupling a benzoic acid with a hydrazine of the formula H₂HNR₁₀R₁₁.

The benzyl alcohols of the invention, compounds of Formula I where Z is CH₂OR₆ and R₆ is hydrogen, are readily prepared by reduction of the corresponding benzoic acid according to the following Scheme 2. 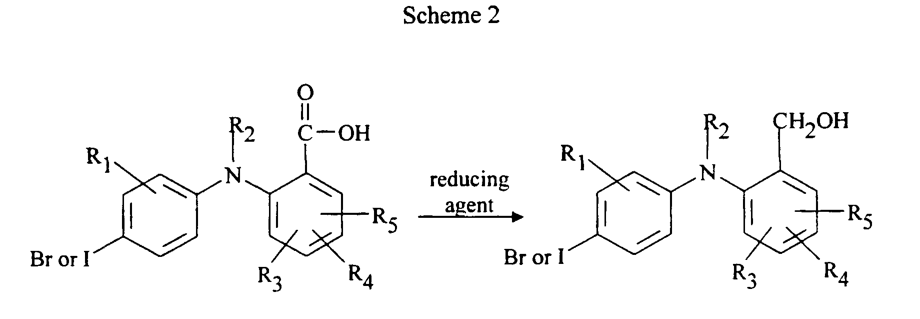 Typical reducing agents commonly employed include borane in tetrahydrofuran. The reduction normally is carried out in an unreactive organic solvent such as tetrahydrofuran, and generally is complete within about 2 hours to about 24 hours when conducted at a temperature of about 0°C to about 40°C.

The following detailed examples illustrate specific compounds provided by this invention.

### EXAMPLE 1

### 4-Fluoro-2-(4-iodo-2-methylphenylamino)benzoic acid

To a stirring solution comprised of 3.16 g (0.0133 mol) of 2-amino-5-iodotoluene in 5 mL of tetrahydrofuran at -78°C was added 10 mL (0.020 mol) of a 2.0 M lithium diisopropylamide in tetrahydrofuran/heptane/ethenylbenzene (Aldrich) solution. The resulting green suspension was stirred vigorously for 15 minutes, after which time a solution of 1.00 g (0.00632 mol) of 2,4-difluorobenzoic acid in 10 mL of tetrahydrofuran was added. The reaction temperature was allowed to increase slowly to room temperature, at which temperature it was stirred for 2 days. The reaction mixture was concentrated. Aqueous HCl (10%) was added to the concentrate, and the solution was extracted with dichloromethane. The organic phase was dried (MgSO₄) and then boiled over a steambath to low volume and cooled to room temperature. The off-white fibers were collected by vacuum filtration, rinsed with hexanes, and vacuum-oven dried. (76°C; ca. 10 mm of Hg) to afford 1.10 g (47%) of the desired material;
mp 224-229.5°C;
¹H NMR (400 MHz; DMSO): δ 9.72 (s, 1H), 7.97 (dd, 1H, J = 7.0, 8.7 Hz), 7.70 (d, 1H, J = 1.5 Hz), 7.57 (dd, 1H, J = 8.4, 1.9 Hz), 7.17 (d, 1H, J = 8.2 Hz), 6.61-6.53 (m, 2H), 2.18 (s, 3H); ¹³C NMR (100 MHz; DMSO): δ 169.87, 167.60, 165.12, 150.17, 150.05, 139.83, 138.49, 136.07, 135.31, 135.20, 135.07, 125.60, 109.32, 105.09, 104.87, 99.72, 99.46, 89.43, 17.52;
¹⁹F NMR (376 MHz; DMSO): δ -104.00 to -104.07 (m);
IR (KBr) 1670 (C = O stretch) cm⁻¹ ;
MS (CI) M+1 = 372.
Analysis calculated for C₁₄H₁₁FINO₂: C, 45.31; H, 2.99; N, 3.77.
Found: C, 45.21; H, 2.77; N, 3.64.

### EXAMPLES 2-30

By following the general procedure of Example 1, the following benzoic acids and salts of Formula (I) were prepared.

| Example No. | Compound | MP °C |
|---|---|---|
| 2 | 3,4,5-Trifluoro-2-(4-iodo-2-methyl-phenylamino)-benzoic acid | 206-210 |
| 3 | 3,4-Difluoro-2-(4-iodo-2-methyl-phenylamino)-benzoic acid | 240.5-244.5 |
| 4 | 5-Bromo-3,4-difluoro-2-(4-iodo-2-methyl-phenylamino)-benzoic acid | 259.5-262 |
| 5 | 5-Chloro-2-(2-chloro-4-iodo-phenylamino)-benzoic acid | 255-260 |
| 6 | 5-Chloro-2-(4-iodo-2-methyl-phenylamino)-benzoic acid | 234-238 |
| 7 | Sodium 5-Chloro-2-(4-iodo-2-methyl-phenylamino)-benzoate | 310-320 DEC |
| 8 | 5-Bromo-2-(4-iodo-2-methyl-phenylamino)-benzoic acid | 239.5-240 |
| 9 | 2-(2-Chloro-4-iodo-phenylamino)-5-nitro-benzoic acid | 289-293 |
| 10 | 4-Fluoro-2-(3-fluoro-4-iodo-2-methyl-phenylamino)-benzoic acid | 233-235 |
| 11 | 2-(4-Iodo-2-methyl-phenylamino)-5-nitro-benzoic acid | 264-267 |
| 12 | 2-(2-Fluoro-4-iodo-phenylamino)-5-nitro-benzoic acid | 256-258 |
| 13 | 2-(4-Bromo-2-methyl-phenylamino)-4-fluoro-benzoic acid | 218.5-220 |
| 14 | 2-(2-Bromo-4-iodo-phenylamino)-5-nitro-benzoic acid | 285-288 DEC |
| 15 | 2-(4-Bromo-2-methyl-phenylamino)-3,4-difluoro-benzoic acid | 230-234 |
| 16 | 3-Fluoro-2-(4-iodo-2-methyl-phenylamino)-benzoic acid | 218-221 |
| 17 | 3,4-Difluoro-2-(4-iodo-2-methoxy-phenylamino)-benzoic acid | 230-233 |
| 18 | 4-Chloro-2-(4-iodo-2-methyl-phenylamino)-benzoic acid | 245-255 DEC |
| 19 | 2-(4-Iodo-2-methyl-phenylamino)-benzoic acid | 218-223 |
| 20 | 5-Fluoro-2-(4-iodo-2-methyl-phenylamino)-benzoic acid | 243-46 |
| 21 | 5-Iodo-2-(4-iodo-2-methyl-phenylamino)-benzoic acid | 241-245 |
| 22 | 2,3,5-Trifluoro-4-(4-iodo-2-methyl-phenylamino)-benzoic acid | 218-222 |
| 23 | 4-Fluoro-2-(3-chloro-4-iodo-2-methyl-phenylamino)-benzoic acid | 248-252.5 |
| 24 | 2-(4-Iodo-phenylamino)-5-methoxy-benzoic acid | 208-211 |
| 25 | 3-Chloro-2-(2-chloro-4-iodo-phenylamino)-benzoic acid | 232-233 |
| 26 | 2-Fluoro-6-(4-iodo-2-methyl-phenylamino)-benzoic acid | 179-182 |
| 27 | 4-Fluoro2-(2,3-dimethyl-4-iodo-2-methyl-phenylamino)benzoic acid | 258-261 |
| 28 | 5-Methyl-2-(4-iodo-2-methyl-phenylamino)-benzoic acid | 209.5-211 |
| 29 | 2-Chloro-6-(4-iodo-2-methyl-phenylamino)-benzoic acid | 171-175 |
| 30 | 2-(4-Iodo-2-methyl-phenylamino)-4-nitro-benzoic acid | 251-263 |

### EXAMPLE 31

### 5-Chloro-N-(2-hydroxyethyl)-2-(4-iodo-2-methyl-phenylamino)-benzamide

To a stirring solution comprised of 0.1020 g (0.2632 mmol) of 5-chloro-2-(4-iodo-2-methyl-phenylamino)-benzoic acid, 0.1 mL (1.7 mmol) of ethanolamine, and 0.05 mL (0.29 mmol) of diisopropylethylamine in 5 mL of a 1:1 (v/v) tetrahydrofuran-dichloromethane solution was added 0.15 g (0.29 mmol) of solid PyBOP powder directly. The reaction mixture was stirred at room temperature overnight. The solvent was removed in vacuo. The crude residue was partitioned between ether (50 mL) and 10% aqueous hydrochloric acid (50 mL). The organic phase was washed with 10% aqueous sodium hydroxide (50 mL), dried (MgSO₄) and concentrated in vacuo to afford a yellow-brown oil which was crystallized from hexanes-ether to afford 0.0831 g (73%) of a green-yellow powder; mp 120-121 °C;
¹H NMR (400 MHz; CDCl₃): δ 9.11 (s, 1H), 7.56 (d, 1H, J = 1.4 Hz), 7.46-7.41 (m, 2H), 7.20 (dd, 1H, J = 8.9, 2.4 Hz), 7.00 (t, 2H, J = 9.6 Hz), 6.55 (broad t, 1H), 3.86 (t, 2H, J = 5.0 Hz), 3.61 (dd, 2H, J = 10.1, 5.5 Hz), 2.23 (s, 3H), 1.56 (broad s, 1H);
IR (KBr) 3297 (O-H stretch), 1627 (C = O stretch) cm⁻¹ ;
MS (CI) M+1 = 431.
Analysis calculated for C₁₆H₁₆CllN₂O₂:
C, 44.62; H, 3.74; N, 6.50.
Found: 44.63; H, 3.67; N, 6.30.

### EXAMPLES 32-48

By following the general procedure of Example 31, the following benzamides were prepared by reacting the corresponding benzoic acid with the corresponding amine.

| Example No. | Compound | MP °C |
|---|---|---|
| 32 | 4-Methoxy-N-(4-methoxy-phenyl)-3-nitro-benzamide | 153.5-156 |
| 33 | 4-Fluoro-2-(4-iodo-2-methyl-phenylamino)-benzamide | 158 |
| 34 | 4-Fluoro-2-(4-iodo-2-methyl-phenylamino)-N-methyl-benzamide | 102.5-104.5 |
| 35 | N-Ethyl-4-fluoro-2-(4-iodo-2-methyl-phenylamino)-benzamide | 90-91 |
| 36 | 4-Fluoro-2-(4-iodo-2-methyl-phenylamino)-N,N-dimethyl-benzamide | oil |
| 37 | 4-Fluoro-2-(4-iodo-2-methyl-phenylamino)-N-(1H-tetrazol-5-yl)-benzamide | 285-288 DEC |
| 38 | 5-Bromo-2-(4-iodo-2-methyl-phenylamino)-benzamide | 180-182 |
| 39 | 5-Chloro-2-(4-iodo-2-methyl-phenylamino)-N,N-dimethyl-benzamide | 137-138 |
| 40 | [5-Chloro-2-(4-iodo-2-methyl-phenylamino)-benzoylamino]-acetic acid | 170-173 |
| 41 | 4-Fluoro-2-(4-iodo-2-methyl-phenylamino)-N-propyl-benzamide | 69-71 |
| 42 | 5-Bromo-N-(2-hydroxy-ethyl)-2-(4-iodo-2-methyl-phenylamino)-benzamide | 132-133.4 |
| 43 | N,N-Diethyl-4-fluoro-2-(4-iodo-2-methyl-phenylamino)-benzamide | oil |
| 44 | 4-Fluoro-N-{3-[4-(2-hydroxy-ethyl)-piperazin-1-yl]-propyl}-2-(4-iodo-2-methyl-phenylamino)-benzamide | 122-124 |
| 45 | N,N-Diethyl-2-(4-iodo-2-methyl-phenylamino)-5-nitro-benzamide | 91-93 |
| 46 | N-Butyl-4-fluoro-2-(4-iodo-2-methyl-phenylamino)-benzamide | 97-99 |
| 47 | 5-Chloro-N,N-diethyl-2-(4-iodo-2-methyl-phenylamino)-benzamide | 118-120 |
| 48 | 5-Bromo-2-(4-iodo-2-methyl-phenylamino)-N,N-dimethyl-benzamide | 142.5-144 |

### EXAMPLE 49

### 4-Fluoro-2-(4-iodo-2-methyl-phenylamino)-benzyl alcohol

4-Fluoro-2-(4-iodo-2-methyl-phenylamino)-benzoic acid (0.50 g, 1.35 mmol) was dissolved in 6 mL (6 mmol) of cold 1.0 M borane-tetrahydrofuran complex in tetrahydrofuran solution. The reaction mixture was stirred under nitrogen atmosphere at room temperature overnight. The reaction was quenched with 80 mL of methanol. Concentration in vacuo produced a clear tan oil which was purified by MPLC. Elution with dichloromethane afforded 0.4285 g (89%) of a white solid; mp 99-100.5°C;
¹H NMR (400 MHz; DMSO): δ 7.57 (d, 1H, J=1.7 Hz), 7.45 (dd, 1H, J=8.4, 1.9 Hz), 7.39 (s, 1H), 7.29 (t, 1H, J=7.5 Hz), 6.89 (d, 1H, J=8.4 Hz), 6.67-6.60 (m, 1H), 5.47 (t, 1H, J=5.5 Hz), 4.49 (d, 2H, 5.1 Hz), 2.14 (s, 3H);
IR (KBr) 3372 (O-H stretch) cm⁻¹;
MS (CI) M+1 = 358.
Analysis calculated for C₁₄H₁₃FINO:
C, 47.08; H, 3.67; N, 3.92.
Found: C, 47.17; H, 3.75; N, 3.72.

### EXAMPLE 50-52

The following benzyl alcohols were prepared by the general procedure of Example 49.

| Example No. | Compound | MP °C |
|---|---|---|
| 50 | [5-Chloro-2-(4-iodo-2-methyl-phenylamino)-phenyl]-methanol | 82-85 |
| 51 | [2-(4-Iodo-2-methyl-phenylamino)-5-nitro-phenyl]-methanol | 126.5-128.5 |
| 52 | [5-Bromo-2-(4-iodo-2-methyl-phenylamino)-phenyl]-methanol | 60.5-63.5 |

Several invention compounds of Formula I were prepared utilizing combinatorial synthetic techniques. The general procedure is as follows:
To a 0.8-mL autosampler vial in a metal block was added 40 µL of a 0.5 M solution of the acid in DMF and 40 µL of the reagent amine (2 M solution in Hunig's base and 1 M in amine in DMF). A 0.5 M solution of PyBrop was freshly prepared and 50 µL were added to the autosampler vial. The reaction was allowed to stand for 24 hours.

The reaction mixture was transferred to a 2-dram vial and diluted with 2 mL of ethyl acetate. The organic layer was washed with 3 mL of distilled water and the water layer washed again with 2 mL of ethyl acetate. The combined organic layers were allowed to evaporate to dryness in an open fume hood.

The residue was taken up in 2 mL of 50% acetonitrile in water and injected on a semi-prep reversed phase column (10 mm × 25 cm, 5 µM spherical silica, pore size 115 A derivatized with C-18, the sample was eluted at 4.7 mL/min with a linear ramp to 100% acetonitrile over 8.5 minutes. Elution with 100% acetonitrile continued for 8 minutes). Fractions were collected by monitoring at 214 nM. The residue was dissolved in chloroform and transferred to a preweighed vial, evaporated, and weighed again to determine the yield.

### EXAMPLES 53-206

The following compounds of Formula I were prepared by combinatorial methodology:

| Example No. | Compound | MS M-H |
|---|---|---|
| 53 | 5-Bromo-3,4-difluoro-N-(2-hydroxy-ethyl)-2-(4-iodo-2-methyl-phenylamino)-benzamide | 510 |
| 54 | N-(2,3-Dihydroxy-propyl)-3,4-difluoro-2-(4-iodo-2-methyl-phenylamino)-benzamide | 462 |
| 55 | 5-Bromo-3,4-difluoro-2-(4-iodo-2-methyl-phenylamino)-N-(2-piperidin-1-yl-ethyl)-benzamide | 577 |
| 56 | 3,4-Difluoro-N-(2-hydroxy-ethyl)-2-(4-iodo-2-methyl-phenylamino)-benzamide | 432 |
| 57 | N-(2,3-Dihydroxy-propyl)-4-fluoro-2-(4-iodo-2-methyl-phenylamino)-benzamide | 444 |
| 58 | 3,4-Difluoro-N-(3-hydroxy-propyl)-2-(4-iodo-2-methyl-phenylamino)-benzamide | 446 |
| 59 | 5-Bromo-3,4-difluoro-2-(4-iodo-2-methyl-phenylamino)-N-(2-pyrrolidin-1-yl-ethyl)-benzamide | 564 |
| 60 | 5-Bromo-3,4-difluoro-2-(4-iodo-2-methyl-phenylamino)-N-(2-pyridin-4-yl-ethyl)-benzamide | 571 |
| 61 | 4-Fluoro-N-(2-hydroxy-ethyl)-2-(4-iodo-2-methyl-phenylamino)-benzamide | 414 |
| 62 | 5-Bromo-N-(3-dimethylamino-propyl)-3,4-difluoro-2-(4-iodo-2-methyl-phenylamino)-benzamide | 551 |
| 63 | 5-Bromo-3,4-difluoro-2-(4-iodo-2-methyl-phenylamino)-N-(2-morpholin-4-yl-ethyl)-benzamide | 580 |
| 64 | 3,4-Difluoro-2-(4-iodo-2-methyl-phenylamino)-N-(2-morpholin-4-yl-ethyl)-benzamide | 501 |
| 65 | 3,4-Difluoro-2-(4-iodo-2-methyl-phenylamino)-N-(2-pyrrolidin-1-yl-ethyl)-benzamide | 485 |
| 66 | 3,4-Difluoro-2-(4-iodo-2-methyl-phenylamino)-N-(2-pyridin-4-yl-ethyl)-benzamide | 493 |
| 67 | N-(3-Dimethylamino-propyl)-3,4-difluoro-2-(4-iodo-2-methyl-phenylamino)-benzamide | 473 |
| 68 | N-Benzyl-4-fluoro-2-(4-iodo-2-methyl-phenylamino)-benzamide | 460 |
| 69 | 2-(4-Bromo-2-methyl-phenylamino)-3,4-difluoro-N-(2-hydroxy-ethyl)-benzamide | 384 |
| 70 | 4-Fluoro-2-(4-iodo-2-methyl-phenylamino)-N-(2-morpholin-4-yl-ethyl)-benzamide | 483 |
| 71 | 4-Fluoro-2-(4-iodo-2-methyl-phenylamino)-N-(3-piperidin-1-yl-propyl)-benzamide | 495 |
| 72 | 3,4-Difluoro-2-(4-iodo-2-methyl-phenylamino)-N-(3-piperidin-1-yl-propyl)-benzamide | 513 |
| 73 | 4-Fluoro-2-(4-iodo-2-methyl-phenylamino)-N-(2-thiophen-2-yl-ethyl)-benzamide | 480 |
| 74 | 4-Fluoro-2-(4-iodo-2-methyl-phenylamino)-N-(2-pyrrolidin-1-yl-ethyl)-benzamide | 467 |
| 75 | 2-(4-Bromo-2-methyl-phenylamino)-3,4-difluoro-N-(2-morpholin-4-yl-ethyl)-benzamide | 453 |
| 76 | 5-Bromo-3,4-difluoro-2-(4-iodo-2-methyl-phenylamino)-N-pyridin-4-ylmethyl-benzamide | 557 |
| 77 | 3,4-Difluoro-2-(4-iodo-2-methyl-phenylamino)-N-pyridin-4-ylmethyl-benzamide | 479 |
| 78 | 2-(4-Bromo-2-methyl-phenylamino)-N-(3-dimethylamino-propyl)-3,4-difluoro-benzamide | 425 |
| 79 | 4-Fluoro-2-(4-iodo-2-methyl-phenylamino)-N-pyridin-4-ylmethyl-benzamide | 461 |
| 80 | 4-Fluoro-2-(4-iodo-2-methyl-phenylamino)-N-(2-pyridin-4-yl-ethyl)-benzamide | 475 |
| 81 | 2-(4-Bromo-2-methyl-phenylamino)-3,4-difluoro-N-(2-pyridin-4-yl-ethyl)-benzamide | 445 |
| 82 | 2-(4-Bromo-2-methyl-phenylamino)-3,4-difluoro-N-(3-hydroxy-propyl)-benzamide | 400 |
| 83 | 2-(4-Bromo-2-methyl-phenylamino)-3,4-difluoro-N-(2-pyrrolidin-1-yl-ethyl)-benzamide | 437 |
| 84 | 4-Fluoro-2-(4-iodo-2-methyl-phenylamino)-N-phenethyl-benzamide | 474 |
| 85 | 2-(4-Bromo-2-methyl-phenylamino)-3,4-difluoro-N-(2-thiophen-2-yl-ethyl)-benzamide | 450 |
| 86 | 2-(4-Bromo-2-methyl-phenylamino)-3,4-difluoro-N-pyridin-4-ylmethyl-benzamide | 431 |
| 87 | 2-(4-Bromo-2-methyl-phenylamino)-3,4-difluoro-N-phenethyl-benzamide | 444 |
| 88 | 2-(4-Bromo-2-methyl-phenylamino)-3,4-difluoro-N-(2-piperidin-1-yl-ethyl)-benzamide | 451 |
| 89 | 5-Chloro-N-{3-[4-(2-hydroxy-ethyl)-piperazin-1-yl]-propyl}-2-(4-iodo-2-methyl- phenylamino)- benzamide | 557* |
| 90 | 5-Fluoro-N-{3-[4-(2-hydroxy-ethyl)-piperazin-1-yl]-propyl}-2-(4-iodo-2-methyl- phenylamino)- benzamide | 541* |
| 91 | 2-(4-Iodo-2-methyl-phenylamino)-5-nitro-N-pyridin-4-yl methyl-benzamide | 487 |
| 92 | 5-Bromo-N-{3-[4-(2-hydroxy-ethyl)-piperazin-1-yl]-propyl}-2-(4-iodo-2-methyl- phenylamino)- benzamide | 601* |
| 93 | 5-Chloro-N-(2-diethylamino-ethyl)-2-(4-iodo-2-methyl-phenylamino)- benzamide | 486* |
| 94 | 5-Chloro-2-(4-iodo-2-methyl-phenylamino)N-(2-piperidin-1-yl-ethyl)-benzamide | 497* |
| 95 | (3-Hydroxy-pyrrolidin- -yl)-[2-(4-iodo-2-methyl-phenylamino)-5-nitro-phenyl]-methanone | 466 |
| 96 | 5-Chloro-2-(4-iodo-2-methyl-phenylamino)-N-(2-pyrrolidin-1-yl-ethyl)-benzamide | 484* |
| 97 | 5-Bromo-N-(2-diethylamino-ethyl)-2-(4-iodo-2-methyl-phenylamino)- benzamide | 530* |
| 98 | N-{2-[Bis-(2-hydroxy-ethyl)-amino]-ethyl}-5-chloro-2-(4-iodo-2-methyl- phenylamino)- benzamide | 518* |
| 99 | N-{2-[Bis-(2-hydroxy-ethyl)-amino]-ethyl}-5-bromo-2-(4-iodo-2-methyl- phenylamino)- benzamide | 562* |
| 100 | [5-Bromo-2-(4-iodo-2-methyl-phenylamino)-phenyl]-(3-hydroxy-pyrrolidin-1-yl)-methanone | 499 |
| 101 | 2-(4-Iodo-2-methyl-phenylamino)-5-nitro-benzoic acid phenethyl ester | 501 |
| 102 | N-{3-[4-(2-Hydroxy-ethyl)-piperazin-1-yl]-propyl}-2-(4-iodo-2-methyl-phenylamino)- benzamide | 568* |
| 103 | [5-Chloro-2-(4-iodo-2-methyl-phenylamino)-phenyl]-(3-hydroxy-pyrrolidin-1-yl)-methanone | 455 |
| 104 | 5-Fluoro-2-(4-iodo-2-methyl-phenylamino)-N-pyridin-4-ylmethyl-benzamide | 460 |
| 105 | 5-Bromo-2-(4-iodo-2-methyl-phenylamino)-N-(2-pyrrolidin-1-yl-ethyl)-benzamide | 528* |
| 106 | 5-Bromo-2-(4-iodo-2-methyl-phenylamino)-N-(2-piperidin-1-yl-ethyl)-benzamide | 542* |
| 107 | 5-Fluoro-2-(4-iodo-2-methyl-phenylamino)-N-(2-pyrrolidin-1-yl-ethyl)-benzamide | 468* |
| 108 | S-Chloro-N-(3-dimethylamino-propyl)-2-(4-iodo-2-methyl-phenylamino)-benzamide | 472* |
| 109 | N-{2-[Bis-(2-hydroxy-ethyl)-amino]-ethyl}-5-fluoro-2-(4-iodo-2-methyl- phenylamino)- benzamide | 502* |
| 110 | 5-Chloro-N-(3-hydroxy-propyl)-2-(4-iodo-2-methyl-phenylamino)-benzamide | 445* |
| 111 | 5-Chloro-N-(3-diethylamino-2-hydroxy-propyl)-2-(4-iodo-2-methyl-phenylamino)- benzamide | 516* |
| 112 | 5-Fluoro-2-(4-iodo-2-methyl-phenylamino)-N-(2-piperidin-1-yl-ethyl)-benzamide | 482* |
| 113 | 5-Bromo-N-(3-hydroxy-propyl)-2-(4-iodo-2-methyl-phenylamino)-benzamide | 489* |
| 114 | 5-Bromo-2-(4-iodo-2-methyl-phenylamino)-N-(3-piperidin-1-yl-propyl)-benzamide | 556* |
| 115 | N-{2-[Bis-(2-hydroxy-ethyl)-amino]-ethyl}-2-(4-iodo-2-methyl-phenylamino)-5-nitro- benzamide | 529* |
| 116 | 5-Chloro-2-(4-iodo-2-methyl-phenylamino)-N-(2-morpholin-4-yl-ethyl)-benzamide | 500* |
| 117 | 5-Chloro-N-(3-diethylamino-propyl)-2-(4-iodo-2-methyl-phenylamino)-benzamide | 500* |
| 118 | 5-Chloro-N-(2-diisopropylamino-ethyl)-2-(4-iodo-2-methyl-phenylamino)-benzamide | 514* |
| 119 | 5-Chloro-2-(4-iodo-2-methyl-phenylamino)-N-(3-piperidin-1-yl-propyl)-benzamide | 512* |
| 120 | 2-(4-Iodo-2-methyl-phenylamino)-5-nitro-N-(2-piperidin-1-yl-ethyl)-benzamide | 509* |
| 121 | 5-Bromo-2-(4-iodo-2-methyl-phenylamino)-N-(2-piperazin-1-yl-ethyl)-benzamide | 544* |
| 122 | N-(2-Diethylamino-ethyl)-5-fluoro-2-(4-iodo-2-methyl-phenylamino)-benzamide | 470* |
| 123 | 5-Bromo-N-(3-dimethylamino-propyl)-2-(4-iodo-2-methyl-phenylamino)-benzamide | 516* |
| 124 | N-(3-Hydroxy-propyl)-2-(4-iodo-2-methyl-phenylamino)-5-nitro-benzamide | 456* |
| 125 | 5-Fluoro-N-(3-hydroxy-propyl)-2-(4-iodo-2-methyl-phenylamino)-benzamide | 429* |
| 126 | N-(3-Diethylamino-propyl)-S-fluoro-2-(4-iodo-2-methyl-phenylamino)-benzamide | 484* |
| 127 | N-(3-Diethylamino-propyl)-2-(4-iodo-2-methyl-phenylamino)-5-nitro-benzamide | 511* |
| 128 | 5-Bromo-2-(4-iodo-2-methyl-phenylamino)-N-(2-morpholin-4-yl-ethyl)-benzamide | 544* |
| 129 | 2-(4-Iodo-2-methyl-phenylamino)-5-nitro-N-(3-piperidin-1-yl-propyl)-benzamide | 523* |
| 130 | [5-Fluoro-2-(4-iodo-2-methyl-phenylamino)-phenyl]-(3-hydroxy-pyrrolidin-1-yl)-methanone | 439 |
| 131 | 5-Bromo-N-(2-diisopropylamino-ethyl)-2-(4-iodo-2-methyl-phenylamino)-benzamide | 558* |
| 132 | 5-Fluoro-2-(4-iodo-2-methyl-phenylamino)-N-(2-morpholin-4-yl-ethyl)-benzamide | 484* |
| 133 | 5-Fluoro-2-(4-iodo-2-methyl-phenylamino)-N-(3-piperidin-1-yl-propyl)-benzamide | 496* |
| 134 | [5-Fluoro-2-(4-iodo-2-methyl-phenylamino)-phenyl]-[4-(2-hydroxy-ethyl)-piperazin-1-yl]-methanone | 482 |
| 135 | N-(3-Diethylamino-2-hydroxy-propyl)-5-fluoro-2-(4-iodo-2-methyl-phenylamino)-benzamide | 500* |
| 136 | [5-Chloro-2-(4-iodo-2-methyl-phenylamino)-benzoylamino]-acetic acid | 443 |
| 137 | 2-(4-lodo-2-methyl-phenylamino)-5-nitro-N-(2-pyrrolidin-1-yl-ethyl)-benzamide | 495* |
| 138 | N-(3-Dimethylamino-propyl)-2-(4-iodo-2-methyl-phenylamino)-5-nitro-benzamide | 483* |
| 139 | N-(2-Diisopropylamino-ethyl)-5-fluoro-2-(4-iodo-2-methyl-phenylamino)- benzamide | 498* |
| 140 | 5-Fluoro-2-(4-iodo-2-methyl-phenylamino)-thiobenzoic acid S-phenethyl ester | 490 |
| 141 | 5-Chloro-2-(4-iodo-2-methyl-phenylamino)-thiobenzoic acid S-phenethyl ester | 506 |
| 142 | 5-Bromo-2-(4-iodo-2-methyl-phenylamino)-thiobenzoic acid S-benzyl ester | 536 |
| 143 | 2-(4-Iodo-2-methyl-phenylamino)-5-nitro-thiobenzoic acid S-benzyl ester | 503 |
| 144 | 5-Fluoro-2-(4-iodo-2-methyl-phenylamino)-thiobenzoic acid S-benzyl ester | 476 |
| 145 | 5-Chloro-2-(4-iodo-2-methyl-phenylamino)-thiobenzoic acid S-benzyl ester | 492 |
| 146 | N-Cyclopropyl-5-fluoro-2-(4-iodo-2-methyl-phenylamino)-benzamide | 409 |
| 147 | 5-Chloro-N-(2-hydroxy-ethyl)-2-(4-iodo-2-methyl-phenylamino)-benzamide | 429 |
| 148 | 5-Fluoro-N-(2-hydroxy-ethyl)-2-(4-iodo-2-methyl-phenylamino)-benzamide | 413 |
| 149 | N-Benzyloxy-5-fluoro-2-(4-iodo-2-methyl-phenylamino)-benzamide | 475 |
| 150 | N-Benzyloxy-5-bromo-2-(4-iodo-2-methyl-phenylamino)-benzamide | 593* |
| 151 | 2-(4-Iodo-2-methyl-phenylamino)-5-nitro-N-(4-sulfamoyl-benzyl)-benzamide | 567 |
| 152 | 5-Bromo-N-(2-hydroxy-ethyl)-2-(4-iodo-2-methyl-phenylamino)-benzamide | 473 |
| 153 | N-(2-Hydroxy-ethyl)-5-iodo-2-(4-iodo-2-methyl-phenylamino)-benzamide | 521 |
| 154 | N-(2-Hydroxy-ethyl)-2-(4-iodo-2-methyl-phenylamino)-5-nitro-benzamide | 440 |
| 155 | 2-(4-Iodo-2-methyl-phenylamino)-N-methyl-5-nitro-N-phenyl-benzamide | 486 |
| 156 | 5-Chloro-N-cyclopropyl-2-(4-iodo-2-methyl-phenylamino)-benzamide | 425 |
| 157 | 5-Fluoro-2-(4-iodo-2-methyl-phenylamino)-N-methyl-N-phenyl-benzamide | 459 |
| 158 | N-Allyl-5-fluoro-2-(4-iodo-2-methyl-phenylamino)-benzamide | 409 |
| 159 | N-Benzyloxy-5-iodo-2-(4-iodo-2-methyl-phenylamino)-benzamide | 583 |
| 160 | 5-Fluoro-2-(4-iodo-2-methyl-phenylamino)-N-(4-sulfamoyl-benzyl)-benzamide | 538 |
| 161 | N-Allyl-5-chloro-2-(4-iodo-2-methyl-phenylamino)-benzamide | 425 |
| 162 | N-Cyclopropyl-2-(4-iodo-2-methyl-phenylamino)-5-nitro-benzamide | 436 |
| 163 | 5-Bromo-N-cyclopropyl-2-(4-iodo-2-methyl-phenylamino)-benzamide | 469 |
| 164 | 5-Chloro-2-(4-iodo-2-methyl-phenylamino)-N-methyl-N-phenyl-benzamide | 475 |
| 165 | 5-Iodo-2-(4-iodo-2-methyl-phenylamino)-(4-sulfamoyl-benzyl)-benzamide | 646 |
| 166 | 5-Bromo-2-(4-iodo-2-methyl-phenylamino)-N-(4-sulfamoyl-benzyl)-benzamide | 598 |
| 167 | N-Allyl-2-(4-iodo-2-methyl-phenylamino)-5-nitro-benzamide | 436 |
| 168 | 2-(4-Iodo-2-methyl-phenylamino)-5-nitro-N-(4-sulfamoyl-benzyl)-benzamide | 565 |
| 169 | N-Allyl-5-bromo-2-(4-iodo-2-methyl-phenylamino)-benzamide | 469 |
| 170 | 5-Fluoro-2-(4-iodo-2-methyl-phenylamino)-N-(3-methyl-benzyl)-benzamide | 473 |
| 171 | N-Cyclopropyl-5-iodo-2-(4-iodo-2-methyl-phenylamino)-benzamide | 517 |
| 172 | 5-Bromo-2-(4-iodo-2-methyl-phenylamino)-N-methyl-N-phenyl-benzamide | 519 |
| 173 | N-Benzyloxy-2-(4-iodo-2-methyl-phenylamino)-5-nitro-benzamide | 502 |
| 174 | N-Cyclohexyl-5-iodo-2-(4-iodo-2-methyl-phenylamino)-benzamide | 559 |
| 175 | N-Allyl-5-iodo-2-(4-iodo-2-methyl-phenylamino)-benzamide | 517 |
| 176 | 5-Iodo-2-(4-iodo-2-methyl-phenylamino)-N-(3-methyl-benzyl)-benzamide | 581 |
| 177 | 2-(4-Iodo-2-methyl-phenylamino)-N-(3-methyl-benzyl)-5-nitro-benzamide | 500 |
| 178 | 5-Iodo-2-(4-iodo-2-methyl-phenylamino)-N-methyl-N-phenyl-benzamide | 567 |
| 179 | N-Cyclohexyl-5-fluoro-2-(4-iodo-2-methyl-phenylamino)-benzamide | 451 |
| 180 | 5-Chloro-N-cyclohexyl-2-(4-iodo-2-methyl-phenylamino)-benzamide | 467 |
| 181 | 5-Bromo-2-(4-iodo-2-methyl-phenylamino)-N-(3-methyl-benzyl)-benzamide | 533 |
| 182 | 5-Bromo-N-cyclohexyl-2-(4-iodo-2-methyl-phenylamino)-benzamide | 511 |
| 183 | 5-Chloro-2-(4-iodo-2-methyl-phenylamino)-N-(3-methyl-benzyl)-benzamide | 489 |
| 184 | N-Cyclohexyl-2-(4-iodo-2-methyl-phenylamino)-5-nitro-benzamide | 478 |
| 185 | N-Benzyloxy-5-bromo-2-(4-iodo-2-methyl-phenylamino)-benzamine | 538 |
| 186 | N-Benzyloxy-5-fluoro-2-(4-iodo-2-methyl-phenylamino)-benzamide | 477 |
| 187 | 5-Chloro-N-(2-hydroxy-ethyl)-2-(4-iodo-2-methyl-phenylamino)-benzamide | 431 |
| 188 | 5-Bromo-N-(2-hydroxy-ethyl)-2-(4-iodo-2-methyl-phenylamino)-benzamide | 475 |
| 189 | 2-(4-Iodo-2-methyl-phenylamino)-N-methyl-5-nitro-N-phenyl-benzamide | 488 |
| 190 | 5-Chloro-2-(4-iodo-2-methyl-phenylamino)-N-methyl-N-phenyl-benzamide | 477 |
| 191 | N-(2-Hydroxy-ethyl)-5-iodo-2-(4-iodo-2-methyl-phenylamino)-benzamide | 523 |
| 192 | 5-Chloro-N-cyclopropyl-2-(4-iodo-2-methyl-phenylamino)-benzamide | 425 |
| 193 | N-Allyl-5-chloro-2-(4-iodo-2-methyl-phenylamino)-benzamide | 427 |
| 194 | S-Fluoro-2-(4-iodo-2-methyl-phenylamino)-N-methyl-N-phenyl-benzamide | 461 |
| 195 | N-(2-Hydroxy-ethyl)-2-(4-iodo-2-methyl-phenylamino)-5-nitro-benzamide | 442 |
| 196 | 5-Fluoro-N-(2-hydroxy-ethyl)-2-(4-iodo-2-methyl-phenylamino)-benzamide | 415 |
| 197 | 5-Bromo-N-cyclopropyl-2-(4-iodo-2-methyl-phenylamino)-benzamide | 472 |
| 198 | N-Cyclopropyl-5-fluoro-2-(4-iodo-2-methyl-phenylamino)-benzamide | 411 |
| 199 | 5-Fluoro-2-(4-iodo-2-methyl-phenylamino)-N-(4-sulfamoyl-benzyl)-benzamide | 540 |
| 200 | N-Cyclopropyl-2(4-iodo-2-methyl-phenylamino)-5-nitro-benzamide | 438 |
| 201 | N-Allyl-5-fluoro-2-(4-iodo-2-methyl-phenylamino)-benzamide | 411 |
| 202 | N-Benzyloxy-5-iodo-2-(4-iodo-2-methyl-phenylamino)-benzamide | 585 |
| 203 | N-Allyl-5-bromo-2-(4-iodo-2-methyl-phenylamino)-benzamide | 472 |
| 204 | 5-Bromo-2-(4-iodo-2-methyl-phenylamino)-N-(4-sulfamoyl-benzyl)-benzamide | 601 |
| 205 | 5-Bromo-2-(4-iodo-2-methyl-phenylamino)-N-methyl-N-phenyl-benzamide | 522 |
| 206 | N-Allyl-2-(4-iodo-2-methyl-phenylamino)-5-nitro-benzamide | 438 |

| | | |
|---|---|---|
| * M+H | | |

### EXAMPLE 207

### Preparation of [4-Chloro-2-(1H-tetrazol-5-yl)-(4-iodo-2-methyl-phenyl)-amine

### Step a: Preparation of 5-chloro-2-fluoro-benzaldehyde

To a solution of 1-chloro-4-fluorobenzne (13.06 g, 0.1 mol) in THF (180 mL), at -78°C, LDA (2M solution in THF, 50 mL, 0.1 mol) was added drop wise. After stirring at -78°C for 1.5 hours, DMF (8 mL) was added to the reaction mixture and allowed to warm up to room temperature overnight. The reaction mixture was partitioned between water and Et₂O. The Et₂O layer was dried (MgSO₄) and the solvent removed in vacuum to give 14.95 g (94%) yield of crude aldehyde: ¹H NMR (CDCl₃): δ, 10.3 (s, -C(=O)H).

### Step b: Preparation of 5-chloro-2-fluoro-benzaldehyde oxime

A solution of 5-chloro-2-fluoro-benzaldehyde (10 g, 0.0631 mol), hydroxylamine hydrochloride (6.57 g, 0.0946 mol) and pyridine (8.3 mL, 0.1010 mol) in EtOH (100 mL) was heated at 75°C (oil bath temperature) for 1 hour and the solvent removed under vacuum to give an oil. The oil was partitioned between water and CH₂Cl₂. The CH₂Cl₂ layer was dried (MgSO₄) and the solvent removed under vacuum to give crude aldoxime as a solid. The solid was purified by medium pressure liquid chromatography on silica. Elution with CH₂Cl₂ gave 4.87 g (28%) of the aldoxime as white solid: mp 95-97°C;
Analysis calculated for C₇H₅NOFCl:
C, 48.44; H, 2.90; N, 8.07.
Found: C, 48.55; H, 2.69, N, 7.90.

### Step c: Preparation of 5-chloro-2-fluoro-benzonirile

A solution of the 5-chloro-2-fluoro-benzaldehyde oxime (3.15 g, 0.0182 mol) in acetic anhydride (150 mL) was refluxed for 16 hours. The reaction mixture was cooled to room temperature and poured into saturated aqueous NaHCO₃ (200 mL) solution. The mixture was extracted with Et₂O. The Et₂O layer was dried (K₂CO₃) and the solvent removed to give the product as an oily solid. The product was used without further purification in the next step.

### Step d: Preparation of 5-(5-chloro-2-fluoro-phenyl)-1H-tetrazole

A mixture of 5-chloro-2-fluoro-benzonitrile (2.84 g, 0.01823 mol), butanol (15 mL), sodium azide (1.543 g, 0.0237 mol), acetic acid (1.36 mL, 0.0237 mol) was refluxed for 24 hours. The reaction mixture was cooled to room temperature, additional 1.543 g sodium azide added, and the reaction mixture refluxed for additional 24 hours. After cooling to room temperature, Et₂O (100 mL) and 10% aqueous NaOH (200 mL) were added sequentially. The mixture was vigorously stirred. The aqueous layer was separated, cooled with ice-methanol bath (-15°C) and acidified to pH 1 with conc. HCl. A gray solid precipitated. The solid was dried in vacuum at 50°C to give 1.76 g (49%) of 5-(5-chloro-2-fluoro-phenyl)-1H-tetrazole: mp partial melt at 110°C, complete melting at 124°C);
¹H (400 Mz, CDCl₃): δ 8.19-8.08 (m, 1H), 7.77-7.71 (m, 1H), 7.61-7.52 (m, 1H); ¹³C (100 Mz, CDCl₃): δ 159.00, 156.49, 140.88, 133.02, 132.93, 130.73, 129.23, 129.21, 129.08, 126.05, 118.96, 118.73, 114.50;
MS (CI) M+1 = 199 (100), M = 198 (6).

### Step e: Preparation of [4-Chloro-2-(1H-tetrazol-5-yl)-(4-iodo-2-methyl-phenyl)-amine

To a solution of 2-methyl-4-iodoaniline (3.52 g, 0.0151 mol) in THF (25 mL) at -78°C, LDA (2 molar solution in THF, 11.33 mL, 0.02267 mol) was added dropwise. After stirring for 0.5 hours, a solution of 1-(tetrazol-5-yl)-2-fluoro-5-chlorobenzene (1.5 g, 0.00756 mol) in THF (15 mL) was added dropwise. The reaction was stirred for 16 hours as it warmed up to room temperature. The reaction mixture was quenched with aqueous conc. NH₄Cl solution and extracted with CH₂Cl₂. The organic layer was dried (MgSO₄) and the solvent removed giving a crude product as an oil. The oil with CH₂Cl₂->CH₂Cl₂:MeOH (9.7:0.3) gave 1.5 g (48%) of the desired product:
mp 205-208°C; ¹H (400 Mz, DMSO): δ 9.13 (s, 1H), 8.00-7.99 (s, 1H), 7.69 (s, 1H), 7.55-7.52 (m, 1H), 7.43-7.40 (m, 1H), 7.12-7.05 (m, 1H), 2.24 (s, 3H);
¹³C (100 Mz, CDCl₃): δ 141.87, 139.28, 138.88, 135.47, 133.71, 131.65, 128.15, 123.69, 121.94, 116.68, 87.79, 17.22; MS (CI) M+2 = 413 (44), M+1 = 412 (85), M = 411 (100).
Analysis calculated for C₁₄H₁₁N₅ClI-0.5H₂O:
C, 39.97; H, 2.87; N, 16.65.
Found: C, 38.87, H, 2.77; N, 16.47.

The following tetrazole substituted phenylamines were prepared by following the general procedure of Example 207.

### EXAMPLE 208

### (4-iodo-2-methyl-phenyl)-[2-(1H-tetrazol-5-yl)-phenyl]amine, mp 231°C (dec)

### EXAMPLE 209

### [4-nitro-2-(1H-tetrazol-5-yl)-(4-iodo-2-methyl-phenyl)-amine, mp 205-208°C.

The 4-bromo and 4-iodo phenylamino benzhydroxamic acid derivatives of Formula II can be prepared from commercially available starting materials utilizing synthetic methodologies well-known to those skilled in organic chemistry. A typical synthesis is carried out by reacting a 4-bromo or 4-iodo aniline with a benzoic acid having a leaving group at the 2-position to give a phenylamino benzoic acid, and then reacting the benzoic acid phenylamino derivative with a hydroxylamine derivative (Scheme 3), where L is a leaving group, for example halo such as fluoro, chloro, bromo or iodo, or an activated hydroxy group such as a diethylphosphate, trimethylsilyloxy, p-nitrophenoxy, or phenylsulfonoxy.

The reaction of aniline and the benzoic acid derivative generally is accomplished by mixing the benzoic acid with an equimolar quantity or excess of the aniline in an unreactive organic solvent such as tetrahydrofuran, or toluene, in the presence of a base such as lithium diisopropylamide, n-butyl lithium, sodium hydride, and sodium amide. The reaction generally is carried out at a temperature of about -78°C to about 25°C, and normally is complete within about 2 hours to about 4 days. The product can be isolated by removing the solvent, for example by evaporation under reduced pressure, and further purified, if desired, by standard methods such as chromatography, crystallization, or distillation.

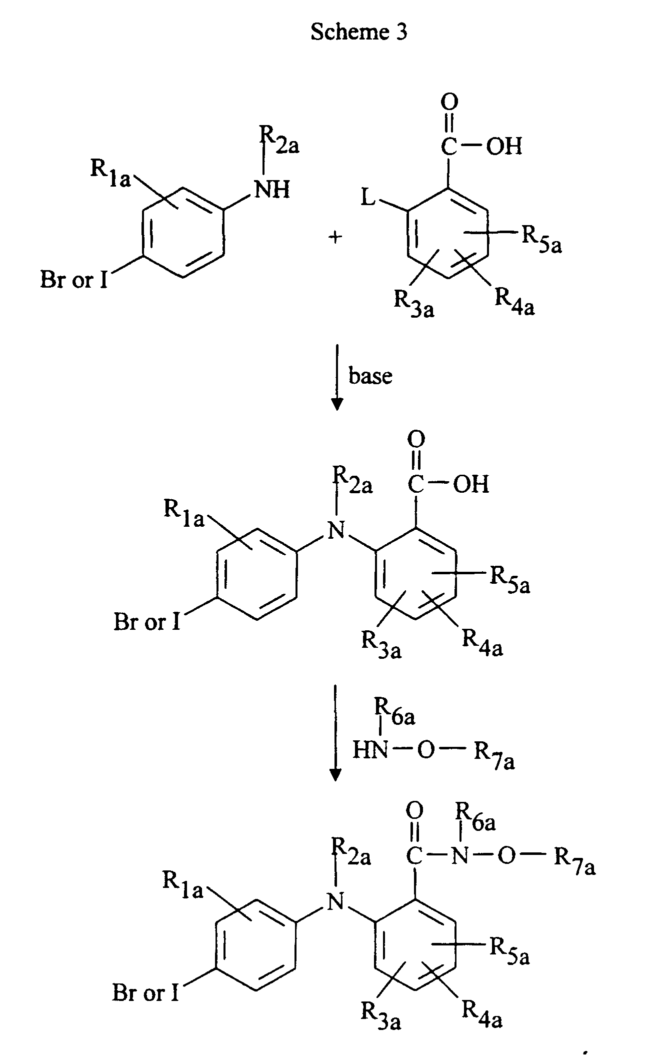

The phenylamino benzoic acid next is reacted with a hydroxylamine derivative HNR₆ₐOR₇ₐ in the presence of a peptide coupling reagent. Hydroxylamine derivatives that can be employed include methoxylamine, N-ethyl-isopropoxy amine, and tetrahydro-oxazine. Typical coupling reagents include 2-ethoxy-1-ethoxycarbonyl-1,2-dihydroquinoline (EEDQ), 1,3-dicyclohexylcarbodiimide (DCC), bromo-tris(pyrrolidino)-phosphonium hexafluorophosphate (PyBrOP) and (benzotriazolyloxy)tripyrrolidino phosphonium hexafluorophosphate (PyBOP). The phenylamino benzoic acid and hydroxylamino derivative normally are mixed in approximately equimolar quantities in an unreactive organic solvent such as dichloromethane, tetrahydrofuran, chloroform, or xylene, and an equimolar quantity of the coupling reagent is added. A base such as triethylamine or diisopropylethylamine can be added to act as an acid scavenger if desired. The coupling reaction generally is complete after about 10 minutes to 2 hours, and the product is readily isolated by removing the reaction solvent, for instance by evaporation under reduced pressure, and purifying the product by standard methods such as chromatography or crystallizations from solvents such as acetone, diethyl ether, or ethanol. An alternative method for making the invention compounds involves first converting a benzoic acid to a hydroxamic acid derivative, and then reacting the hydroxamic acid derivative with an aniline. This synthetic sequence is depicted in Scheme 4, where L is a leaving group. The general reaction conditions for both of the steps in Scheme 4 are the same as those described above for Scheme 3.

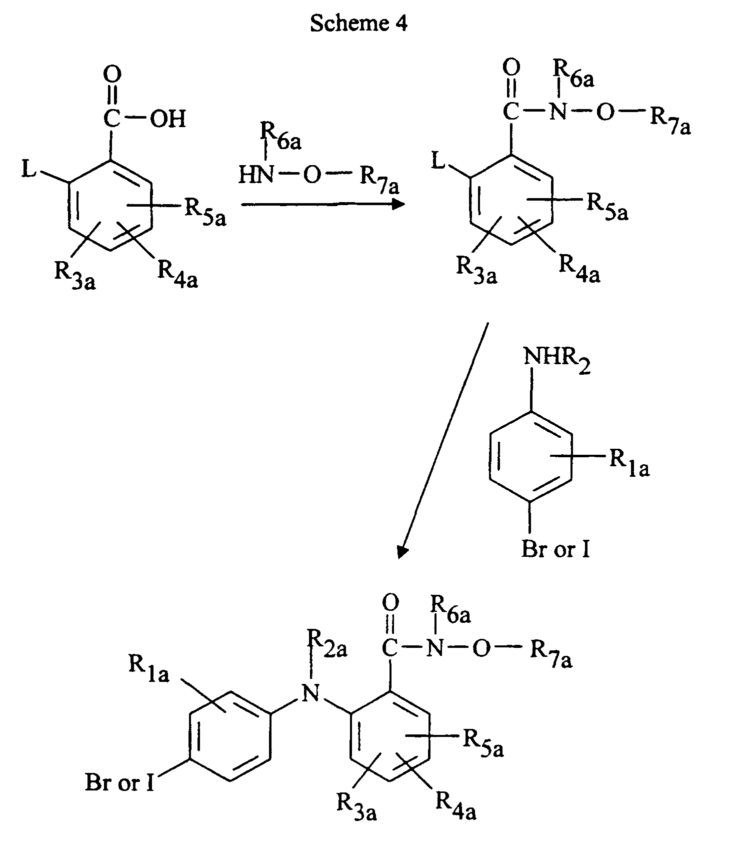

Yet another method for making invention compounds comprises reacting a phenylamino benzhydroxamic acid with an ester forming group as depicted in Scheme 5, where L is a leaving group such as halo, and a base is triethylamine or diisopropylamine.

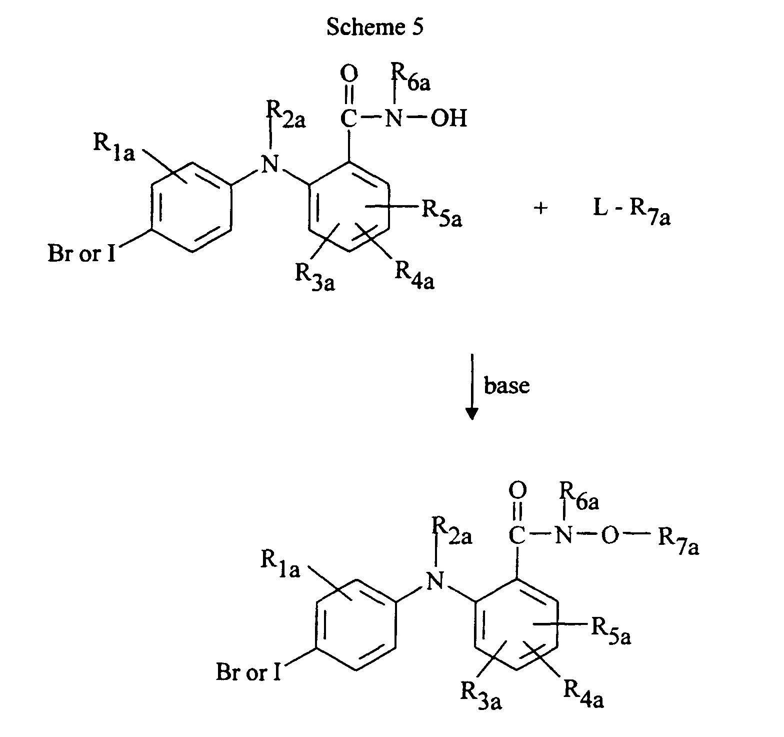

The synthesis of compounds of Formula (II) is further illustrated by the following detailed examples.

### EXAMPLE 1a

### 4-Fluoro-N-hydroxy-2-(4-iodo-2-methyl-phenylamino)-benzamide

### (a) Preparation of 4-Fluoro-2-(4-iodo-2-methyl-phenylamino)-benzoic acid

To a stirred solution containing 3.16 g (0.0133 mol) of 2-amino-5-iodotoluene in 5 mL of tetrahydrofuran at -78°C was added 10 mL (0.020 mol) of a 2.0 M lithium diisopropylamide in tetrahydrofuran/heptane/ethylbenzene (Aldrich) solution. The resulting green suspension was stirred vigorously for 15 minutes, after which time a solution of 1.00 g (0.00632 mol) of 2,4-difluorobenzoic acid in 10 mL of tetrahydrofuran was added. The reaction temperature was allowed to increase slowly to room temperature, at which temperature the mixture was stirred for 2 days. The reaction mixture was concentrated by evaporation of the solvent under reduced pressure. Aqueous HCl (10%) was added to the concentrate, and the solution was extracted with dichloromethane. The organic phase was dried (MgSO₄) and then concentrated over a steambath to low volume (10 mL) and cooled to room temperature. The off-white fibers which formed were collected by vacuum filtration, rinsed with hexane, and dried in a vacuum-oven (76°C; ca. 10 mm of Hg) to afford 1.10 g (47%) of the desired material; mp 224-229.5°C;
¹H NMR (400 MHz, DMSO): δ 9.72 (s, 1H), 7.97 (dd, 1H, J=7.0, 8.7 Hz), 7.70 (d, 1H, J=1.5 Hz), 7.57 (dd, 1H, J=8.4, 1.9 Hz), 7.17 (d, 1H, J=8.2 Hz), 6.61-6.53 (m, 2H), 2.18 (s, 3H);
¹³C NMR (100 MHz, DMSO): δ 169.87, 166.36 (d, J_{C-F}=249.4 Hz), 150.11 (d, J_{C-F}=11.4 Hz), 139.83, 138.49, 136.07, 135.26 (d, J_{C-F}=11.5 Hz), 135.07, 125.60, 109.32, 104.98 (d, J_{C-F}=21.1 Hz), 99.54 (d, J_{C-F}=26.0 Hz), 89.43, 17.52;
¹⁹F NMR (376 MHz, DMSO): δ -104.00 to -104.07 (m);
IR (KBr) 1670 (C=O stretch)cm⁻¹;
MS (CI) M+1 = 372.
Analysis calculated for C₁₄H₁₁ FINO₂:
C, 45.31; H, 2.99; N, 3.77.
Found: C, 45.21; H, 2.77; N, 3.64.

### (b) Preparation of 4-Fluoro-N-hydroxy-2-(4-iodo-2-methyl-phenylamino)-benzamide

To a stirred solution of 4-fluoro-2-(4-iodo-2-methyl-phenylamino)-benzoic acid (0.6495 g, 0.001750 mol), O-(tetrahydro-2H-pyran-2-yl)-hydroxylamine (0.2590 g, 0.002211 mol), and diisopropylethylamine (0.40 mL, 0.0023 mol) in 31 mL of an equivolume tetrahydrofuran-dichloromethane solution was added 1.18 g (0.00227 mol) of solid PyBOP ([benzotriazolyloxy]tripyrrolidino phosphonium hexafluorophosphate, Advanced ChemTech) directly. The reaction mixture was stirred for 30 minutes after which time it was concentrated in vacuo. The brown oil was treated with 10% aqueous hydrochloric acid. The suspension was extracted with ether. The organic extraction was washed with 10% sodium hydroxide followed by another 10% hydrochloric acid wash, was dried (MgSO₄) and concentrated in vacuo to afford 1.0 g of a light-brown foam. This intermediate was dissolved in 25 mL of ethanolic hydrogen chloride, and the solution was allowed to stand at room temperature for 15 minutes. The reaction mixture was concentrated in vacuo to a brown oil that was purified by flash silica chromatography. Elution with a gradient (100 % dichloromethane to 0.6 % methanol in dichloromethane) afforded 0.2284 g of a light-brown viscous oil. Scratching with pentane-hexanes and drying under high vacuum afforded 0.1541 g (23%) of an off white foam; mp 61-75°C;
¹H NMR (400 MHz, DMSO): δ 11.34 (s, 1H), 9.68 (s, 1H), 9.18 (s, 1H), 7.65 (d, 1H, J=1.5 Hz), 7.58 (dd, 1H, J=8.7, 6.8 Hz), 7.52 (dd, 1H, J=8.4, 1.9 Hz), 7.15 (d, 1H, J=8.4 Hz), 6.74 (dd, 1H, J=11.8, 2.4 Hz), 6.62 (ddd, 1H, J=8.4, 8.4, 2.7 Hz), 2.18 (s, 3H);
¹³C NMR (100 MHz, DMSO): δ 165.91, 164.36 (d, J_{C-F}=247.1 Hz), 146.78, 139.18, 138.77, 135.43, 132.64, 130.60 (d, J_{C-F}=11.5 Hz), 122.23, 112.52, 104.72 (d, J=22.1 Hz), 100.45 (d, J_{C-F}=25.2 Hz), 86.77, 17.03;
¹⁹F NMR (376 MHz, DMSO): δ -107.20 to -107.27 (m);
IR (KBr) 3307 (broad, O-H stretch), 1636 (C=O stretch) cm⁻¹;
MS (CI) M+1 = 387.
Analysis calculated for C₁₄H₁₂FIN₂O₂:
C, 43.54; H, 3.13; N, 7.25.
Found: C, 43.62; H, 3.24; N, 6.98.

### EXAMPLE 2a

### 5-Bromo-3,4-difluoro-N-hydroxy-2-(4-iodo-2-methyl-phenylamino)-benzamide

### (a) Preparation of 5-Bromo-2,3,4-trifluorobenzoic acid

To a stirred solution comprised of 1-bromo-2,3,4-trifluorobenzene (Aldrich, 99%; 5.30 g, 0.0249 mol) in 95 mL of anhydrous tetrahydrofuran cooled to -78°C was slowly added 12.5 mL of 2.0 M lithium diisopropylamide in heptane/tetrahydrofuran/ethylbenzene solution (Aldrich). The mixture was stirred for 1 hour and transferred by canula into 700 mL of a stirred saturated ethereal carbon dioxide solution cooled to -78°C. The cold bath was removed, and the reaction mixture was stirred for 18 hours at ambient temperature. Dilute (10%) aqueous hydrochloric acid (ca. 500 mL) was poured into the reaction mixture, and the mixture was subsequently concentrated on a rotary evaporator to a crude solid. The solid product was partitioned between diethyl ether (150 mL) and aq. HCl (330 mL, pH 0). The aqueous phase was extracted with a second portion (100 mL) of diethyl ether, and the combined ethereal extracts were washed with 5% aqueous sodium hydroxide (200 mL) and water (100 mL, pH 12). These combined alkaline aqueous extractions were acidified to pH 0 with concentrated aqueous hydrochloric acid. The resulting suspension was extracted with ether (2 × 200 mL). The combined organic extracts were dried (MgSO₄), concentrated in vacuo, and subjected to high vacuum until constant mass was achieved to afford 5.60 g (88% yield) of an off-white powder; mp 139-142.5°C;
¹H NMR (400 MHz, DMSO): δ 13.97 (broad s, 1H, 8.00-7.96 (m, 1H);
¹³C NMR (100 MHz, DMSO): δ 162.96, 129.34, 118.47, 104.54 (d, J_{C-F}=22.9 Hz);
¹⁹F NMR (376 MHz, DMSO): δ -120.20 to -120.31 (m), -131.75 to -131.86 (m), -154.95 to -155.07 (m);
IR (KBr) 1696 (C=O stretch) cm⁻¹;
MS (CI) M+1 = 255.
Analysis calculated for C₇₄H₂₁ BrF₃O₂:
C, 32.97; H, 0.79; N, 0.00; Br, 31.34; F, 22.35.
Found: C, 33.18; H, 0.64; N, 0.01; Br, 30.14; F, 22.75.

### (b) Preparation of 5-Bromo-3,4-difluoro-2-(4-iodo-2-methyl-phenylamino)-benzoic acid

To a stirred solution comprised of 1.88 g (0.00791 mol) of 2-amino-5-iodotoluene in 10 mL of tetrahydrofuran at -78°C was added 6 mL (0.012 mol) of a 2.0 M lithium diisopropylamide in tetrahydrofuran/heptane/ethylbenzene (Aldrich) solution. The resulting green suspension was stirred vigorously for 10 minutes, after which time a solution of 1.00 g (0.00392 mol) of 5-bromo-2,3,4-trifluorobenzoic acid in 15 mL of tetrahydrofuran was added. The cold bath was subsequently removed, and the reaction mixture stirred for 18 hours. The mixture was concentrated, and the concentrate was treated with 100 mL of dilute (10%) aqueous hydrochloric acid. The resulting suspension was extracted with ether (2 × 150 mL), and the combined organic extractions were dried (MgSO₄) and concentrated in vacuo to give an orange solid. The solid was triturated with boiling dichloromethane, cooled to ambient temperature, and collected by filtration. The solid was rinsed with dichloromethane, and dried in the vacuum-oven (80°C) to afford 1.39 g (76%) of a yellow-green powder; mp 259.5-262°C;
¹H NMR (400 MHz, DMSO): δ 9.03 (s, 1H), 7.99 (dd, 1H, J=7.5, 1.9 Hz), 7.57 (dd, 1H, J=1.5 Hz), 7.42 (dd, 1H, J=8.4, 1.9 Hz), 6.70 (dd, 1H, J=8.4, 6.0 Hz), 2.24 (s, 3H);
¹⁹F NMR (376 MHz, DMSO): δ -123.40 to -123.47 (m); -139.00 to -139.14 (m); IR (KBr) 1667 (C=O stretch)cm⁻¹;
MS (CI) M+1 = 469.
Analysis calculated for C₁₄H₉BrF₂INO₂:
C, 35.93; H, 1.94; N, 2.99; Br, 17.07; F, 8.12; I, 27.11.
Found: C, 36.15; H, 1.91; N, 2.70; Br, 16.40; F, 8.46; I, 26.05.

### (c) Preparation of 5-Bromo-3,4-difluoro-N-hydroxy-2-(4-iodo-2-methylphenylamino)-benzamide

To a stirred solution comprised of 5-bromo-3,4-difluoro-2-(4-iodo-2-methyl-phenylamino)-benzoic acid (0.51 g, 0.0011 mol), O-(tetrahydro-2H-pyran-2-yl)-hydroxylamine (0.15 g, 0.0013 mol), and diisopropylethylamine (0.25 mL, 0.0014 mol) in 20 mL of an equivolume tetrahydrofuran-dichloromethane solution was added 0.6794 g (0.001306 mol) of solid PyBOP (Advanced ChemTech) directly. The reaction mixture was stirred at 24°C for 10 minutes, and then was concentrated to dryness in vacuo. The concentrate was suspended in 100 mL of 10% aqueous hydrochloric acid. The suspension was extracted with 125 mL of diethyl ether. The ether layer was separated, washed with 75 mL of 10% aqueous sodium hydroxide, and then with 100 mL of dilute acid. The ether solution was dried (MgSO₄) and concentrated in vacuo to afford 0.62 g (100%) of an off-white foam. The foam was dissolved in ca. 15 mL of methanolic hydrogen chloride. After 5 minutes, the solution was concentrated in vacuo to an oil, and the oil was purified by flash silica chromatography. Elution with dichloromethane: dichloromethane-methanol (99:1) afforded 0.2233 g (42%) of a yellow powder. The powder was dissolved in diethyl ether and washed with dilute hydrochloric acid. The organic phase was dried (MgSO₄) and concentrated in vacuo to afford 0.200 g of a foam. This product was triturated with pentane to afford 0.1525 g of a powder that was repurified by flash silica chromatography. Elution with dichloromethane afforded 0.0783 g (15%) of an analytically pure title compound, mp 80-90°C;
¹H NMR (400 MHz, DMSO): δ 11.53 (s, 1H), 9.38 (s, 1H), 8.82 (s, 1H), 7.70 (dd, 1H, J=7.0, 1.9 Hz), 7.53 (s, 1H), 7.37 (dd, 1H, J=8.4, 1.9 Hz), 6.55 (dd, 1H, J=8.2, 6.5 Hz), 2.22 (s, 3H);
¹⁹F NMR (376 MHz, DMSO): δ -126.24 to -126.29 (m), -137.71 to -137.77 (m);
IR (KBr) 3346 (broad, O-H stretch), 1651 (C=O stretch)cm⁻¹;
MS (CI) M+1 = 484.
Analysis calculated for C₁₄H₁₀BrF₂IN₂O₂:
C, 34.81; H, 2.09; N, 5.80.
Found: C, 34.53; H, 1.73; N, 5.52.

Examples 3a to 12a in the table below were prepared by the general procedure of Examples 1a and 2a.

### EXAMPLES 13a-77a

Examples 13a to 77a were prepared utilizing combinatorial synthetic methodology by reacting appropriately substituted phenylamino benzoic acids (e.g., as shown in Scheme 1) and hydroxylamines (e.g., (NHR₆ₐ)-O-R₇ₐ. A general method is given below:
To a 0.8-mL autosampler vial in a metal block was added 40 µL of a 0.5 M solution of the acid in DMF and 40 µL of the hydroxylamine (2 M solution in Hunig's base and 1 M in amine in DMF). A 0.5 M solution of PyBrOP was freshly prepared, and 50 µL were added to the autosampler vial. The reaction was allowed to stand for 24 hours.
The reaction mixture was transferred to a 2-dram vial and diluted with 2 mL of ethyl acetate. The organic layer was washed with 3 mL of distilled water and the water layer washed again with 2 mL of ethyl acetate. The combined organic layers were allowed to evaporate to dryness in an open fume hood.
The residue was taken up in 2 mL of 50% acetonitrile in water and injected on a semi-prep reversed phase column (10 mm × 25 cm, 5 µM spherical silica, pore Size 115 A derivatized with C-18, the sample was eluted at 4.7 mL/min with a linear ramp to 100% acetonitrile over 8.5 minutes. Elution with 100% acetonitrile continued for 8 minutes.) Fractions were collected by monitoring at 214 nM. The desired fractions were evaporated using a Zymark Turbovap. The product was dissolved in chloroform and transferred to a preweighed vial, evaporated, and weighed again to determine the yield. The structure was confirmed by mass spectroscopy.

### EXAMPLES 3a-77a

| Example No. | Compound | Melting Point (°C) | MS (M-H⁺) |
|---|---|---|---|
| 3a | 2-(4-bromo-2-methyl-phenylamino)-4-fluoro-N-hydroxy-benzamide | 56-75 dec | 523 |
| 4a | 5-Chloro-N-hydroxy-2-(4-iodo-2-methyl-phenylamino)-benzamide | 65 dec | |
| 5a | 5-Chloro-N-hydroxy-2-(4-iodo-2-methyl-phenylamino)-N-methyl-benzamide | 62-67 | |
| 6a | 5-Chloro-2-(4-iodo-2-methyl-phenylamino)-N-(terahydropyran-2-yloxy)benzamide | 105-108 | |
| 7a | 5-Chloro-2-(4-iodo-2-methyl-phenylamino)-N-methoxybenzamide | 64-68 | |
| 8a | 4-Fluoro-N-hydroxy-2-(4-fluoro-2-methyl-phenylamino)-benzamide | 119-135 | |
| 9a | 4-Fluoro-N-hydroxy-2-(2-methyl phenylamino)-benzamide | 101-103 | |
| 10a | 4-Fluoro-2-(4-fluor-2-methyl-phenylamino)-N-(terahydropyran-2-yloxy)benzamide | 142-146 | |
| 11a | 4-Fluoro-N-hydroxy-2-(4-cluoro-2-methyl-phenylamino)-benzamide | 133.5-135 | |
| 12a | 4-Fluoro-2-(4-iodo-2-methyl-phenylamino)-N-phenylmethoxy-benzamide | 107-109.5 | |
| 13a | 4-Fluoro-2-(4-iodo-2-methyl-phenylamino)-N-methoxy-benzamide | | 399 |
| 14a | 3,4-Difluoro-2-(4-iodo-2-methyl-phenylamino)-N-methoxy-benzamide | | 417 |
| 15a | 2-(4-Bromo-2-methyl-phenylamino)-3,4-difluoro-N-methoxy-benzamide | | 369 |
| 16a | 2-(4-Bromo-2-methyl-phenylamino)-N-ethoxy-3,4-difluoro-benzamide | | 342* (M-EtO) |
| 17a | 5-Bromo-N-ethoxy-3,4-difluoro-2-(4-iodo-2-methyl-phenylamino)-benzamide | | 509 |
| 18a | 3,4-Difluoro-2-(4-iodo-2-methyl-phenylamino)-N-isopropoxy-benzamide | | 445 |
| 19a | 2-(4-Bromo-2-methyl-phenylamino)-3,4-difluoro-N-isopropoxy-benzamide | | 397 |
| 20a | 4-Fluoro-N-(furan-3-ylmethoxy)-2-(4-iodo-2-methyl-phenylamino)-benzamide | | 465 |
| 21a | 3,4-Difluoro-N-(furan-3-ylmethoxy)-2-(4-iodo-2-methyl-phenylamino)-benzamide | | 483 |
| 22a | 2-(4-Bromo-2-methyl-phenylamino)-3,4-difluoro-N-(furan-3-ylmethoxy)-benzamide | | 435 |
| 23a | 5-Bromo-3,4-difluoro-N-(furan-3-ylmethoxy)-2-(4-iodo-2-methyl-phenylamino)-benzamide | | 561 |
| 24a | 5-Bromo-N-(but-2-enyloxy)-3,4-difluoro-2-(4-iodo-2-methyl-phenylamino)-benzamide | | 536 |
| 25a | 4-Fluoro-2-(4-iodo-2-methyl-phenylamino)-N-(prop-2-ynyloxy)-benzamide | | 423 |
| 26a | 3,4-Difluoro-2-(4-iodo-2-methyl-phenylamino)-N-(prop-2-ynyloxy)-benzamide | | 441 |
| 27a | 3,4-Difluoro-2-(4-iodo-2-methyl-phenylamino)-N-(1-methyl-prop-2-ynyloxy)-benzamide | | 455 |
| 28a | 2-(4-Bromo-2-methyl-phenylamino)-3,4-difluoro-N-(1-methyl-prop-2-ynyloxy)-benzamide | | 407 |
| 29a | N-(But-3-ynyloxy)-3,4-difluoro-2-(4-iodo-2-methyl-phenylamino)-benzamide | | 455 |
| 30a | 2-(4-Bromo-2-methyl-phenylamino)-N-(but-3-ynyloxy)-3,4-difluoro-benzamide | | 407 |
| 31a | 5-Bromo-N-(but-3-ynyloxy)-3,4-difluoro-2-(4-iodo-2-methyl-phenylamino)-benzamide | | 533 |
| 32a | 3,4-Difluoro-2-(4-iodo-2-methyl-phenylamino)-N-(3-phenyl-prop-2-ynyloxy)-benzamide | | 517 |
| 33a | 3,4-Difluoro-2-(4-bromo-2-methyl-phenylamino)-N-(3-phenyl-prop-2-ynyloxy)-benzamide | | 469 |
| 34a | 3,4-Difluoro-N-[3-(3-fluoro-phenyl)-prop-2-ynyloxy]-2-(4-iodo-2-methyl-phenylamino)-benzamide | | 535 |
| 35a | 2-(4-Bromo-2-methyl-phenylamino)-3,4-difluoro-N-[3-(3-fluoro-phenyl)-prop-2-ynyloxy]-benzamide | | 487 |
| 36a | 3,4-Difluoro-N-[3-(2-fluoro-phenyl)-prop-2-ynyloxy]-2-(4-iodo-2-methyl-phenylamino)-benzamide | | 535 |
| 37a | 5-Bromo-3,4-difluoro-N-[3-(2-fluoro-phenyl)-prop-2-ynyloxy]-2-(4-iodo-2-methyl-phenylamino)-benzamide | | 613 |
| 38a | 3,4-Difluoro-2-(4-iodo-2-methyl-phenylamino)-N-(3-methyl-5-phenyl-pent-2-en-4-ynyloxy)-benzamide | | 557* *(M+H) |
| 39a | 2-(4-Bromo-2-methyl-phenylamino)-3,4-difluoro-N-(3-methyl-5-phenyl-pent-2-en-4-ynyloxy)-benzamide | | 510 |
| 40a | N-Ethoxy-3,4-difluoro-2-(4-iodo-2-methyl-phenylamino)-benzamide | | 431 |
| 41a | 2-(4-Bromo-2-methyl-phenylamino)-N-ethoxy-3,4-difluoro-benzamide | | 383 |
| 42a | 4-Fluoro-2-(4-iodo-2-methyl-phenylamino)-N-propoxy-benzamide | | 427 |
| 43a | 3,4-Difluoro-2-(4-iodo-2-methyl-phenylamino)-N-propoxy-benzamide | | 445 |
| 44a | 2-(4-Bromo-2-methyl-phenylamino)-3,4-difluoro-N-propoxy-benzamide | | 397 |
| 45a | 5-Bromo-3,4-difluoro-2-(4-iodo-2-methyl-phenylamino)-N-propoxy-benzamide | | 523 |
| 46a | 4-Fluoro-2-(4-iodo-2-methyl-phenylamino)-N-isopropoxy-benzamide | | 427 |
| 47a | 3,4-Difluoro-2-(4-iodo-2-methyl-phenylamino)-N-isopropoxy-benzamide | | 445 |
| 48a | 2-(4-Bromo-2-methyl-phenylamino)-3,4-difluoro-N-isopropoxy-benzamide | | 397 |
| 49a | 5-Bromo-3,4-difluoro-2-(4-iodo-2-methyl-phenylamino)-N-isopropoxy-benzamide | | 523 |
| 50a | N-Cyclobutyloxy-3,4-difluoro-2-(4-iodo-2-methyl-phenylamino)-benzamide | | 457 |
| 51a | 2-(4-Bromo-2-methyl-phenylamino)-N-cyclobutyloxy-3,4-difluoro-benzamide | | 409 |
| 52a | N-Cyclopentyloxy-4-fluoro-2-(4-iodo-2-methyl-phenylamino)-benzamide | | 453 |
| 53a | N-Cyclopentyloxy-3,4-difluoro-2-(4-iodo-2-methyl-phenylamino)-benzamide | | 471 |
| 54a | 2-(4-Bromo-2-methyl-phenylamino)-N-cyclopentyloxy-3,4-difluoro-benzamide | | 423 |
| 55a | N-Cyclopropylmethoxy-4-fluoro-2-(4-iodo-2-methyl-phenylamino)-benzamide | | 439 |
| 56a | N-Cyclopropylmethoxy-3,4-difluoro-2-(4-iodo-2-methyl-phenylamino)-benzamide | | 457 |
| 57a | 2-(4-Bromo-2-methyl-phenylamino)-N-cyclopropylmethoxy-3,4-difluoro-benzamide | | 409 |
| 58a | 5-Bromo-N-cyclopropylmethoxy-3,4-difluoro-2-(4-iodo-2-methyl-phenylamino) | | 435 |
| 59a | 4-Fluoro-2-(4-iodo-2-methyl-phenylamino)-N-(2-phenoxy-ethoxy)-benzamide | | 505 |
| 60a | 3,4-Difluoro-2-(4-iodo-2-methyl-phenylamino)-N-(2-phenoxy-ethoxy)-benzamide | | 523 |
| 61 a | 2-(4-Bromo-2-methyl-phenylamino)-3,4-difluoro-N-(2-phenoxy-ethoxy)-benzamide | | 475 |
| 62a | 4-Fluoro-2-(4-iodo-2-methyl-phenylamino)-N-(thiophen-2-ylmethoxy)-benzamide | | 481 |
| 63a | 3,4-Difluoro-2-(4-iodo-2-methyl-phenylamino)-N-(thiophen-2-ylmethoxy)-benzamide | | 499 |
| 64a | 2-(4-Bromo-2-methyl-phenylamino)-3,4-difluoro-N-(thiophen-2-ylmethoxy)-benzamide | | 451 |
| 65a | 4-Fluoro-2-(4-iodo-2-methyl-phenylamino)-N-(2-methyl-allyloxy)-benzamide | | 439 |
| 66a | 3,4-Difluoro-2-(4-iodo-2-methyl-phenylamino)-N-(2-methyl-allyloxy)-benzamide | | 457 |
| 67a | 2-(4-Bromo-2-methyl-phenylamino)-3,4-difluoro-N-(2-methyl-allyloxy)-benzamide | | 410 |
| 68a | N-(But-2-enyloxy)-4-fluoro-2-(4-iodo-2-methyl-phenylamino)-benzamide | | 439 |
| 69a | N-(But-2-enyloxy)-3,4-difluoro-2-(4-iodo-2-methyl-phenylamino)-benzamide | | 457 |
| 70a | 2-(4-Bromo-2-methyl-phenylamino)-N-(but-2-enyloxy)-3,4-difluoro-benzamide | | 410 |
| 71a | 3,4-Difluoro-2-(4-iodo-2-methyl-phenylamino)-N-(prop-2-ynyloxy)-benzamide | | 441 |
| 72a | N-(But-3-ynyloxy)-3,4-difluoro-2-(4-iodo-2-methyl-phenylamino)-benzamide | | 455 |
| 73a | 2-(4-Bromo-2-methyl-phenylamino)-N-(4,4-dimethyl-pent-2-ynyloxy)-3,4-difluoro-benzamide | | 449 |
| 74a | N-(But-2-enyloxy)-3,4-difluoro-2-(4-iodo-2-methyl-phenylamino)-benzamide | | 457 |
| 75a | 2-(4-Bromo-2-methyl-phenylamino)-N-(but-2-enyloxy)-3,4-difluoro-benzamide | | 410 |
| 76a | N-(3-tert-butyl-propyn-2-yl)oxy-4-fluoro-2-(4-iodo-2-methyl-phenylamino)-benzamide | | 479 |
| 77a | 4-Fluoro-2-(4-iodo-2-methyl-phenylamino)-N-phenylmethoxy-benzamide | | 577* *CI |

### D. Pharmacological Activity

The anticancer activity of the combinations provided by this invention has been evaluated in standard assays designed to measure anticancer utility. In a typical cell culture assay using colon 26 carcinoma cells, paclitaxel in combination with a MEK inhibitor proved to be more efficacious than either agent alone, thus establishing a surprising synergistic effect. The colon 26 carcinoma cells were originally collected from a mouse that had undergone surgery to remove the infected section of the colon, and are now readily available from Southern Research Institute (Birmingham, Alabama, USA). The cells were cultured to approximately 80% confluency on Day 0 of the assay. At 72 hours after the 80% confluency was established, dimethylsulfoxide (DMSO) was added to one set of cells to act as untreated controls. Paclitaxel at concentrations of 30 nM and 100 nM was added to other sets of cells. All of the cells were incubated at 38°C for 48 hours, at which time MEK inhibitor 2-(2-chloro-4-iodophenylamino)-N-cyclopropylmethoxy-3,4-difluorobenzamide (PD184352), at a concentration of 1.0 micromolar, was added to one set of the DMSO control cells, and to the cells containing the two concentrations of paclitaxel. All cells were again incubated for an additional 48-hour period. The cells were harvested from the growth medium, and were fixed in ethanol. The cells were then treated with FITC (fluorescein isothiocyanate)-labeled phalloidin (Sigma). Binding of phalloidin-FITC to depolymerized actin thereby serves as a measure of apoptosis. Propidium iodide was also added to the treated and control cells for the purpose of staining all cells. The extent of apoptosis of tumor cells was measured by flow cytometry analysis.

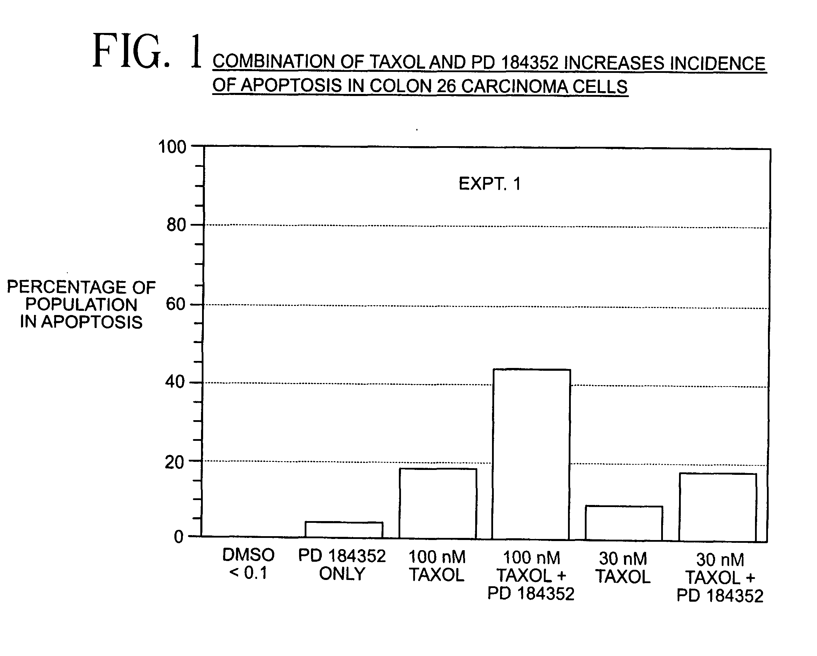

Figure 1 shows the results of the foregoing assay. The data establish that the vehicle alone (DMSO) caused no effect on apoptosis (programmed cell death) of the colon 26 carcinoma cells. The MEK inhibitor caused about 5% increase of apoptosis at 30 nM, and paclitaxel caused about 18% increase at 100 nM, and about 9% increase at 30 nM. Surprisingly, the combination of MEK inhibitor and paclitaxel (at 100 nM) caused a dramatic 44% incidence in the programmed cell death of the carcinoma cells. At the 30 nM concentration of paclitaxel, the combination caused about an 18 % incidence in apoptosis. These results establish the combination of MEK inhibitors and paclitaxel provided by this invention is surprisingly effective at killing cancer cells, and accordingly is useful to treat patients suffering from cancer and in need of treatment.

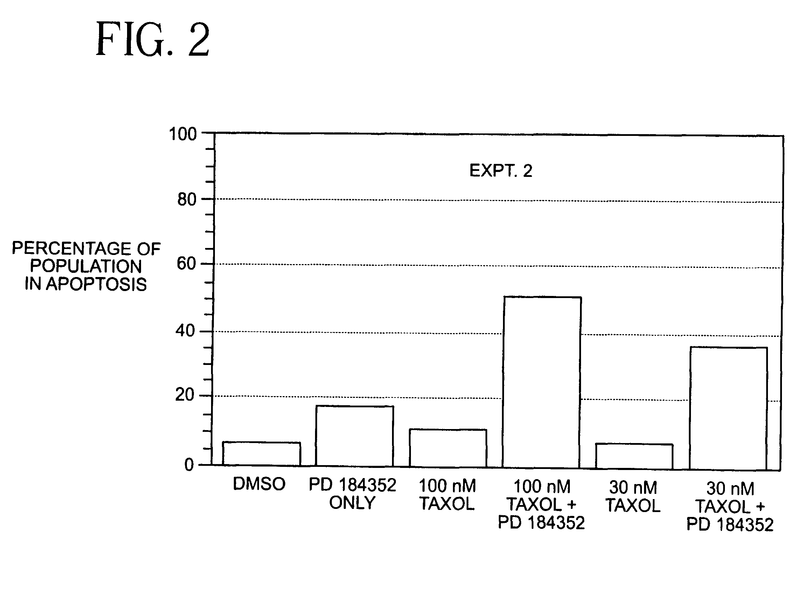

The assay described above was repeated, and the results (see Figure 2) confirmed that the combinations of this invention are useful to treat and control cancer. In this second study, DMSO did cause measurable cell death, somewhat similar to that observed with the 30 nM concentration of paclitaxel alone. The MEK inhibitor 2-(2-chloro-4-iodophenylamino)-N-cyclopropylmethoxy-3,4-difluorobenzamide (PD184352) caused about an 18% incidence in apoptosis when administered alone, and paclitaxel caused only about an 11% incidence when administered at 100 nM alone. As in the assay results discussed above, the combination of MEK inhibitor and paclitaxel caused a dramatic and unexpected increase in cancer cell death. These results further establish the antitumor activity of the combinations provided by this invention.

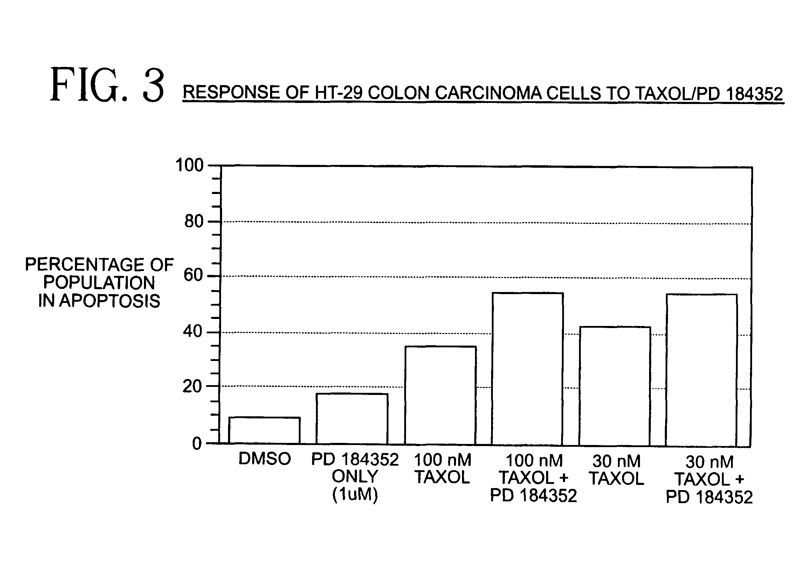

Another cell culture assay was carried out using HT-29 colon carcinoma cells. Paclitaxel and 2-(2-chloro-4-iodophenylamino)-N-cyclopropylmethoxy-3,4-difluorobenzamide (PD 184352) were evaluated for their effect on apoptosis alone and in combination (see Figure 3). Again, the combination of mitotic agent and selective MEK inhibitor proved to be more efficacious than using either agent alone.

Further support for the claims of the present invention was provided by the use of non-small cell lung carcinoma cells (A549) in culture using the protocol used previously for the colon cell lines. In this case, only one set of experiments was performed and repetition is planned. The tumor line treated with Taxol alone showed a much higher incidence of apoptosis than the colon lines (41% at 10 nM Taxol). Ten nanomolar Taxol with 1 micromolar PD 184352 gave a 47% incidence in apoptosis (6% increase). The A549 cells appear to be quite sensitive to Taxol alone.

## Claims

1. An anticancer combination which comprises a mitotic inhibitor and a MEK inhibitor wherein the MEK inhibitor is a phenyl amine compound of Formula I: 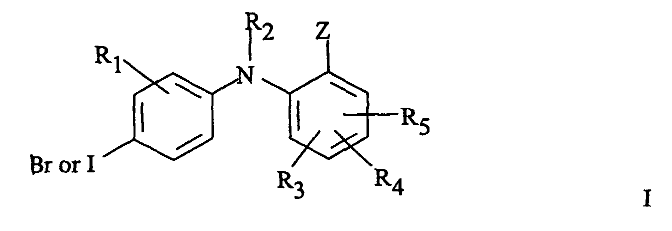 wherein:
R₁ is hydrogen, hydroxy, C₁-C₈ alkyl, C₁-C₈ alkoxy, halo, trifluoromethyl, or CN;
R₂ is hydrogen;
R₃, R₄, and R₅ independently are hydrogen, hydroxy, halo, trifluoromethyl, C₁-C₈ alkyl, C₁-C₈ alkoxy, nitro, CN, or -(O or NH)ₘ-(CH₂)ₙ-R₉, where R₉ is hydrogen, hydroxy, COOH, or NR₁₀R₁₁;
n is 0-4;
m is 0 or 1;
R₁₀ and R₁₁ independently are hydrogen or C₁-C₈ alkyl, or taken together with the nitrogen to which they are attached can complete a 3-10 member cyclic ring optionally containing 1, 2, or 3 additional heteroatoms selected from O, S, NH, or N-C₁-C₈ alkyl;
Z is COOR₇, tetrazolyl, CONR₆R₇, CONHNR₁₀R₁₁, or CH₂OR₇;
R₆ and R₇ independently are hydrogen, C₁-C₈ alkyl, C₂-C₈ alkenyl, C₂-C₈ alkynyl, (CO)-C₁-C₈ alkyl, aryl, heteroaryl, C₃-C₁₀ cycloalkyl, or C₃-C₁₀ cycloalkyl (optionally containing 1, 2, or 3 heteroatoms selected from O, S, NH, or N alkyl); or R₆ and R₇ together with the nitrogen to which they are attached complete a 3-10 member cyclic ring optionally containing 1, 2, or 3 additional heteroatoms selected from O, S, NH, or N alkyl;
and wherein any of the foregoing alkyl, alkenyl, aryl, heteroaryl, heterocyclic, and alkynyl groups can be unsubstituted or substituted by halo, hydroxy, C₁-C₆ alkoxy, amino, nitro, C₁-C₄ alkylamino, di(C₁-C₄)alkylamino, C₃-C₆ cycloalkyl, phenyl, phenoxy, C₃-C₅ heteroaryl or heterocyclic radical, or C₃-C₅ heteroaryloxy or heterocyclic radical-oxy;
or a pharmaceutically acceptable salt thereof.

2. The combination according to Claim 1 wherein the mitotic inhibitor is selected from paclitaxel, docetaxel, vincristine, vinblastine, vinorelbine, and vinflunine.

3. The combination according to Claim 1 wherein the MEK inhibitor is a phenyl amine selected from:
[4-Chloro-2-(1H-tetrazol-5-yl)-phenyl(4-iodo-2-methyl-phenyl)-amine;
(4-Iodo-2-methyl-phenyl)-[2-(1H-tetrazol-5-yl)-phenyl]amine;
[4-Nitro-2-(1H-tetrazol-5-yl)-phenyl-(4-iodo-2-methyl-phenyl)-amine;
4-Fluoro-2-(4-iodo-2-methylphenylamino)benzoic acid;
3,4,5-Trifluoro-2-(4-iodo-2-methyl-phenylamino)-benzoic acid;
3,4-Difluoro-2-(4-iodo-2-methyl-phenylamino)-benzoic acid;
5-Bromo-3,4-difluoro-2-(4-iodo-2-methyl-phenylamino)-benzoic acid;
5-Chloro-2-(4-iodo-2-methyl-phenylamino)-benzoic acid;
Sodium 5-Chloro-2-(4-iodo-2-methyl-phenylamino)-benzoate;
5-Bromo-2-(4-iodo-2-methyl-phenylamino)-benzoic acid;
2-(4-Iodo-2-methyl-phenylamino)-5-nitro-benzoic acid;
4-Chloro-2-(4-iodo-2-methyl-phenylamino)-benzoic acid;
2-(4-Iodo-2-methyl-phenylamino)-benzoic acid;
5-Fluoro-2-(4-iodo-2-methyl-phenylamino)-benzoic acid;
5-Iodo-2-(4-iodo-2-methyl-phenylamino)-benzoic acid;
2,3,5-Trifluoro-4-(4-iodo-2-methyl-phenylamino)-benzoic acid;
2-(4-Iodo-phenylamino)-5-methoxy-benzoic acid;
5-Methyl-2-(4-iodo-2-methyl-phenylamino)-benzoic acid;
2-(4-Iodo-2-methyl-phenylamino)-4-nitro-benzoic acid;
2-(4-Bromo-2-methyl-phenylamino)-4-fluoro-benzoic acid;
2-(2-Bromo-4-iodo-phenylamino)-5-nitro-benzoic acid;
2-(4-Bromo-2-methyl-phenylamino)-3,4-difluoro-benzoic acid;
5-Chloro-N-(2-hydroxyethyl)-2-(4-iodo-2-methyl-phenylamino)-benzamide;
4-Fluoro-2-(4-iodo-2-methyl-phenylamino)-benzamide;
4-Fluoro-2-(4-iodo-2-methyl-phenylamino)-N-methyl-benzamide;
N-Ethyl-4-fluoro-2-(4-iodo-2-methyl-phenylamino)-benzamide;
4-Fluoro-2-(4-iodo-2-methyl-phenylamino)-N,N-dimethyl-benzamide;
4-Fluoro-2-(4-iodo-2-methyl-phenylamino)-N-(1H-tetrazol-5-yl)-benzamide;
5-Bromo-2-(4-iodo-2-methyl-phenylamino)-benzamide;
5-Chloro-2-(4-iodo-2-methyl-phenylamino)-N,N-dimethyl-benzamide;
[5-Chloro-2-(4-iodo-2-methyl-phenylamino)-benzoylamino]-acetic acid;
4-Fluoro-2-(4-iodo-2-methyl-phenylamino)-N-propyl-benzamide;
5-Bromo-N-(2-hydroxy-ethyl)-2-(4-iodo-2-methyl-phenylamino)-benzamide;
N,N-Diethyl-4-fluoro-2-(4-iodo-2-methyl-phenylamino)-benzamide;
4-Fluoro-N-{3-[4-(2-hydroxy-ethyl)-piperazin-1-yl]-propyl}-2-(4-iodo-2-methyl-phenylamino)-benzamide;
N,N-Diethyl-2-(4-iodo-2-methyl-phenylamino)-5-nitro-benzamide;
N-Butyl-4-fluoro-2-(4-iodo-2-methyl-phenylamino)-benzamide;
5-Chloro-N,N-diethyl-2-(4-iodo-2-methyl-phenylamino)-benzamide;
5-Bromo-2-(4-iodo-2-methyl-phenylamino)-N,N-dimethyl-benzamide;
5-Bromo-3,4-difluoro-N-(2-hydroxy-ethyl)-2-(4-iodo-2-methylphenylamino)-benzamide;
N-(2,3-Dihydroxy-propyl)-3,4-difluoro-2-(4-iodo-2-methyl-phenylamino)-benzamide;
5-Bromo-3,4-difluoro-2-(4-iodo-2-methyl-phenylamino)-N-(2-piperidin-1-yl-ethyl)-benzamide;
3,4-Difluoro-N-(2-hydroxy-ethyl)-2-(4-iodo-2-methyl-phenylamino)-benzamide;
N-(2,3-Dihydroxy-propyl)-4-fluoro-2-(4-iodo-2-methyl-phenylamino)-benzamide;
3,4-Difluoro-N-(3-hydroxy-propyl)-2-(4-iodo-2-methyl-phenylamino)-benzamide;
5-Bromo-3,4-difluoro-2-(4-iodo-2-methyl-phenylamino)-N-(2-pyrrolidin-1-yl-ethyl)-benzamide;
5-Bromo-3,4-difluoro-2-(4-iodo-2-methyl-phenylamino)-N-(2-pyridin-4-yl-ethyl)-benzamide;
4-Fluoro-N-(2-hydroxy-ethyl)-2-(4-iodo-2-methyl-phenylamino)-benzamide;
5-Bromo-N-(3-dimethylamino-propyl)-3,4-difluoro-2-(4-iodo-2-methylphenylamino)-benzamide;
5-Bromo-3,4-difluoro-2-(4-iodo-2-methyl-phenylamino)-N-(2-morpholin-4-yl-ethyl)-benzamide;
3,4-Difluoro-2-(4-iodo-2-methyl-phenylamino)-N-(2-morpholin-4-yl-ethyl)-benzamide;
3,4-Difluoro-2-(4-iodo-2-methyl-phenylamino)-N-(2-pyrrolidin-1-yl-ethyl)-benzamide;
3,4-Difluoro-2-(4-iodo-2-methyl-phenylamino)-N-(2-pyridin-4-yl-ethyl)-benzamide;
N-(3-Dimethylamino-propyl)-3,4-difluoro-2-(4-iodo-2-methylphenylamino)-benzamide;
N-Benzyl-4-fluoro-2-(4-iodo-2-methyl-phenylamino)-benzamide;
2-(4-Bromo-2-methyl-phenylamino)-3,4-difluoro-N-(2-hydroxy-ethyl)-benzamide;
4-Fluoro-2-(4-iodo-2-methyl-phenylamino)-N-(2-morpholin-4-yl-ethyl)-benzamide;
4-Fluoro-2-(4-iodo-2-methyl-phenylamino)-N-(3-piperidin-1-yl-propyl)-benzamide;
3,4-Difluoro-2-(4-iodo-2-methyl-phenylamino)-N-(3-piperidin-1-yl-propyl)-benzamide;
4-Fluoro-2-(4-iodo-2-methyl-phenylamino)-N-(2-thiophen-2-yl-ethyl)-benzamide;
4-Fluoro-2-(4-iodo-2-methyl-phenylamino)-N-(2-pyrrolidin-1-yl-ethyl)-benzamide;
2-(4-Bromo-2-methyl-phenylamino)-3,4-difluoro-N-(2-morpholin-4-yl-ethyl)-benzamide;
5-Bromo-3,4-difluoro-2-(4-iodo-2-methyl-phenylamino)-N-pyridin-4-ylmethyl-benzamide;
3,4-Difluoro-2-(4-iodo-2-methyl-phenylamino)-N-pyridin-4-ylmethyl-benzamide;
2-(4-Bromo-2-methyl-phenylamino)-N-(3-dimethylaminopropyl)-3,4-difluoro-benzamide;
4-Fluoro-2-(4-iodo-2-methyl-phenylamino)-N-pyridin-4-ylmethyl-benzamide;
4-Fluoro-2-(4-iodo-2-methyl-phenylamino)-N-(2-pyridin-4-yl-ethyl)-benzamide;
2-(4-Bromo-2-methyl-phenylamino)-3,4-difluoro-N-(2-pyridin-4-yl-ethyl)-benzamide;
2-(4-Bromo-2-methyl-phenylamino)-3,4-difluoro-N-(3-hydroxy-propyl)-benzamide;
2-(4-Bromo-2-methyl-phenylamino)-3,4-difluoro-N-(2-pyrrolidin-1-yl-ethyl)-benzamide;
4-Fluoro-2-(4-iodo-2-methyl-phenylamino)-N-phenethyl-benzamide;
2-(4-Bromo-2-methyl-phenylamino)-3,4-difluoro-N-(2-thiophen-2-yl-ethyl)-benzamide;
2-(4-Bromo-2-methyl-phenylamino)-3,4-difluoro-N-pyridin-4-ylmethyl-benzamide;
2-(4-Bromo-2-methyl-phenylamino)-3,4-difluoro-N-phenethyl-benzamide;
2-(4-Bromo-2-methyl-phenylamino)-3,4-difluoro-N-(2-piperidin-1-yl-ethyl)-benzamide;
5-Chloro-N-{3-[4-(2-hydroxy-ethyl)-piperazin-1-yl]-propyl}-2-(4-iodo-2-methyl-phenylamino)-benzamide;
5-Fluoro-N-{3-[4-(2-hydroxy-ethyl)-piperazin-1-yl]-propyl}-2-(4-iodo-2-methyl-phenylamino)-benzamide;
2-(4-Iodo-2-methyl-phenylamino)-5-nitro-N-pyridin-4-yl methyl-benzamide;
5-Bromo-N-{3-[4-(2-hydroxy-ethyl)-piperazin-1-yl]-propyl}-2-(4-iodo-2-methyl-phenylamino)-benzamide;
5-Chloro-N-(2-diethylamino-ethyl)-2-(4-iodo-2-methyl-phenylamino)-benzamide;
5-Chloro-2-(4-iodo-2-methyl-phenylamino)-N-(2-piperidin-1-yl-ethyl)-benzamide;
(3-Hydroxy-pyrrolidin-1-yl)-[2-(4-iodo-2-methyl-phenylamino)-5-nitrophenyl];
5-Chloro-2-(4-iodo-2-methyl-phenylamino)-N-(2-pyrrolidin-1-yl-ethyl)-benzamide;
5-Bromo-N-(2-diethylamino-ethyl)-2-(4-iodo-2-methyl-phenylamino)-benzamide;
N- {2-[Bis-(2-hydroxy-ethyl)-amino]-ethyl}-5-chloro-2-(4-iodo-2-methylphenylamino)-benzamide;
N-{2-[Bis-(2-hydroxy-ethyl)-amino]-ethyl}-5-bromo-2-(4-iodo-2-methylphenylamino)-benzamide;
N-{3-[4-(2-Hydroxy-ethyl)-piperazin-1-yl]-propyl}-2-(4-iodo-2-methylphenylamino)-benzamide;
5-Fluoro-2-(4-iodo-2-methyl-phenylamino)-N-pyridin-4-ylmethyl-benzamide;
5-Bromo-2-(4-iodo-2-ethyl-phenylamino)-N-(2-pyrrolidin-1-yl-ethyl)-benzamide;
5-Bromo-2-(4-iodo-2-methyl-phenylamino)-N-(2-piperidin-1-yl-ethyl)-benzamide;
5-Fluoro-2-(4-iodo-2-methyl-phenylamino)-N-(2-pyrrolidin-1-yl-ethyl)-benzamide;
5-Chloro-N-(3-dimethylamino-propyl)-2-(4-iodo-2-methyl-phenylamino)-benzamide;
N-{2-[Bis-(2-hydroxy-ethyl)-amino]-ethyl}-5-fluoro-2-(4-iodo-2-methylphenylamino)-benzamide;
5-Chloro-N-(3-hydroxy-propyl)-2-(4-iodo-2-methyl-phenylamino)-benzamide;
5-Chloro-N-(3-diethylamino-2-hydroxy-propyl)-2-(4-iodo-2-methylphenylamino)-benzamide;
5-Fluoro-2-(4-iodo-2-methyl-phenylamino)-N-(2-piperidin-1-yl-ethyl)-benzamide;
5-Bromo-N-(3-hydroxy-propyl)-2-(4-iodo-2-methyl-phenylamino)-benzamide;
5-Bromo-2-(4-iodo-2-methyl-phenylamino)-N-(3-piperidin-1-yl-propyl)-benzamide;
N-{2-[Bis-(2-hydroxy-ethyl)-amino]-ethyl}-2-(4-iodo-2-methylphenylamino)-5-nitro-benzamide;
5-Chloro-2-(4-iodo-2-methyl-phenylamino)-N-(2-morpholin-4-yl-ethyl)-benzamide;
5-Chloro-N-(3-diethylamino-propyl)-2-(4-iodo-2-methyl-phenylamino)-benzamide;
5-Chloro-N-(2-diisopropylamino-ethyl)-2-(4-iodo-2-methylphenylamino)-benzamide;
5-Chloro-2-(4-iodo-2-methyl-phenylamino)-N-(3-piperidin-1-yl-propyl)-benzamide;
2-(4-Iodo-2-methyl-phenylamino)-5-nitro-N-(2-piperidin-1-yl-ethyl)-benzamide;
5-Bromo-2-(4-iodo-2-methyl-phenylamino)-N-(2-piperazin-1-yl-ethyl)-benzamide;
N-(2-Diethylamino-ethyl)-5-fluoro-2-(4-iodo-2-methyl-phenylamino)-benzamide;
5-Bromo-N-(3-dimethylamino-propyl)-2-(4-iodo-2-methyl-phenylamino)-benzamide;
N-(3-Hydroxy-propyl)-2-(4-iodo-2-methyl-phenylamino)-5-nitro-benzamide;
5-Fluoro-N-(3-hydroxy-propyl)-2-(4-iodo-2-methyl-phenylamino)-benzamide;
N-(3-Diethylamino-propyl)-5-fluoro-2-(4-iodo-2-methyl-phenylamino)-benzamide;
N-(3-Diethylamino-propyl)-2-(4-iodo-2-methyl-phenylamino)-5-nitro-benzamide;
5-Bromo-2-(4-iodo-2-methyl-phenylamino)-N-(2-morpholin-4-yl-ethyl)-benzamide;
2-(4-Iodo-2-methyl-phenylamino)-5-nitro-N-(3-piperidin-1-yl-propyl)-benzamide;
[5-Fluoro-2-(4-iodo-2-methyl-phenylamino)-phenyl]-(3-hydroxy-pyrrolidin-1-yl)-methanone
5-Bromo-N-(2-diisopropylamino-ethyl)-2-(4-iodo-2-methylphenylamino)-benzamide;
5-Fluoro-2-(4-iodo-2-methyl-phenylamino)-N-(2-morpholin-4-yl-ethyl)-benzamide;
5-Fluoro-2-(4-iodo-2-methyl-phenylamino)-N-(3-piperidin-1-yl-propyl)-benzamide;
[5-Fluoro-2-(4-iodo-2-methyl-phenylamino)-phenyl]-[4-(2-hydroxyethyl)-piperazin-1-yl]-methanone;
N-(3-Diethylamino-2-hydroxy-propyl)-5-fluoro-2-(4-iodo-2-methylphenylamino)-benzamide;
N-Cyclopropyl-5-fluoro-2-(4-iodo-2-methyl-phenylamino)-benzamide;
5-Chloro-N-(2-hydroxy-ethyl)-2-(4-iodo-2-methyl-phenylamino)-benzamide;
5-Fluoro-N-(2-hydroxy-ethyl)-2-(4-iodo-2-methyl-phenylamino)-benzamide;
N-Benzyloxy-5-fluoro-2-(4-iodo-2-methyl-phenylamino)-benzamide;
N-Benzyloxy-5-bromo-2-(4-iodo-2-methyl-phenylamino)-benzamide;
2-(4-Iodo-2-methyl-phenylamino)-5-nitro-N-(4-sulfamoyl-benzyl)-benzamide;
5-Bromo-N-(2-hydroxy-ethyl)-2-(4-iodo-2-methyl-phenylamino)-benzamide;
N-(2-Hydroxy-ethyl)-5-iodo-2-(4-iodo-2-methyl-phenylamino)-benzamide;
N-(2-Hydroxy-ethyl)-2-(4-iodo-2-ethyl-phenylamino)-5-nitro-benzamide;
2-(4-Iodo-2-methyl-phenylamino)-N-methyl-5-nitro-N-phenyl-benzamide;
5-Chloro-N-cyclopropyl-2-(4-iodo-2-methyl-phenylamino)-benzamide;
5-Fluoro-2-(4-iodo-2-methyl-phenylamino)-N-methyl-N-phenyl-benzamide;
N-Allyl-5-fluoro-2-(4-iodo-2-methyl-phenylamino)-benzamide;
N-Benzyloxy-5-iodo-2-(4-iodo-2-methyl-phenylamino)-benzamide;
5-Fluoro-2-(4-iodo-2-methyl-phenylamino)-N-(4-sulfamoyl-benzyl)-benzamide;
N-Allyl-5-chloro-2-(4-iodo-2-methyl-phenylamino)-benzamide;
N-Cyclopropyl-2-(4-iodo-2-methyl-phenylamino)-5-nitro-benzamide;
5-Bromo-N-cyclopropyl-2-(4-iodo-2-methyl-phenylamino)-benzamide;
5-Chloro-2-(4-iodo-2-methyl-phenylamino)-N-methyl-N-phenyl-benzamide;
5-Iodo-2-(4-iodo-2-methyl-phenylamino)-N-(4-sulfamoyl-benzyl)-benzamide;
5-Bromo-2-(4-iodo-2-methyl-phenylamino)-N-(4-sulfamoyl-benzyl)-benzamide;
N-Allyl-2-(4-iodo-2-methyl-phenylamino)-5-nitro-benzamide;
2-(4-Iodo-2-methyl-phenylamino)-5-nitro-N-(4-sulfamoyl-benzyl)-benzamide;
N-Allyl-5-bromo-2-(4-iodo-2-methyl-phenylamino)-benzamide;
5-Fluoro-2-(4-iodo-2-methyl-phenylamino)-N-(3-methyl-benzyl)-benzamide;
N-Cyclopropyl-5-iodo-2-(4-iodo-2-methyl-phenylamino)-benzamide;
5-Bromo-2-(4-iodo-2-methyl-phenylamino)-N-methyl-N-phenyl-benzamide;
N-Benzyloxy-2-(4-iodo-2-methyl-phenylamino)-5-nitro-benzamide;
N-Cyclohexyl-5-iodo-2-(4-iodo-2-methyl-phenylamino)-benzamide;
N-Allyl-5-iodo-2-(4-iodo-2-methyl-phenylamino)-benzamide;
5-Iodo-2-(4-iodo-2-methyl-phenylamino)-N-(3-methyl-benzyl)-benzamide;
2-(4-Iodo-2-methyl-phenylamino)-N-(3-methyl-benzyl)-5-nitro-benzamide;
5-Iodo-2-(4-iodo-2-methyl-phenylamino)-N-methyl-N-phenyl-benzamide;
N-Cyclohexyl-5-fluoro-2-(4-iodo-2-methyl-phenylamino)-benzamide;
5-Chloro-N-cyclohexyl-2-(4-iodo-2-methyl-phenylamino)-benzamide;
5-Bromo-2-(4-iodo-2-methyl-phenylamino)-N-(3-methyl-benzyl)-benzamide;
5-Bromo-N-cyclohexyl-2-(4-iodo-2-methyl-phenylamino)-benzamide;
5-Chloro-2-(4-iodo-2-methyl-phenylamino)-N-(3-methyl-benzyl)-benzamide;
N-Cyclohexyl-2-(4-iodo-2-methyl-phenylamino)-5-nitro-benzamide;
N-Benzyloxy-5-bromo-2-(4-iodo-2-methyl-phenylamino)-benzamide;
N-Benzyloxy-5-fluoro-2-(4-iodo-2-methyl-phenylamino)-benzamide;
5-Chloro-N-(2-hydroxy-ethyl)-2-(4-iodo-2-methyl-phenylamino)-benzamide;
5-Bromo-N-(2-hydroxy-ethyl)-2-(4-iodo-2-methyl-phenylamino)-benzamide;
2-(4-Iodo-2-methyl-phenylamino)-N-methyl-5-nitro-N-phenyl-benzamide;
5-Chloro-2-(4-iodo-2-methyl-phenylamino)-N-methyl-N-phenyl-benzamide;
N-(2-Hydroxy-ethyl)-5-iodo-2-(4-iodo-2-methyl-phenylamino)-benzamide;
5-Chloro-N-cyclopropyl-2-(4-iodo-2-methyl-phenylamino)-benzamide;
N-Allyl-5-chloro-2-(4-iodo-2-methyl-phenylamino)-benzamide;
5-Fluoro-2-(4-iodo-2-methyl-phenylamino)-N-methyl-N-phenyl-benzamide;
N-(2-Hydroxy-ethyl)-2-(4-iodo-2-methyl-phenylamino)-5-nitro-benzamide;
5-Fluoro-N-(2-hydroxy-ethyl)-2-(4-iodo-2-methyl-phenylamino)-benzamide;
5-Bromo-N-cyclopropyl-2-(4-iodo-2-methyl-phenylamino)-benzamide;
N-Cyclopropyl-5-fluoro-2-(4-iodo-2-methyl-phenylamino)-benzamide;
5-Fluoro-2-(4-iodo-2-methyl-phenylamino)-N-(4-sulfamoyl-benzyl)-benzamide;
N-Cyclopropyl-2-(4-iodo-2-methyl-phenylamino)-5-nitro-benzamide;
N-Allyl-5-fluoro-2-(4-iodo-2-methyl-phenylamino)-benzamide;
N-Benzyloxy-5-iodo-2-(4-iodo-2-methyl-phenylamino)-benzamide;
N-Allyl-5-bromo-2-(4-iodo-2-methyl-phenylamino)-benzamide;
5-Bromo-2-(4-iodo-2-methyl-phenylamino)-N-(4-sulfamoyl-benzyl)-benzamide;
5-Bromo-2-(4-iodo-2-methyl-phenylamino)-N-methyl-N-phenyl-benzamide;
N-Allyl-2-(4-iodo-2-methyl-phenylamino)-5 -nitro-benzamide;
4-Fluoro-2-(4-iodo-2-methyl-phenylamino)-benzyl alcohol;
[5-Chloro-2-(4-iodo-2-methyl-phenylamino)-phenyl]-methanol;
[2-(4-Iodo-2-methyl-phenylamino)-5-nitro-phenyl]-methanol;
[5-Bromo-2-(4-iodo-2-methyl-phenylamino)-phenyl]-methanol; and
N-Allyl-2-(4-iodo-2-methyl-phenylamino)-5-nitro-benzamide.

4. An anticancer combination which comprises a mitotic inhibitor and a MEK inhibitor wherein the MEK inhibitor is a phenyl amine compound of Formula II: 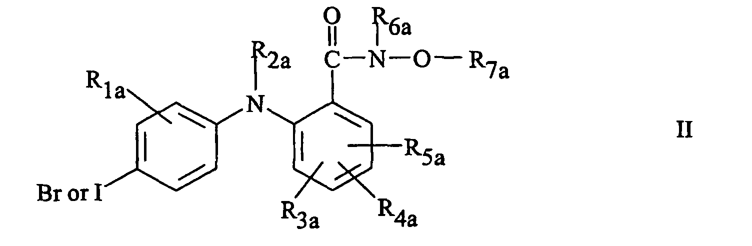 wherein:
R₁ₐ is hydrogen, hydroxy, C₁-C₈ alkyl, C₁-C₈ alkoxy, halo, trifluoromethyl, or CN;
R₂ₐ is hydrogen;
R₃ₐ, R₄ₐ, and R₅ₐ independently are hydrogen, hydroxy, halo, trifluoromethyl, C₁-C₈ alkyl, C₁-C₈ alkoxy, nitro, CN, or (O or NH)ₘ-(CH₂)ₙ-R₉ₐ, where R₉ₐ is hydrogen, hydroxy, CO₂H or NR₁₀ₐR₁₁ₐ.
n is 0-4;
m is 0 or 1;
R₁₀ₐ and R₁₁ₐ independently are hydrogen or C₁-C₈ alkyl, or taken together with the nitrogen to which they are attached can complete a 3- to 10-member cyclic ring optionally containing one, two, or three additional heteroatoms selected from O, S, NH, or N-C₁-C₈ alkyl;
R₆ₐ is hydrogen, C₁-C₈ alkyl, (CO)-C₁-C₈ alkyl, aryl, aralkyl, or C₃-C₁₀ cycloalkyl;
R₇ₐ is hydrogen, C₁-C₈ alkyl, C₂-C₈ alkenyl, C₂-C₈ alkynyl, C₃-C₁₀ cycloalkyl or cycloalkyl (optionally containing a heteroatom selected from O, S, or NR₉ₐ);
and wherein any of the foregoing alkyl, alkenyl, aryl, heteroaryl, heterocyclic, and alkynyl groups can be unsubstituted or substituted by halo, hydroxy, C₁-C₆ alkoxy, amino, nitro, C₁-C₄ alkylamino, di(C₁-C₄)alkylamino, C₃-C₆ cycloalkyl, phenyl, phenoxy, C₃-C₅ heteroaryl or heterocyclic radical, or C₃-C₅ heteroaryloxy or heterocyclic radical-oxy; or R₆ₐ and R₇ₐ taken together with the N to which they are attached can complete a 5- to 10-membered cyclic ring, optionally containing one, two, or three additional heteroatoms selected from O, S, or NR₁₀ₐR₁₁ₐ; or a pharmaceutically acceptable salt thereof.

5. An anticancer combination comprising a mitotic inhibitor and a selective MEK 1 or MEK 2 inhibitor selected from:
4-Fluoro-N-hydroxy-2-(4-iodo-2-methyl-phenylamino)-benzamide;
4-Fluoro-2-(4-iodo-2-methyl-phenylamino) N-(methoxy)-benzamide;
4-Fluoro-2-(4-iodo-2-methyl-phenylamino)-N-(prop-2-ynyloxy)-benzamide;
4-Fluoro-2-(4-iodo-2-methyl-phenylamino)-N-(2-phenoxyethoxy)-benzamide;
4-Fluoro-2-(4-iodo-2-methyl-phenylamino)-N-(2-thienylmethoxy)-benzamide;
4-Fluoro-2-(4-iodo-2-methyl-phenylamino)-N-(prop-2-enyloxy)-benzamide;
4-Fluoro-2-(4-iodo-2-methyl-phenylamino)-N-(cyclopropylmethoxy)-benzamide;
4-Fluoro-2-(4-iodo-2-methyl-phenylamino)-N-(cyclopentoxy)-benzamide;
3,4-Difluoro-2-(4-iodo-2-methyl-phenylamino)-N-(3-furylmethoxy)-benzamide;
3,4-Difluoro-2-(4-iodo-2-methyl-phenylamino)-N-ethoxy-benzamide;
3,4-Difluoro-2-(4-iodo-2-methyl-phenylamino)-N-(but-2-enyloxy)-benzamide;
3,4-Difluoro-2-(4-iodo-2-methyl-phenylamino)-N-(cyclopropylmethoxy)-benzamide;
3,4-Difluoro-2-(4-iodo-2-methyl-phenylamino)-N-(1-methylprop-2-ynyloxy)-benzamide;
3,4-Difluoro-2-(4-iodo-2-methyl-phenylamino)-N-(3-phenylprop-2-ynyloxy)-benzamide;
3,4-Difluoro-2-(4-iodo-2-methyl-phenylamino)-N-(3-methyl-5-phenylpent-2-en-4-ynyloxy)-benzamide;
3,4-Difluoro-2-(4-iodo-2-methyl-phenylamino)-N-(prop-2-ynyloxy)-benzamide;
3,4-Difluoro-2-(4-iodo-2-methyl-phenylamino)-N-(propoxy)-benzamide;
3,4-Difluoro-2-(4-iodo-2-methyl-phenylamino)-N-(cyclobutyloxy)-benzamide;
3,4-Difluoro-2-(4-iodo-2-methyl-phenylamino)-N-(2-thienylmethoxy)-benzamide;
3,4-Difluoro-2-(4-iodo-2-methyl-phenylamino)-N-(2-methyl-prop-2-enyloxy)-benzamide;
3,4-Difluoro-2-(4-iodo-2-methyl-phenylamino)-N-(2-phenoxyethoxy)-benzamide;
3,4-Difluoro-2-(4-iodo-2-methyl-phenylamino)-N-(but-2-enyloxy)-benzamide;
3,4-Difluoro-2-(4-iodo-2-methyl-phenylamino)-N-(but-3-ynyloxy)-benzamide;
3,4-Difluoro-2-(4-iodo-2-methyl-phenylamino)-N-(cyclopentyloxy)-benzamide;
3,4-Difluoro-2-(4-iodo-2-methyl-phenylamino)-N-(3-(2-fluorophenyl)-prop-2-ynyloxy)-benzamide;
5-Bromo-3,4-difluoro-N-hydroxy-2-(4-iodo-2-methyl-phenylamino)-benzamide;
5-Bromo-3,4-difluoro-2-(4-iodo-2-methyl-phenylamino)-N-(n-propoxy)-benzamide;
5-Bromo-3,4-difluoro-N-(furan-3-ylmethoxy)-2-(4-iodo-2-methylphenylamino)-benzamide;
5-Bromo-N-(but-2-enyloxy)-3,4-difluoro-2-(4-iodo-2-methylphenylamino)-benzamide
5-Bromo-N-butoxy-3,4-difluoro-2-(4-iodo-2-methyl-phenylamino)-benzamide;
5-Bromo-3,4-difluoro-2-(4-iodo-2-methyl-phenylamino)-N-(3-methyl-but-2-enyloxy)-benzamide;
5-Bromo-3,4-difluoro-2-(4-iodo-2-methyl-phenylamino)-N-(3-methyl-pent-2-en-4-ynyloxy)-benzamide;
5-Bromo-3,4-difluoro-2-(4-iodo-2-methyl-benzyl)-N-[5-(3-methoxyphenyl)-3-methyl-pent-2-en-4-ynyloxy]-benzamide;
5-Bromo-3,4-difluoro-2-(4-iodo-2-methyl-phenylamino)-N-(prop-2-ynyloxy)-benzamide;
5-Bromo-3,4-difluoro-2-(4-iodo-2-methyl-phenylamino)-N-[3-(3-methoxy-phenyl)-prop-2-ynyloxy]-benzamide;
5-Bromo-3,4-difluoro-2-(4-iodo-2-methyl-phenylamino)-N-(thiopen-2-ylmethoxy)-benzamide;
5-Bromo-3,4-difluoro-2-(4-iodo-2-methyl-phenylamino)-N-(pyridin-3-ylmethoxy)-benzamide;
5-Bromo-3-4-difluoro-2-(4-iodo-2-methyl-phenylamino)-N-(3-(2-fluorophenyl)-prop-2-ynyloxy)-benzamide;
5-Bromo-3,4-difluoro-2-(4-iodo-2-methyl-phenylamino)-N-(ethoxy)-benzamide;
5-Bromo-3,4-difluoro-2-(4-iodo-2-methyl-phenylamino)-N-(cyclopropylmethoxy)-benzamide;
5-Bromo-3,4-difluoro-2-(4-iodo-2-methyl-phenylamino)-N-(isopropoxy)-benzamide;
5-Bromo-3,4-difluoro-2-(4-iodo-2-methyl-phenylamino)-N-but-3-ynyloxy)-benzamide;
5-Chloro-N-hydroxy-2-(4-iodo-2-methyl-phenylamino)-benzamide;
5-Chloro-2-(4-iodo-2-methyl-phenylamino)-N-(tetrahydro-pyran-2-yloxy)-benzamide;
5-Chloro-2-(4-iodo-2-methyl-phenylamino)-N-methoxy-benzamide;
4-Bromo-2-(4-iodo-2-methyl-phenylamino)-N-phenylmethoxy-benzamide;
4-Fluoro-2-(4-iodo-2-methyl-phenylamino)-N-phenylmethoxy-benzamide;
5-Fluoro-N-hydroxy-2-(4-iodo-2-methyl-phenylamino)-benzamide;
5-Iodo-2-(4-iodo-2-methyl-phenylamino)-N-phenylmethoxy-benzamide;
5-Fluoro-2-(4-iodo-2-methyl-phenylamino)-N-(tetrahydropyran-2-yloxy)-benzamide;
3,4-Difluoro-2-(4-bromo-2-methyl-phenylamino)-N-(3-phenylprop-2-ynyloxy)-benzamide;
3,4-Difluoro-2-(4-bromo-2-methyl-phenylamino)-N-(3-furylmethoxy)-benzamide;
3,4-Difluoro-2-(4-bromo-2-methyl-phenylamino)-N-(2-thienylmethoxy)-benzamide;
3,4-Difluoro-2-(4-bromo-2-methyl-phenylamino)-N-(but-3-ynyloxy)-benzamide;
3,4-Difluoro-2-(4-bromo-2-methyl-phenylamino)-N-(2-methyl-prop-2-enyloxy)-benzamide;
3,4-Difluoro-2-(4-bromo-2-methyl-phenylamino)-N-(but-2-enyloxy)-benzamide;
3,4-Difluoro-2-(4-bromo-2-methyl-phenylamino)-N-(methoxy)-benzamide;
3,4-Difluoro-2-(4-bromo-2-methyl-phenylamino)-N-(ethoxy)-benzamide;
3,4-Difluoro-2-(4-bromo-2-methyl-phenylamino)-N-(cyclobutoxy)-benzamide;
3,4-Difluoro-2-(4-bromo-2-methyl-phenylamino)-N-(isopropoxy)-benzamide;
3,4-Difluoro-2-(4-bromo-2-methyl-phenylamino)-N-(2-phenoxyethoxy)-benzamide;
3,4-Difluoro-2-(4-bromo-2-methyl-phenylamino)-N-(cyclopropyl-methoxy)-benzamide;
3,4-Difluoro-2-(4-bromo-2-methyl-phenylamino)-N-(n-propoxy)-benzamide;
3,4-Difluoro-2-(4-bromo-2-methyl-phenylamino)-N-(1-methyl-prop-2-ynyloxy)-benzamide;
3,4-Difluoro-2-(4-bromo-2-methyl-phenylamino)-N-(3-(3-fluorophenyl)-prop-2-ynyloxy)-benzamide;
3,4-Difluoro-2-(4-bromo-2-methyl-phenylamino)-N-(4,4-dimethylpent-2-ynyloxy)-benzamide;
3,4-Difluoro-2-(4-bromo-2-methyl-phenylamino)-N-(cyclopentoxy)-benzamide;
3,4,5-Trifluoro-N-hydroxy-2-(4-iodo-2-methyl-phenylamino)-benzamide;
5-Chloro-3,4-difluoro-N-hydroxy-2-(4-iodo-2-methyl-phenylamino)-benzamide;
5-Bromo-3,4-difluoro-2-(2-fluoro-4-iodo-phenylamino)-N-hydroxy-benzamide;
N-Hydroxy-2-(4-iodo-2-methyl-phenylamino)-4-nitro-benzamide;
3,4,5-Trifluoro-2-(2-fluoro-4-iodo-phenylamino)-N-hydroxy-benzamide;
5-Chloro-3,4-difluoro-2-(2-fluoro-4-iodo-phenylamino)-N-hydroxy-benzamide;
5-Bromo-2-(2-chloro-4-iodo-phenylamino)-3,4-difluoro-N-hydroxy-benzamide;
2-(2-Fluoro-4-iodo-phenylamino)-N-hydroxy-4-nitro-benzamide;
2-(2-Chloro-4-iodo-phenylamino)-3,4,5-trifluoro-N-hydroxy-benzamide;
5-Chloro-2-(2-chloro-4-iodo-phenylamino)-3,4-difluoro-N-hydroxy-benzamide;
5-Bromo-2-(2-bromo-4-iodo-phenylamino)-3,4-difluoro-N-hydroxy-benzamide;
2-(2-Chloro-4-iodo-phenylamino)-N-hydroxy-4-methyl-benzamide;
2-(2-Bromo-4-iodo-phenylamino)-3,4,5-trifluoro-N-hydroxy-benzamide;
2-(2-Bromo-4-iodo-phenylamino)-5-chloro-3,4-difluoro-N-hydroxy-benzamide;
2-(2-Bromo-4-iodo-phenylamino)-N-hydroxy-4-nitro-benzamide;
4-Fluoro-2-(2-fluoro-4-iodo-phenylamino)-N-hydroxy-benzamide;
3,4-Difluoro-2-(2-fluoro-4-iodo-phenylamino)-N-hydroxy-benzamide;
2-(2-Chloro-4-iodo-phenylamino)-4-fluoro-N-hydroxy-benzamide;
2-(2-Chloro-4-iodo-phenylamino)-3,4-difluoro-N-hydroxy-benzamide;
2-(2-Bromo-4-iodo-phenylamino)-4-fluoro-N-hydroxy-benzamide;
2-(2-Bromo-4-iodo-phenylamino)-3,4-difluoro-N-hydroxy-benzamide;.
N-Cyclopropylmethoxy-3,4,5-trifluoro-2-(4-iodo-2-methyl-phenylamino)-benzamide;
5-Chloro-N-cyclopropylmethoxy-3,4-difluoro-2-(4-iodo-2-methylphenylamino)-benzamide;
5-Bromo-N-cyclopropylmethoxy-3,4-difluoro-2-(2-fluoro-4-iodo-phenylamino)-benzamide;
N-Cyclopropylmethoxy-2-(4-iodo-2-methyl-phenylamino)-4-nitro-benzamide;
N-Cyclopropylmethoxy-3,4,5-trifluoro-2-(2-fluoro-4-iodo-phenylamino)-benzamide;
5-Chloro-N-cyclopropylmethoxy-3,4-difluoro-2-(2-fluoro-4-iodo-phenylamino)-benzamide;
5-Bromo-2-(2-chloro-4-iodo-phenylamino)-N-cyclopropylmethoxy-3,4-difluoro-benzamide;
N-Cyclopropylmethoxy-2-(2-fluoro-4-iodo-phenylamino)-4-nitro-benzamide;
2-(2-Chloro-4-iodo-phenylamino)-N-cyclopropylmethoxy-3,4,5-trifluoro-benzamide;
5-Chloro-2-(2-chloro-4-iodo-phenylamino)-N-cyclopropylmethoxy-3,4-difluoro-benzamide;
5-Bromo-2-(2-bromo-4-iodo-phenylamino)-N-ethoxy-3,4-difluoro-benzamide;
2-(2-Chloro-4-iodo-phenylamino)-N-ethoxy-4-nitro-benzamide;
2-(2-Bromo-4-iodo-phenylamino)-N-cyclopropylmethoxy-3,4,5-trifluoro-benzamide;
2-(2-Bromo-4-iodo-phenylamino)-5-chloro-N-cyclopropylmethoxy-3,4-difluoro-benzamide
2-(2-Bromo-4-iodo-phenylamino)-N-cyclopropylmethoxy-4-nitro-benzamide;
N-Cyclopropylmethoxy-4-fluoro-2-(2-fluoro-4-iodo-phenylamino)-benzamide;
N-Cyclopropylmethoxy-3,4-difluoro-2-(2-fluoro-4-iodo-phenylamino)-benzamide;
2-(2-Chloro-4-iodo-phenylamino)-N-cyclopropylmethoxy-4-fluoro-benzamide;
2-(2-Chloro-4-iodo-phenylamino)-N-cyclopropylmethoxy-3,4-difluoro-benzamide;
2-(2-Bromo-4-iodo-phenylamino)-N-cyclopropylmethoxy-4-fluoro-benzamide; or
2-(2-Bromo-4-iodo-phenylamino)-N-cyclopropylmethoxy-3,4-difluoro-benzamide.

6. The combination according to Claim 1 wherein the MEK inhibitor is a MEK1 or MEK 2 inhibitor selected from:
2-(2-chloro-4-iodophenylamino)-N-cyclopropylmethoxy-3,4-difluorobenzamide (PD184352);
2-(2-methyl-4-iodophenylamino)-N-hydroxy-4-fluorobenzamide (PD170611);
2-(2-methyl-4-iodophenylamino)-N-hydroxy-3,4-difluoro-5-bromobenzamide (PD171984);
2-(2-methyl-4-iodophenylamino)-N-cyclopropylmethoxy-3,4-difluoro-5-bromobenzamide (PD177168);
2-(2-methyl-4-iodophenylamino)-N-cyclobutylmethoxy-3,4-difluoro-5-bromobenzamide (PD 180841);
2-(2-chloro-4-iodophenylamino)-N-cyclopropylmethoxy-3,4-difluoro-5-bromobenzamide (PD 184161);
2-(2-chloro-4-iodophenylamino)-N-hydroxy-3,4-difluoro-5-bromobenzamide (PD184386);
2-(2-chloro-4-iodophenylamino)-N-cyclobutylmethoxy-3,4-difluorobenzamide (PD 185625);
2-(2-chloro-4-iodophenylamino)-N-hydroxy-4-fluorobenzamide (PD 185848);
2-(2-methyl-4-iodophenylamino)-N-hydroxy-3,4-difluorobenzamide (PD 188563);
2-(2-methyl-4-iodophenylamino)-N-cyclopropylmethoxy-3,4,5-trifluorobenzamide (PD 198306); and
2-(2-chloro-4-iodophenylamino)-N-cyclopropylmethoxy-4-fluorobenzamide (PD 203311).

7. An anticancer combination comprising paclitaxel and the MEK inhibitor 2-(2-chloro-4-iodophenylamino)-N-cyclopropylmethoxy-3,4-difluorobenzamide (PD184352).

8. Use of an anticancer combination comprising a mitotic inhibitor and a MEK inhibitor according to claims 1 to 7 for the manufacture of pharmaceuticals for the treatment of cancer.

9. A use of claim 8, for the manufacture of non-simultaneously administrable pharmaceuticals.

10. A use according to Claim 8 wherein the MEK inhibitor is a phenyl amine of Formula I.

11. A use according to Claim 8 wherein the MEK inhibitor is a phenyl amine of Formula II.

12. A use according to Claim 8 wherein the MEK inhibitor used in combination with a mitotic inhibitor is a selective MEK 1 or MEK 2 inhibitor.

13. A use according to claim 11, wherein the MEK inhibitor is a compound selected from:
2-(2-chloro-4-iodophenylamino)-N-cyclopropylmethoxy-3,4-difluorobenzamide (PD184352);
2-(2-methyl-4-iodophenylamino)-N-hydroxy-4-fluorobenzamide (PD170611);
2-(2-methyl-4-iodophenylamino)-N-hydroxy-3,4-difluoro-5-bromobenzamide (PD171984);
2-(2-methyl-4-iodophenylamino)-N-cyclopropylmethoxy-3,4-difluoro-5-bromobenzamide (PD177168);
2-(2-methyl-4-iodophenylamino)-N-cyclobutylmethoxy-3,4-difluoro-5-bromobenzamide (PD 180841);
2-(2-chloro-4-iodophenylamino)-N-cyclopropylmethoxy-3,4-difluoro-5-bromobenzamide (PD 184161);
2-(2-chloro-4-iodophenylamino)-N-hydroxy-3,4-difluoro-5-bromobenzamide (PD1843 86);
2-(2-chloro-4-iodophenylamino)-N-cyclobutylmethoxy-3,4-difluorobenzamide (PD 185625);
2-(2-chloro-4-iodophenylamino)-N-hydroxy-4-fluorobenzamide (PD 185848);
2-(2-methyl-4-iodophenylamino)-N-hydroxy-3,4-difluorobenzamide (PD 188563);
2-(2-methyl-4-iodophenylamino)-N-cyclopropylmethoxy-3,4,5-trifluorobenzamide (PD 198306); and
2-(2-chloro-4-iodophenylamino)-N-cyclopropylmethoxy-4-fluorobenzamide (PD 203311).

14. The use according to Claim 8 wherein the mitotic inhibitor is selected from paclitaxel, docetaxel, vincristine, vinblastine, vinorelbine, and vinflunine.

15. The use according to Claim 14 wherein the mitotic inhibitor is paclitaxel.

16. The use according to Claim 14 wherein the mitotic inhibitor is docetaxel.

17. The use according to Claim 14 wherein the mitotic inhibitor is vincristine.

18. The use according to Claim 14 wherein the mitotic inhibitor is vinblastine.

19. The use according to Claim 14 wherein the mitotic inhibitor is vinorelbine.

20. The use according to Claim 14 wherein the mitotic inhibitor is vinflunine.

21. The use according to Claim 14 wherein the MEK inhibitor is 2-(2-chloro-4-iodophenylamino)-N-cyclopropylmethoxy-3,4-difluorobenzamide (PD184352).

22. A use according to Claim 13, wherein the mitotic inhibitor is paclitaxel, docetaxel, or vincristine.

## Patentansprüche

1. Anti-Krebs-Kombination, enthaltend einen Mitoseinhibitor und einen MEK-Inhibitor, wobei letzterer eine Phenylaminverbindung der Formel I 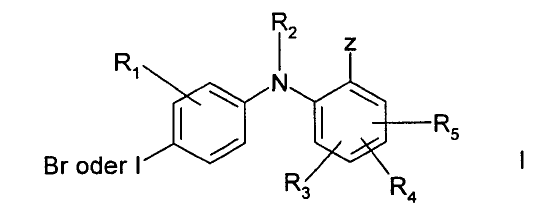 oder deren pharmazeutisch akzeptables Salz darstellt, worin
R₁ für Wasserstoff, Hydroxy, C₁-C₈-Alkyl, C₁-C₈-Alkoxy, Halogen, Trifluormethyl oder CN steht;
R₂ für Wasserstoff steht;
R₃, R₄ und R₅ unabhängig für Wasserstoff, Hydroxy, Halogen, Trifluormethyl, C₁-C₈-Alkyl, C₁-C₈-Alkoxy, Nitro, CN oder -(O oder NH)ₘ-(CH₂)ₙ-R₉ stehen, wobei R₉ Wasserstoff, Hydroxy, COOH oder NR₁₀R₁₁ bedeutet;
n eine Zahl von 0 bis 4 bedeutet;
m 0 oder 1 bedeutet;
R₁₀ und R₁₁ unabhängig für Wasserstoff oder C₁-C₈-Alkyl stehen oder gemeinsam mit dem Stickstoffatom, an das sie gebunden sind, einen 3- bis 10-gliedrigen Ring bilden, der optional 1, 2 oder 3 zusätzliche Heteroatome enthält, die ausgewählt sind aus O, S, NH und N-C₁-C₈-Alkyl;
Z für COOR₇, Tetrazolyl, CONR₆R₇, CONHNR₁₀R₁₁ oder CH₂OR₇ steht;
R₆ und R₇ unabhängig für Wasserstoff, C₁-C₈-Alkyl, C₂-C₈-Alkenyl, C₂-C₈-Alkinyl, (CO)-C₁-C₈-Alkyl, Aryl, Heteroaryl oder C₃-C₁₀-Cycloalkyl, das optional 1, 2 oder 3 Heteroatome aus der Gruppe O, S, NH und N-Alkyl enthält, stehen; oder R₆ und R₇ bilden gemeinsam mit dem Stickstoffatom, an das sie gebunden sind, einen 3- bis 10-gliedrigen Ring, der optional 1, 2 oder 3 zusätzliche Heteroatome enthält, die ausgewählt sind aus O, S, NH und N-Alkyl;
und wobei die genannten Alkyl-, Alkenyl, Aryl-, Heteroaryl-, heterocyclischen und Alkinylgruppen unsubstituiert oder jeweils durch Halogen, Hydroxy, C₁-C₆-Alkoxy, Amino, Nitro, C₁-C₄-Alkylamino, Di(C₁-C₄)alkylamino, C₃-C₆-Cycloalkyl, Phenyl, Phenoxy, C₃-C₅-Heteroaryl oder heterocyclischen Rest oder C₃-C₅-Heteroaryloxy oder Heterocyclyloxy-Gruppe substituiert sein können.

2. Kombination nach Anspruch 1, wobei der Mitoseinhibitor ausgewählt ist aus Paclitaxel, Docetaxel, Vincristin, Vinblastin, Vinorelbin und Vinflunin.

3. Kombination nach Anspruch 1, wobei der MEK-Inhibitor ein Phenylamin darstellt, das ausgewählt ist aus:
[4-Chlor-2-(1H-tetrazol-5-yl)-phenyl-(4-jod-2-methyl-phenyl)-amin;
(4-Jod-2-methyl-phenyl)-[2-(1H-tetrazol-5-yl)-phenyl]-amin;
[4-Nitro-2-(1H-tetrazol-5-yl)-phenyl-(4-jod-2-methyl-phenyl)-amin;
4-Fluor-2-(4-jod-2-methyl-phenylamino)-benzoesäure;
3,4,5-Trifluor-2-(4-jod-2-methyl-phenylamino)-benzoesäure;
3,4-Difluor-2-(4-jod-2-methyl-phenylamino)-benzoesäure;
5-Brom-3,4-difluor-2-(4-jod-2-methyl-phenylamino)-benzoesäure;
5-Chlor-2-(4-jod-2-methyl-phenylamino)-benzoesäure;
5-Chlor-2-(4-jod-2-methyl-phenylamino)-benzoesäure-Natriumsalz;
5-Brom-2-(4-jod-2-methyl-phenylamino)-benzoesäure;
2-(4-Jod-2-methyl-phenylamino)-5-nitro-benzoesäure;
4-Chlor-2-(4-jod-2-methyl-phenylamino)-benzoesäure-Natriumsalz;
2-(4-Jod-2-methyl-phenylamino)-benzoesäure;
5-Fluor-2-(4-jod-2-methyl-phenylamino)-benzoesäure;
5-Jod-2-(4-jod-2-methyl-phenylamino)-benzoesäure;
2,3,5-Trifluor-4-(4-jod-2-methyl-phenylamino)-benzoesäure;
2-(4-Jod-phenylamino)-5-methoxy-benzoesäure;
5-Methyl-2-(4-jod-2-methyl-phenylamino)-benzoesäure;
2-(4-Jod-2-methyl-phenylamino)-4-nitro-benzoesäure;
2-(4-Brom-2-methyl-phenylamino)-4-fluor-benzoesäure;
2-(2-Brom-4-jod-phenylamino)-5-nitro-benzoesäure;
2-(4-Brom-2-methyl-phenylamino)-3,4-difluor-benzoesäure;
5-Chlor-N-(2-hydroxyethyl)-2-(4-jod-2-methyl-phenylamino)-benzamid;
4-Fluor-2-(4-jod-2-methyl-phenylamino)-benzamid;
4-Fluor-2-(4-jod-2-methyl-phenylamino)-N-methyl-benzamid;
N-Ethyl-4-fluor-2-(4-jod-2-methyl-phenylamino)-benzamid;
4-Fluor-2-(4-jod-2-methyl-phenylamino)-N,N-dimethyl-benzamid;
4-Fluor-2-(4-jod-2-methyl-phenylamino)-N-(1H-tetrazol-5-yl)-benzamid;
5-Brom-2-(4-jod-2-methyl-phenylamino)-benzamid;
5-Chlor-2-(4-jod-2-methyl-phenylamino)-N,N-dimethyl-benzamid;
[5-Chlor-2-(4-jod-2-methyl-phenylamino)-benzoylamino]-essigsäure;
4-Fluor-2-(4-jod-2-methyl-phenylamino)-N-propyl-benzamid;
5-Brom-N-(2-hydroxy-ethyl)-2-(4-jod-2-methyl-phenylamino)-benzamid;
N,N-Diethyl-4-fluor-2-(4-jod-2-methyl-phenylamino)-benzamid;
4-Fluor-N-{3-[4-(2-hydroxy-ethyl)-piperazin-1-yl]-propyl}-2-(4-jod-2-methyl-phenylamino)-benzamid;
N,N-Diethyl-2-(4-jod-2-methyl-phenylamino)-5-nitro-benzamid;
N-Butyl-4-fluor-2-(4-jod-2-methyl-phenylamino)-benzamid;
5-Chlor-N,N-diethyl-2-(4-jod-2-methyl-phenylamino)-benzamid;
5-Brom-2-(4-jod-2-methyl-phenylamino)-N,N-dimethyl-benzamid;
5-Brom-3,4-difluor-N-(2-hydroxy-ethyl)-2-(4-jod-2-methyl-phenylamino)-benzamid;
N-(2, 3-dihydroxy-propyl)-3,4-difluor-2-(4-jod-2-methyl-phenylamino)-benzamid;
5-Brom-3,4-difluor-2-(4-jod-2-methyl-phenylamino)-N-(2-piperidin-1-yl-ethyl)-benzamid;
3,4-Difluor-N-(2-hydroxy-ethyl)-2-(4-jod-2-methyl-phenylamino)-benzamid;
N-(2,3-Dihydroxy-propyl)-4-fluor-2-(4-jod-2-methyl-phenylamino)-benzamid;
3,4-Difluor-N-(3-hydroxy-propyl)-2-(4-jod-2-methyl-phenylamino)-benzamid;
5-Brom-3,4-difluor-2-(4-jod-2-methyl-phenylamino)-N-(2-pyrrolidin-1-yl-ethyl)-benzamid;
5-Brom-3,4-difluor-2-(4-jod-2-methyl-phenylamino)-N-(2-pyridin-4-yl-ethyl)-benzamid;
4-Fluor-N-(2-hydroxy-ethyl)-2-(4-jod-2-methyl-phenylamino)-benzamid;
5-Brom-N-(3-dimethylamino-propyl)-3,4-difluor-2-(4-jod-2-methyl-phenylamino)-benzamid;
5-Brom-3,4-difluor-2-(4-jod-2-methyl-phenylamino)-N-(2-morpholin-4-yl-ethyl)-benzamid;
3,4-Difluor-2-(4-jod-2-methyl-phenylamino)-N-(2-morpholin-4-yl-ethyl)-benzamid;
3,4-Difluor-2-(4-jod-2-methyl-phenylamino)-N-(2-pyrrolidin-1-yl-ethyl)-benzamid;
3,4-Difluor-2-(4-jod-2-methyl-phenylamino)-N-(2-pyridin-4-yl-ethyl)-benzamid;
N-(3-Dimethylamino-propyl)-3,4-difluor-2-(4-jod-2-methyl-phenylamino)-benzamid;
N-Benzyl-4-fluor-2-(4-jod-2-methyl-phenylamino)-benzamid;
2-(4-Brom-2-methyl-phenylamino)-3,4-difluor-N-(2-hydroxy-ethyl)-benzamid;
4-Fluor-2-(4-jod-2-methyl-phenylamino)-N-(2-morpholin-4-yl-ethyl)-benzamid;
4-Fluor-2-(4-jod-2-methyl-phenylamino)-N-(3-piperidin-1-yl-propyl)-benzamid;
3,4-Difluor-2-(4-jod-2-methyl-phenylamino)-N-(3-piperidin-1-yl-propyl)-benzamid;
4-Fluor-2-(4-jod-2-methyl-phenylamino)-N-(2-thiophen-2-yl-ethyl)-benzamid;
4-Fluor-2-(4-jod-2-methyl-phenylamino)-N-(2-pyrrolidin-1-yl-ethyl)-benzamid;
2-(4-Brom-2-methyl-phenylamino)-3,4-difluor-N-(2-morpholin-4-yl-ethyl)-benzamid;
5-Brom-3,4-difluor-2-(4-jod-2-methyl-phenylamino)-N-pyridin-4-ylmethyl-benzamid;
3,4-Difluor-2-(4-jod-2-methyl-phenylamino)-N-pyridin-4-ylmethyl-benzamid;
2-(4-Brom-2-methyl-phenylamino)-N-(3-dimethylamino-propyl)-3,4-difluor-benzamid;
4-Fluor-2-(4-jod-2-methyl-phenylamino)-N-pyridin-4-ylmethyl-benzamid;
4-Fluor-2-(4-jod-2-methyl-phenylamino)-N-(2-pyridin-4-yl-ethyl)-benzamid;
2-(4-Brom-2-methyl-phenylamino)-3,4-difluor-N-(2-pyridin-4-yl-ethyl)-benzamid;
2-(4-Brom-2-methyl-phenylamino)-3,4-difluor-N-(3-hydroxy-propyl)-benzamid;
2-(4-Brom-2-methyl-phenylamino)-3,4-difluor-N-(2-pyrrolidin-1-yl-ethyl)-benzamid;
4-Fluor-2-(4-jod-2-methyl-phenylamino)-N-phenethyl-benzamid;
2-(4-Brom-2-methyl-phenylamino)-3,4-difluor-N-(2-thiophen-2-yl-ethyl)-benzamid;
2-(4-Brom-2-methyl-phenylamino)-3,4-difluor-N-pyridin-4-ylmethyl-benzamid;
2-(4-Brom-2-methyl-phenylamino)-3,4-difluor-N-phenethyl-benzamid;
2-(4-Brom-2-methyl-phenylamino)-3,4-difluor-N-(2-piperidin-1-yl-ethyl)-benzamid;
5-Chlor-N-{3-[4-(2-hydroxy-ethyl)-piperazin-1-yl]-propyl}-2-(4-jod-2-methyl-phenylamino)-benzamid;
5-Fluor-N-{3-[4-(2-hydroxy-ethyl)-piperazin-1-yl]-propyl}-2-(4-jod-2-methyl-phenylamino)-benzamid;
2-(4-Jod-2-methyl-phenylamino)-5-nitro-N-pyridin-4-ylmethyl-benzamid;
5-Brom-N-{3-[4-(2-hydroxy-ethyl)-piperazin-1-yl]-propyl}-2-(4-jod-2-methyl-phenylamino)-benzamid;
5-Chlor-N-(2-diethylamino-ethyl)-2-(4-jod-2-methyl-phenylamino)-benzamid;
5-Chlor-2-(4-jod-2-methyl-phenylamino)-N-(2-piperidin-1 -yl-ethyl)-benzamid;
(3-Hydroxy-pyrrolidin-1-yl)-[2-(4-jod-2-methyl-phenylamino)-5-nitro-phenyl];
5-Chlor-2-(4-jod-2-methyl-phenylamino)-N-(2-pyrrolidin-1-yl-ethyl)-benzamid;
5-Brom-N-(2-diethylamino-ethyl)-2-(4-jod-2-methyl-phenylamino)-benzamid;
N-{2-[Bis-(2-hydroxy-ethyl)-amino]-ethyl}-5-chlor-2-(4-jod-2-methyl-phenylamino)-benzamid;
N-(2-[Bis-(2-hydroxy-ethyl)-amino]-ethyl)-5-brom-2-(4-jod-2-methyl-phenylamino)-benzamid;
N-{3-[4-(2-Hydroxy-ethyl)-piperazin-1-yl]-propyl}-2-(4-jod-2-methyl-phenylamino)-benzamid;
5-Fluor-2-(4-jod-2-methyl-phenylamino)-N-pyridin-4-ylmethyl-benzamid;
5-Brom-2-(4-jod-2-ethyl-phenylamino)-N-(2-pyrrolidin-1-yl-ethyl)-benzamid;
5-Brom-2-(4-jod-2-methyl-phenylamino)-N-(2-piperidin-1-yl-ethyl)-benzamid;
5-Fluor-2-(4-jod-2-methyl-phenylamino)-N-(2-pyrrolidin-1-yl-ethyl)-benzamid;
5-Chlor-N-(3-dimethylamino-propyl)-2-(4-jod-2-methyl-phenylamino)- benzamid;
N-{2-[Bis-(2-hydroxy-ethyl)-amino]-ethyl}-5-fluor-2-(4-jod-2-methyl-phenylamino)-benzamid;
5-Chlor-N-(3-hydroxy-propyl)-2-(4-jod-2-methyl-phenylamino)- benzamid;
5-Chlor-N-(3-diethylamino-2-hydroxy-propyl)-2-(4-jod-2-methyl-phenylamino)-benzamid;
5-Fluor-2-(4-jod-2-methyl-phenylamino)-N-(2-piperidin-1-yl-ethyl)-benzamid;
5-Brom-N-(3-hydroxy-propyl)-2-(4-jod-2-methyl-phenylamino)-benzamid;
5-Brom-2-(4-jod-2-methyl-phenylamino)-N-(3-piperidin-1-yl-propyl)-benzamid;
N-{2-[Bis-(2-hydroxy-ethyl)-amino]-ethyl}-2-(4-jod-2-methyl-phenylamino)-5-nitro-benzamid;
5-Chlor-2-(4-jod-2-methyl-phenylamino)-N-(2-morpholin-4-yl-ethyl)-benzamid;
5-Chlor-N-(3-diethylamino-propyl)-2-(4-jod-2-methyl-phenylamino)-benzamid;
5-Chlor-N-(2-diisopropylamino-ethyl)-2-(4-jod-2-methyl-phenylamino)-benzamid;
5-Chlor-2-(4-jod-2-methyl-phenylamino)-N-(3-piperidin-1-yl-propyl)-benzamid;
2-(4-Jod-2-methyl-phenylamino)-5-nitro-N-(2-piperidin-1-yl-ethyl)-benzamid;
5-Brom-2-(4-jod-2-methyl-phenylamino)-N-(2-piperazin-1-yl-ethyl)-benzamid;
N-(2-Diethylamino-ethyl)-5-fluor-2-(4-jod-2-methyl-phenylamino)-benzamid;
5-Brom-N-(3-dimethylamino-propyl)-2-(4-jod-2-methyl-phenylamino)-benzamid;
N-(3-Hydroxy-propyl)-2-(4-jod-2-methyl-phenylamino)-5-nitro-benzamid;
5-Fluor-N-(3-hydroxy-propyl)-2-(4-jod-2-methyl-phenylamino)-benzamid;
N-(3-Diethylamino-propyl)-5-fluor-2-(4-jod-2-methyl-phenylamino)-benzamid;
N-(3-Diethylamino-propyl)-2-(4-jod-2-methyl-phenylamino)-5-nitro-benzamid;
5-Brom-2-(4-jod-2-methyl-phenylamino)-N-(2-morpholin-4-yl-ethyl)-benzamid;
2-(4-Jod-2-methyl-phenylamino)-5-nitro-N-(3-piperidin-1-yl-propyl)-benzamid;
[5-Fluor-2-(4-jod-2-methyl-phenylamino)-phenyl]-(3-hydroxy-pyrrolidin-1-yl)-methanon;
5-Brom-N-(2-diisopropylamino-ethyl)-2-(4-jod-2-methyl-phenylamino)-benzamid;
5-Fluor-2-(4-jod-2-methyl-phenylamino)-N-(2-morpholin-4-yl-ethyl)-benzamid;
5-Fluor-2-(4-jod-2-methyl-phenylamino)-N-(3-piperidin-1-yl-propyl)-benzamid;
[5-Fluor-2-(4-jod-2-methyl-phenylamino)-phenyl]-[4-(2-hydroxy-ethyl)-piperazin-1-yl]-methanon;
N-(3-Diethylamino-2-hydroxy-propyl)-5-fluor-2-(4-jod-2-methyl-phenylamino)-benzamid;
N-Cyclopropyl-5-fluor-2-(4-jod-2-methyl-phenylamino)-benzamid;
5-Chlor-N-(2-hydroxy-ethyl)-2-(4-jod-2-methyl-phenylamino)-benzamid;
5-Fluor-N-(2-hydroxy-ethyl)-2-(4-jod-2-methyl-phenylamino)-benzamid;
N-Benzyloxy-5-fluor-2-(4-jod-2-methyl-phenylamino)-benzamid;
N-Benzyloxy-5-brom-2-(4-jod-2-methyl-phenylamino)-benzamid;
2-(4-Jod-2-methyl-phenylamino)-5-nitro-N-(4-sulfamoyl-benzyl)-benzamid;
5-Brom-N-(2-hydroxy-ethyl)-2-(4-jod-2-methyl-phenylamino)-benzamid;
N-(2-Hydroxy-ethyl)-5-jod-2-(4-jod-2-methyl-phenylamino)-benzamid;
N-(2-Hydroxy-ethyl)-2-(4-jod-2-ethyl-phenylamino)-5-nitro-benzamid;
2-(4-Jod-2-methyl-phenylamino)-N-methyl-5-nitro-N-phenyl-benzamid;
5-Chlor-N-cyclopropyl-2-(4-jod-2-methyl-phenylamino)-benzamid;
5-Fluor-2-(4-jod-2-methyl-phenylamino)-N-methyl-N-phenyl-benzamid;
N-Allyl-5-fluor-2-(4-jod-2-methyl-phenylamino)-benzamid;
N-Benzyloxy-5-jod-2-(4-jod-2-methyl-phenylamino)-benzamid;
5-Fluor-2-(4-jod-2-methyl-phenylamino)-N-(4-sulfamoyl-benzyl)-benzamid;
N-Allyl-5-chlor-2-(4-jod-2-methyl-phenylamino)-benzamid;
N-Cyclopropyl-2-(4-jod-2-methyl-phenylamino)-5-nitro-benzamid;
5-Brom-N-cyclopropyl-2-(4-jod-2-methyl-phenylamino)-benzamid;
5-Chlor-2-(4-jod-2-methyl-phenylamino)-N-methyl-N-phenyl-benzamid;
5-Jod-2-(4-jod-2-methyl-phenylamino)-N-(4-sulfamoyl-benzyl)-benzamid;
5-Brom-2-(4-jod-2-methyl-phenylamino)-N-(4-sulfamoyl-benzyl)-benzamid;
N-Allyl-2-(4-jod-2-methyl-phenylamino)-5-nitro-benzamid;
2-(4-Jod-2-methyl-phenylamino)-5-nitro-N-(4-sulfamoyl-benzyl)-benzamid;
N-Allyl-5-brom-2-(4-jod-2-methyl-phenylamino)-benzamid;
5-Fluor-2-(4-jod-2-methyl-phenylamino)-N-(3-methyl-benzyl)-benzamid;
N-Cyclopropyl-5-jod-2-(4-jod-2-methyl-phenylamino)-benzamid;
5-Brom-2-(4-jod-2-methyl-phenylamino)-N-methyl-N-phenyl-benzamid;
N-Benzyloxy-2-(4-jod-2-methyl-phenylamino)-5-nitro-benzamid;
N-Cyclohexyl-5-jod-2-(4-jod-2-methyl-phenylamino)-benzamid;
N-Allyl-5-jod-2-(4-jod-2-methyl-phenylamino)-benzamid;
5-Jod-2-(4-jod-2-methyl-phenylamino)-N-(3-methyl-benzyl)-benzamid;
2-(4-Jod-2-methyl-phenylamino)-N-(3-methyl-benzyl)-5-nitro-benzamid;
5-Jod-2-(4-jod-2-methyl-phenylamino)-N-methyl-N-phenyl-benzamid;
N-Cyclohexyl-5-fluor-2-(4-jod-2-methyl-phenylamino)-benzamid;
5-Chlor-N-cyclohexyl-2-(4-jod-2-methyl-phenylamino)-benzamid;
5-Brom-2-(4-jod-2-methyl-phenylamino)-N-(3-methyl-benzyl)-benzamid;
5-Brom-N-cyclohexyl-2-(4-jod-2-methyl-phenylamino)-benzamid;
5-Chlor-2-(4-jod-2-methyl-phenylamino)-N-(3-methyl-benzyl)-benzamid;
N-Cyclohexyl-2-(4-jod-2-methyl-phenylamino)-5-nitro-benzamid;
N-Benzyloxy-5-brom-2-(4-jod-2-methyl-phenylamino)-benzamid;
N-Benzyloxy-5-fluor-2-(4-jod-2-methyl-phenylamino)-benzamid;
5-Chlor-N-(2-hydroxy-ethyl)-2-(4-jod-2-methyl-phenylamino)-benzamid;
5-Brom-N-(2-hydroxy-ethyl)-2-(4-jod-2-methyl-phenylamino)-benzamid;
2-(4-Jod-2-methyl-phenylamino)-N-methyl-5-nitro-N-phenyl-benzamid;
5-Chlor-2-(4-jod-2-methyl-phenylamino)-N-methyl-N-phenyl-benzamid;
N-(2-Hydroxy-ethyl)-5-jod-2-(4 jod-2-methyl-phenylamino)-benzamid;
5-Chlor-N-cyclopropyl-2-(4-jod-2-methyl-phenylamino)-benzamid;
N-Allyl-5-chlor-2-(4-jod-2-methyl-phenylamino)-benzamid;
5-Fluor-2-(4-jod-2-methyl-phenylamino)-N-methyl-N-phenyl-benzamid;
N-(2-Hydroxy-ethyl)-2-(4 jod-2-methyl-phenylamino)-5-nitro-benzamid;
5-Fluor-N-(2-hydroxy-ethyl)-2-(4-jod-2-methyl-phenylamino)-benzamid;
5-Brom-N-cyclopropyl-2-(4-jod-2-methyl-phenylamino)-benzamid;
N-Cyclopropyl-5-fluor-2-(4-jod-2-methyl-phenylamino)-benzamid;
5-Fluor-2-(4-jod-2-methyl-phenylamino)-N-(4-sulfamoyl-benzyl)-benzamid;
N-Cyclopropyl-2-(4-jod-2-methyl-phenylamino)-5-nitro-benzamid;
N-Allyl-5-fluor-2-(4-jod-2-methyl-phenylamino)-benzamid;
N-Benzyloxy-5-jod-2-(4-jod-2-methyl-phenylamino)-benzamid;
N-Allyl-5-brom-2-(4-jod-2-methyl-phenylamino)-benzamid;
5-Brom-2-(4-jod-2-methyl-phenylamino)-N-(4-sulfamoyl-benzyl)-benzamid;
5-Brom-2-(4-jod-2-methyl-phenylamino)-N-methyl-N-phenyl-benzamid;
N-Allyl-2-(4-jod-2-methyl-phenylamino)-5-nitro-benzamid;
4-Fluor-2-(4-jod-2-methyl-phenylamino)-benzylalkohol ;
[5-Chlor-2-(4-jod-2-methyl-phenylamino)-phenyl]-methanol;
[2-(4-Jod-2-methyl-phenylamino)-5-nitro-phenyl]-methanol;
[5-Brom-2-(4-jod-2-methyl-phenylamino)-phenyl]-methanol und
N-Allyl-2-(4-jod-2-methyl-phenylamino)-5-nitro-benzamid.

4. Anti-Krebs-Kombination, enthaltend einen Mitoseinhibitor und einen MEK-Inhibitor, wobei letzterer eine Phenylaminverbindung der Formel II 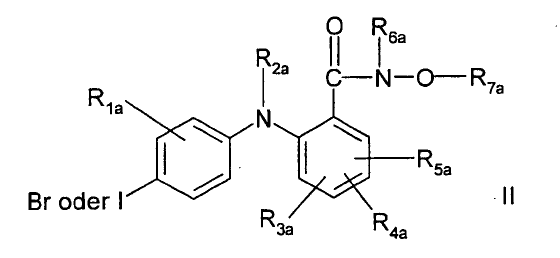 oder deren pharmazeutisch akzeptables Salz darstellt, worin
R₁ₐ für Wasserstoff, Hydroxy, C₁-C₈-Alkyl, C₁-C₈-Alkoxy, Halogen, Trifluormethyl oder CN steht;
R₂ₐ für Wasserstoff steht;
R₃ₐ, R₄ₐ und R₅ₐ unabhängig für Wasserstoff, Hydroxy, Halogen, Trifluormethyl, C₁-C₈-Alkyl, C₁-C₈-Alkoxy, Nitro, CN oder (O oder NH)ₘ-(CH₂)ₙ-R₉ₐ stehen, wobei R₉ₐ Wasserstoff, Hydroxy, CO₂H oder NR₁₀ₐR₁₁ₐ bedeutet;
n eine Zahl von 0 bis 4 bedeutet;
m 0 oder 1 bedeutet;
R₁₀ₐ und R₁₁ₐ unabhängig für Wasserstoff oder C₁-C₈-Alkyl stehen oder gemeinsam mit dem Stickstoffatom, an das sie gebunden sind, einen 3- bis 10-gliedrigen Ring bilden, der optional 1, 2 oder 3 zusätzliche Heteroatome enthält, die ausgewählt sind aus O, S, NH und N-C₁-C₈-Alkyl;
R₆ₐ für Wasserstoff, C₁-C₈-Alkyl, (CO)-C₁-C₈-Alkyl, Aryl, Aralkyl oder C₃-C₁₀-Cycloalkyl steht;
R₇ₐ für Wasserstoff, C₁-C₈-Alkyl, C₂-C₈-Alkenyl, C₂-C₈-Alkinyl oder C₃-C₁₀-Cycloalkyl, das optional ein Heteroatom aus der Gruppe O, S, und NR₉ₐ enthält, steht;
und wobei die genannten Alkyl-, Alkenyl, Aryl-, Heteroaryl- und Alkinylgruppen sowie die genannten heterocyclischen Reste jeweils durch Halogen, Hydroxy, C₁-C₆-Alkoxy, Amino, Nitro, C₁-C₄-Alkylamino, Di(C₁-C₄)alkylamino, C₃-C₆-Cycloalkyl, Phenyl, Phenoxy, C₃-C₅-Heteroaryl oder heterocyclischen Rest oder C₃-C₅Heteroaryloxy oder eine Heterocyclyloxy-Gruppe substituiert sein können, oder R₆ₐ und R₇ₐ gemeinsam mit dem Stickstoffatom, an das sie gebunden sind, einen 5- bis 10-gliedrigen Ring bilden, der optional ein, zwei oder drei zusätzliche Heteroatome enthält, die ausgewählt sind aus O, S und NR₁₀ₐR₁₁ₐ.

5. Anti-Krebs-Kombination, enthaltend einen Mitoseinhibitor und einen selektiven MEK1- oder MEK2-Inhibitor, der ausgewählt ist aus:
4-Fluor-N-hydroxy-2-(4-jod-2-methyl-phenylamino)-benzamid;
4-Fluor-2-(4-jod-2-methyl-phenylamino)-N-(methoxy)-benzamid;
4-Fluor-2-(4-jod-2-methyl-phenylamino)-N-(prop-2-inyloxy)-benzamid;
4-Fluor-2-(4-jod-2-methyl-phenylamino)-N-(2-phenoxyethoxy)-benzamid;
4-Fluor-2-(4-jod-2-methyl-phenylamino)-N-(2-thienylmethoxy)-benzamid;
4-Fluor-2-(4 jod-2-methyl-phenylamino)-N-(prop-2-enyloxy)-benzamid;
4-Fluor-2-(4-jod-2-methyl-phenylamino)-N-(cyclopropylmethoxy)-benzamid;
4-Fluor-2-(4-jod-2-methyl-phenylamino)-N-(cyclopentoxy)-benzamid;
3,4-Difluor-2-(4-jod-2-methyl-phenylamino)-N-(3-furylmethoxy)-benzamid;
3,4-Difluor-2-(4 jod-2-methyl-phenylamino)-N-ethoxy-benzamid;
3,4-Difluor-2-(4-jod-2-methyl-phenylamino)-N-(but-2-enyloxy)-benzamid;
3,4-Difluor-2-(4-jod-2-methyl-phenylamino)-N-(cyclopropylmethoxy)-benzamid;
3,4-Difluor-2-(4-jod-2-methyl-phenylamino)-N-(1-methylprop-2-inyloxy)-benzamid;
3,4-Difluor-2-(4-jod-2-methyl-phenylamino)-N-(3-phenylprop-2-inyloxy)-benzamid;
3,4-Difluor-2-(4-jod-2-methyl-phenylamino)-N-(3-methyl-5-phenylpent-2-en-4-inyloxy)-benzamid;
3,4-Difluor-2-(4-jod-2-methyl-phenylamino)-N-(prop-2-inyloxy)-benzamid;
3,4-Difluor-2-(4-jod-2-methyl-phenylamino)-N-(propoxy)-benzamid;
3,4-Difluor-2-(4-jod-2-methyl-phenylamino)-N-(cyclobutyloxy)-benzamid;
3,4-Difluor-2-(4-jod-2-methyl-phenylamino)-N-(2-thienylmethoxy)-benzamid;
3,4-Difluor-2-(4-jod-2-methyl-phenylamino)-N-(2-methyl-prop-2-enyloxy)-benzamid;
3,4-Difluor-2-(4-jod-2-methyl-phenylamino)-N-(2-phenoxyethoxy)-benzamid;
3,4-Difluor-2-(4-jod-2-methyl-phenylamino)-N-(but-2-enyloxy)-benzamid;
3,4-Difluor-2-(4-jod-2-methyl-phenylamino)-N-(but-3-inyloxy)-benzamid;
3,4-Difluor-2-(4-jod-2-methyl-phenylamino)-N-(cyclopentyloxy)-benzamid;
3,4-Difluor-2-(4-jod-2-methyl-phenylamino)-N-(3-(2-fluorphenyl)-prop-2-inyloxy)-benzamid;
5-Brom-3,4-difluor-N-hydroxy-2-(4-jod-2-methyl-phenylamino)-benzamid;
5-Brom-3,4-difluor-2-(4-jod-2-methyl-phenylamino)-N-(n-propoxy)-benzamid;
5-Brom-3,4-difluor-N-(furan-3-ylmethoxy)-2-(4-jod-2-methyl-phenylamino)-benzamid;
5-Brom-N-(but-2-enyloxy)-3,4-difluor-2-(4-jod-2-methyl-phenylamino)-benzamid;
5-Brom-N-butoxy-3,4-difluor-2-(4-jod-2-methyl-phenylamino)-benzamid;
5-Brom-3,4-difluor-2-(4-jod-2-methyl-phenylamino)-N-(3-methyl-but-2-enyloxy)-benzamid;
5-Brom-3,4-difluor-2-(4-jod-2-methyl-phenylamino)-N-(3-methyl-pent-2-en-4-inyloxy)-benzamid;
5-Brom-3,4-difluor-2-(4-jod-2-methyl-benzylamino)-N-[5-(3-methoxy-phenyl)-3-methyl-pent-2-en-4-inyloxy]-benzamid;
5-Brom-3,4-difluor-2-(4-jod-2-methyl-phenylamino)-N-(prop-2-inyloxy)-benzamid;
5-Brom-3,4-difluor-2-(4-jod-2-methyl-phenylamino)-N-[3-(3-methoxy-phenyl)-prop-2-inyloxy]-benzamid;
5-Brom-3,4-difluor-2-(4-jod-2-methyl-phenylamino)-N-(thiophen-2-ylmethoxy)-benzamid;
5-Brom-3,4-difluor-2-(4-jod-2-methyl-phenylamino)-N-(pyridin-3-ylmethoxy)-benzamid;
5-Brom-3,4-difluor-2-(4-jod-2-methyl-phenylamino)-N-(3-(2-fluorphenyl)-prop-2-inyloxy)-benzamid;
5-Brom-3,4-difluor-2-(4-jod-2-methyl-phenylamino)-N-(ethoxy)-benzamid;
5-Brom-3,4-difluor-2-(4-jod-2-methyl-phenylamino)-N-(cyclopropylmethoxy)-benzamid;
5-Brom-3,4-difluor-2-(4-jod-2-methyl-phenylamino)-N-(isopropoxy)-benzamid;
5-Brom-3,4-difluor-2-(4-jod-2-methyl-phenylamino)-N-(but-3-inyloxy)-benzamid;
5-Chlor-N-hydroxy-2-(4-jod-2-methyl-phenylamino)-benzamid;
5-Chlor-2-(4-jod-2-methyl-phenylamino)-N-(tetrahydro-pyran-2-yloxy)-benzamid;
5-Chlor-2-(4-jod-2-methyl-phenylamino)-N-methoxy-benzamid;
4-Brom-2-(4-jod-2-methyl-phenylamino)-N-phenylmethoxy-benzamid;
4-Fluor-2-(4-jod-2-methyl-phenylamino)-N-phenylmethoxy-benzamid;
5-Fluor-N-hydroxy-2-(4-jod-2-methyl-phenylamino)-benzamid;
5-Jod-2-(4 jod-2-methyl-phenylamino)-N-phenylmethoxy-benzamid;
5-Fluor-2-(4-jod-2-methyl-phenylamino)-N-(tetrahydropyran-2-yloxy)-benzamid;
3,4-Difluor-2-(4-brom-2-methyl-phenylamino)-N-(3-phenylprop-2-inyloxy)-benzamid;
3,4-Difluor-2-(4-brom-2-methyl-phenylamino)-N-(3-furylmethoxy)-benzamid;
3,4-Difluor-2-(4-brom-2-methyl-phenylamino)-N-(2-thienylmethoxy)-benzamid;
3,4-Difluor-2-(4-brom-2-methyl-phenylamino)-N-(but-3-inyloxy)-benzamid;
3,4-Difluor-2-(4-brom-2-methyl-phenylamino)-N-(2-methyl-prop-2-enyloxy)-benzamid;
3,4-Difluor-2-(4-brom-2-methyl-phenylamino)-N-(but-2-enyloxy)-benzamid;
3,4-Difluor-2-(4-brom-2-methyl-phenylamino)-N-(methoxy)-benzamid;
3,4-Difluor-2-(4-brom-2-methyl-phenylamino)-N-(ethoxy)-benzamid;
3,4-Difluor-2-(4-brom-2-methyl-phenylamino)-N-(cyclobutoxy)-benzamid;
3,4-Difluor-2-(4-brom-2-methyl-phenylamino)-N-(isopropoxy)-benzamid;
3,4-Difluor-2-(4-brom-2-methyl-phenylamino)-N-(2-phenoxyethoxy)-benzamid;
3,4-Difluor-2-(4-brom-2-methyl-phenylamino)-N-(cyclopropylmethoxy)-benzamid;
3,4-Difluor-2-(4-brom-2-methyl-phenylamino)-N-(n-propoxy)-benzamid;
3,4-Difluor-2-(4-brom-2-methyl-phenylamino)-N-(1-methyl-prop-2-inyloxy)-benzamid;
3,4-Difluor-2-(4-brom-2-methyl-phenylamino)-N-(3-(3-fluorphenyl)-prop-2-inyloxy)-benzamid;
3,4-Difluor-2-(4-brom-2-methyl-phenylamino)-N-(4,4-dimethylpent-2-inyloxy)-benzamid;
3,4-Difluor-2-(4-brom-2-methyl-phenylamino)-N-(cyclopentoxy)-benzamid;
3,4,5-Trifluor-N-hydroxy-2-(4-jod-2-methyl-phenylamino)-benzamid;
5-Chlor-3,4-difluor-N-hydroxy-2-(4-jod-2-methyl-phenylamino)-benzamid;
5-Brom-3,4-difluor-2-(2-fluor-4-jod-phenylamino)-N-hydroxy-benzamid;
N-Hydroxy-2-(4-jod-2-methyl-phenylamino)-4-nitro-benzamid;
3,4,5-Trifluor-2-(2-fluor-4-jod-phenylamino)-N-hydroxy-benzamid;
5-Chlor-3,4-difluor-2-(2-fluor-4-jod-phenylamino)-N-hydroxy-benzamid;
5-Brom-2-(2-chlor-4-jod-phenylamino)-3,4-difluor-N-hydroxy-benzamid;
2-(2-Fluor-4-jod-phenylamino)-N-hydroxy-4-nitro-benzamid;
2-(2-Chlor-4-jod-phenylamino)-3,4,5-trifluor-N-hydroxy-benzamid;
5-Chlor-2-(2-chlor-4-jod-phenylamino)-3,4-difluor-N-hydroxy-benzamid;
5-Brom-2-(2-brom-4-jod-phenylamino)-3,4-difluor-N-hydroxy-benzamid;
2-(2-Chlor-4-jod-phenylamino)-N-hydroxy-4-methyl-benzamid;
2-(2-Brom-4-jod-phenylamino)-3,4,5-trifluor-N-hydroxy-benzamid;
2-(2-Brom-4-jod-phenylamino)-5-chlor-3,4-difluor-N-hydroxy-benzamid;
2-(2-Brom-4-jod-phenylamino)-N-hydroxy-4-nitro-benzamid;
4-Fluor-2-(2-fluor-4-jod-phenylamino)-N-hydroxy-benzamid;
3,4-Difluor-2-(2-fluor-4-jod-phenylamino)-N-hydroxy-benzamid;
2-(2-Chlor-4-jod-phenylamino)-4-fluor-N-hydroxy-benzamid;
2-(2-Chlor-4-jod-phenylamino)-3,4-difluor-N-hydroxy-benzamid;
2-(2-Brom-4-jod-phenylamino)-4-fluor-N-hydroxy-benzamid;
2-(2-Brom-4-jod-phenylamino)-3,4-difluor-N-hydroxy-benzamid;
N-Cyclopropylmethoxy-3,4,5-trifluor-2-(4-jod-2-methyl-phenylamino)-benzamid;
5-Chlor-N-cyclopropylmethoxy-3,4-difluor-2-(4-jod-2-methyl-phenylamino)-benzamid;
5-Brom-N-cyclopropylmethoxy-3,4-difluor-2-(2-fluor-4-jod-phenylamino)-benzamid;
N-Cyclopropylmethoxy-2-(4-jod-2-methyl-phenylamino)-4-nitro-benzamid;
N-Cyclopropylmethoxy-3,4,5-trifluor-2-(2-fluor-4-jod-phenylamino)-benzamid;
5-Chlor-N-cyclopropylmethoxy-3,4-difluor-2-(2-fluor-4-jod-phenylamino)-benzamid;
5-Brom-2-(2-chlor-4-jod-phenylamino)-N-cyclopropylmethoxy-3,4-difluor-benzamid;
N-Cyclopropylmethoxy-2-(2-fluor-4-jod-phenylamino)-4-nitro-benzamid;
2-(2-Chlor-4-jod-phenylamino)-N-cyclopropylmethoxy-3,4,5-trifluor-benzamid;
5-Chlor-2-(2-chlor-4-jod-phenylamino)-N-cyclopropylmethoxy-3,4-difluor-benzamid;
5-Brom-2-(2-brom-4-jod-phenylamino)-N-ethoxy-3,4-difluor-benzamid;
2-(2-Chlor-4-jod-phenylamino)-N-ethoxy-4-nitro-benzamid;
2-(2-Brom-4-jod-phenylamino)-N-cyclopropylmethoxy-3,4,5-trifluor-benzamid;
2-(2-Brom-4-jod-phenylamino)-5-chlor-N-cyclopropylmethoxy-3,4-difluor-benzamid;
2-(2-Brom-4-jod-phenylamino)-N-cyclopropylmethoxy-4-nitro-benzamid;
N-Cyclopropylmethoxy-4-fluor-2-(2-fluor-4-jod-phenylamino)-benzamid;
N-Cyclopropylmethoxy-3,4-difluor-2-(2-fluor-4-jod-phenylamino)-benzamid;
2-(2-Chlor-4-jod-phenylamino)-N-cyclopropylmethoxy-4-fluor-benzamid;
2-(2-Chlor-4-jod-phenylamino)-N-cyclopropylmethoxy-3,4-difluor-benzamid;
2-(2-Brom-4-jod-phenylamino)-N-cyclopropylmethoxy-4-fluor-benzamid und
2-(2-Brom-4 jod-phenylamino)-N-cyclopropylmethoxy-3,4-difluor-benzamid.

6. Kombination nach Anspruch 1, wobei der MEK-Inhibitor einen MEK1- oder MEK2-Inhibitor darstellt, der ausgewählt ist aus:
2-(2-Chlor-4-jod-phenylamino)-N-cyclopropylmethoxy-3,4-difluorbenzamid (PD184352);
2-(2-Methyl-4-jodphenylamino)-N-hydroxy-4-fluorbenzamid (PD170611);
2-(2-Methyl-4-jodphenylamino)-N-hydroxy-3,4-difluor-5-brombenzamid (PD171984);
2-(2-Methyl-4-jodphenylamino)-N-cyclopropylmethoxy-3,4-difluor-5-brombenzamid (PD177168);
2-(2-Methyl-4-jodphenylamino)-N-cyclobutylmethoxy-3,4-difluor-5-brombenzamid (PD 180841);
2-(2-Chlor-4-jodphenylamino)-N-cyclopropylmethoxy-3,4-difluor-5-brombenzamid (PD 184161);
2-(2-Chlor-4-jodphenylamino)-N-hydroxy-3,4-difluor-5-brombenzamid (PD184386);
2-(2-Chlor-4-jodphenylamino)-N-cyclobutylmethoxy-3,4-difluorbenzamid (PD185625);
2-(2-Chlor-4-jodphenylamino)-N-hydroxy-4-fluorbenzamid (PD185848);
2-(2-Methyl-4-jodphenylamino)-N-hydroxy-3,4-difluorbenzamid (PD188563);
2-(2-Methyl-4 jodphenylamino)-N-cyclopropylmethoxy-3,4,5-trifluorbenzamid (PD 198306) und
2-(2-Chlor-4-jodphenylamino)-N-cyclopropylmethoxy-4-fluorbenzamid (PD 203311).

7. Anti-Krebs-Kombination, enthaltend Paclitaxel und den MEK-Inhibitor 2-(2-Chlor-4-jodphenylamino)-N-cyclopropylmethoxy-3,4-difluorbenzamid (PD184352).

8. Verwendung einer einen Mitoseinhibitor und einen MEK-Inhibitor enthaltenden Anti-Krebs-Kombination nach den Ansprüchen 1 - 7 zur Herstellung von Arzneimitteln zur Behandlung von Krebs.

9. Verwendung nach Anspruch 8 zur Herstellung von nicht gleichzeitig zu verabreichenden Arzneimitteln.

10. Verwendung nach Anspruch 8, wobei der MEK-Inhibitor ein Phenylamin der Formel I darstellt.

11. Verwendung nach Anspruch 8, wobei der MEK-Inhibitor ein Phenylamin der Formel II darstellt.

12. Verwendung nach Anspruch 8, wobei der in Kombination mit einem Mitoseinhibitor verwendete MEK-Inhibitor einen selektiven MEK1- oder MEK2-Inhibitor darstellt.

13. Verwendung nach Anspruch 11, wobei der MEK-Inhibitor ausgewählt ist aus:
2-(2-Chlor-4 jod-phenylamino)-N-cyclopropylmethoxy-3,4-difluorbenzamid (PD184352);
2-(2-Methyl-4-jodphenylamino)-N-hydroxy-4-fluorbenzamid (PD170611);
2-(2-Methyl-4-jodphenylamino)-N-hydroxy-3,4-difluor-5-brombenzamid (PD171984);
2-(2-Methyl-4-jodphenylamino)-N-cyclopropylmethoxy-3,4-d ifluor-5-brombenzamid (PD177168);
2-(2-Methyl-4-jodphenylamino)-N-cyclobutylmethoxy-3,4-difluor-5-brombenzamid (PD 180841);
2-(2-Chlor-4-jodphenylamino)-N-cyclopropylmethoxy-3,4-difluor-5-brombenzamid (PD 184161);
2-(2-Chlor-4-jodphenylamino)-N-hydroxy-3,4-difluor-5-brombenzamid (PD184386);
2-(2-Chlor-4-jodphenylamino)-N-cyclobutylmethoxy-3,4-difluorbenzamid (PD185625);
2-(2-Chlor-4-jodphenylamino)-N-hydroxy-4-fluorbenzamid (PD185848);
2-(2-Methyl-4-jodphenylamino)-N-hydroxy-3,4-difluorbenzamid (PD188563);
2-(2-Methyl-4-jodphenylamino)-N-cyclopropylmethoxy-3,4,5-trifluorbenzamid (PD 198306) und
2-(2-Chlor-4-jodphenylamino)-N-cyclopropylmethoxy-4-fluorbenzamid (PD 203311).

14. Verwendung nach Anspruch 8, wobei der Mitoseinhibitor ausgewählt ist aus Paclitaxel, Docetaxel, Vincristin, Vinblastin, Vinorelbin und Vinflunin.

15. Verwendung nach Anspruch 14, wobei der Mitoseinhibitor Paclitaxel darstellt.

16. Verwendung nach Anspruch 14, wobei der Mitoseinhibitor Docetaxel darstellt.

17. Verwendung nach Anspruch 14, wobei der Mitoseinhibitor Vincristin darstellt.

18. Verwendung nach Anspruch 14, wobei der Mitoseinhibitor Vinblastin darstellt.

19. Verwendung nach Anspruch 14, wobei der Mitoseinhibitor Vinorelbin darstellt.

20. Verwendung nach Anspruch 14, wobei der Mitoseinhibitor Vinflunin darstellt.

21. Verwendung nach Anspruch 14, wobei der MEK-Inhibitor 2-(2-Chlor-4-jodphenylamino)-N-cyclopropylmethoxy-3,4-difluorbenzamid (PD184352) darstellt.

22. Verwendung nach Anspruch 13, wobei der Mitoseinhibitor Paclitaxel, Docetaxel oder Vincristin darstellt.

## Revendications

1. Combinaison anticancéreuse qui comprend un inhibiteur mitotique et un inhibiteur MEK, dans laquelle l'inhibiteur MEK représente un composé phénylamine de Formule I : 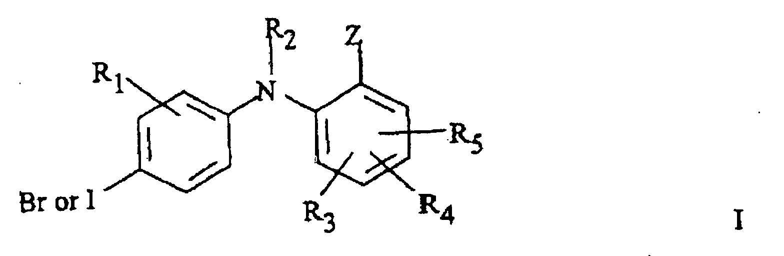 dans laquelle :
R₁ représente un atome d'hydrogène, un groupe hydroxy, un groupe alkyle en (C₁-C₈), un groupe alcoxy en (C₁-C₈), un groupe halogéno, un groupe trifluorométhyle, ou CN ;
R₂ représente un atome d'hydrogène ;
R₃, R₄ et R₅ représentent indépendamment un atome d'hydrogène, un groupe hydroxy, un groupe halogéno, un groupe trifluorométhyle, un groupe alkyle en (C₁-C₈), un groupe alcoxy (C₁-C₈), un groupe nitro, CN, ou -(O ou NH)ₘ-(CH₂)ₙ-R₉, où R₉ représente un atome d'hydrogène, un groupe hydroxy, COOH, ou NR₁₀R₁₁ ;
n est compris entre 0 et 4 ;
m vaut 0 ou 1 ;
R₁₀ et R₁₁ représentent indépendamment un atome d'hydrogène ou un groupe alkyle en (C₁-C₈), ou pris conjointement avec l'atome d'azote auquel ils sont attachés peuvent compléter un noyau cyclique de 3 à 10 membres contenant éventuellement 1, 2 ou 3 hétéroatomes supplémentaires choisis parmi O, S, NH, ou N-(alkyle en (C₁-C₈)) ;
Z représente COOR₇, un groupe tétrazolyle, CONR₆R₇, CONHNR₁₀R₁₁, ou CH₂OR₇ ;
R₆ et R₇ représentent indépendamment un atome d'hydrogène, un groupe alkyle en (C₁-C₈), un groupe alcényle en (C₂-C₈), un groupe alcynyle en (C₂-C₈), un groupe (CO)-(alkyle en (C₁-C₈)), un groupe aryle, un groupe hétéroaryle, un groupe cycloalkyle en (C₃-C₁₀) (ou un groupe cycloalkyle en (C₃-C₁₀) contenant éventuellement 1, 2 ou 3 hétéroatomes choisis parmi O, S, NH, ou un groupe N-alkyle) ; ou bien R₆ et R₇ conjointement avec l'atome d'azote auquel ils sont attachés complètent un noyau cyclique de 3 à 10 membres contenant éventuellement 1, 2 ou 3 hétéroatomes supplémentaires choisis parmi O, S, NH, ou un groupe N-alkyle ;
et dans laquelle l'un quelconque des groupes alkyle, alcényle, aryle, hétéroaryle, hétérocyclique et alcynyle précédents peut être non substitué ou substitué par un groupe halogéno, un groupe hydroxy, un groupe alcoxy en (C₁-C₆), un groupe amino, un groupe nitro, un groupe alkyle en (C₁-C₄)amino, un groupe di(alkyle en (C₁-C₄))amino, un groupe cycloalkyle en (C₃-C₆), un groupe phényle, un groupe phénoxy, un groupe hétéroaryle en (C₃-C₅) ou un radical hétérocyclique, ou bien un groupe hétéroaryloxy en (C₃-C₅) ou un radical-oxy hétérocyclique ;
ou un sel pharmaceutiquement acceptable de ceux-ci.

2. Combinaison selon la revendication 1, dans laquelle l'inhibiteur mitotique est choisi parmi le paclitaxel, le docétaxel, la vincristine, la vinblastine, la vinorelbine et la vinflunine.

3. Combinaison selon la revendication 1, dans laquelle l'inhibiteur MEK représente une phénylamine choisie parmi :
la [4-Chloro-2-(1H-tétrazol-5-yl)-phényl(4-iodo-2-méthyl-phényl)-amine ;
la (4-lodo-2-méthyl-phényl)- [2-(1H- tétrazol-5-yl)-phényl]amine ;
la [4-Nitro-2-(1H-tétrazol-5-yl)-phényl-(4-iodo-2-méthyl-phényl)-amine ;
l'Acide 4-fluoro-2-(4-iodo-2-méthylphénylamino)benzoïque ;
l'Acide 3,4,5-trifluoro-2-(4-iodo-2-méthyl-phénylamino)-benzoïque ;
l'Acide 3,4-difluoro-2-(4-iodo-2-méthyl-phénylamino)-benzoïque ;
l'Acide 5-bromo-3,4-difluoro-2-(4-iodo-2-méthyl-phénylamino)-benzoïque ;
l'Acide 5-chloro-2-(4-iodo-2-méthyl-phénylamino)-benzoïque ;
le 5-Chloro-2-(4-iodo-2-méthyl-phénylamino)-benzoate de sodium ;
l'Acide 5-bromo-2-(4-iodo-2-méthyl-phénylamino)-benzoïque ;
l'Acide 2-(4-iodo-2-méthyl-phénylamino)-5-nitro-benzoïque ;
l'Acide 4-chloro-2-(4-iodo-2-méthyl-phénylamino)-benzoïque ;
l'Acide 2-(4-iodo-2-méthyl-phénylamino)-benzoïque ;
l'Acide 5-fluoro-2-(4-iodo-2-méthyl-phénylamino)-benzoïque ;
l'Acide 5-iodo-2-(4-iodo-2-méthyl-phénylamino)-benzoïque ;
l'Acide 2,3,5-trifluoro-4-(4-iodo-2-méthyl-phénylamino)-benzoïque ;
l'Acide 2-(4-iodo-phénylamino)-5-méthoxy-benzoïque ;
l'Acide 5-méthyl-2-(4-iodo-2-méthyl-phénylamino)-benzoïque ;
l'Acide 2-(4-iodo-2-méthyl-phénylamino)-4-nitro-benzoïque ;
l'Acide 2-(4-bromo-2-méthyl-phénylamino)-4-fluoro-benzoïque ;
l'Acide 2-(2-bromo-4-iodo-phénylamino)-5-nitro-benzoïque ;
l'Acide 2-(4-bromo-2-méthyl-phénylamino)-3,4-difluoro-benzoïque ;
le 5-Chloro-N-(2-hydroxyéthyl)-2-(4-iodo-2-méthyl-phénylamino)-benzamide ;
le 4-Fluoro-2-(4-iodo-2-méthyl-phénylamino)-benzamide ;
le 4-Fluoro-2-(4-iodo-2-méthyl-phénylamino)-N-méthyl-benzamide ;
le N-Ethyl-4-fluoro-2-(4-iodo-2-méthyl-phénylamino)-benzamide ;
le 4-Fluoro-2-(4-iodo-2-méthyl-phénylamino)-N,N-diméthyl-benzamide ;
le 4-Fluoro-2-(4-iodo-2-méthyl-phénylamino)-N-(1H-tétrazol-5-yl)-benzamide ;
le 5-Bromo-2-(4-iodo-2-méthyl-phénylamino)-benzamide ;
le 5-Chloro-2-(4-iodo-2-méthyl-phénylamino)-N,N-diméthyl-benzamide ;
l'Acide [5-chloro-2-(4-iodo-2-méthyl-phénylamino)-benzoylamino]-acétique ;
le 4-Fluoro-2-(4-iodo-2-méthyl-phénylamino)-N-propyl-benzamide ;
le 5-Bromo-N-(2-hydroxy-éthyl)-2-(4-iodo-2-méthyl-phénylamino)-benzamide ;
le N,N-Diéthyl-4-fluoro-2-(4-iodo-2-méthyl-phénylamino)-benzamide ;
le 4-Fluoro-N-{3-[4-(2-hydroxy-éthyl)-pipérazin-1-yl]-prooyl}-2-(4-iodo-2-méthyl-phénylamino)-benzamide ;
le N,N-Diéthyl-2-(4-iodo-2-méthyl-phénylamino)-5-nitro-benzamide ;
le N-Butyl-4-fluoro-2-(4-iodo-2-méthyl-phénylamino)-benzamide ;
le 5-Chloro-N,N-diéthyl-2-(4-iodo-2-méthyl-phénylamino)-benzamide ;
le 5-Bromo-2-(4-iodo-2-méthyl-phénylamino)-N,N-diméthyl-benzamide ;
le 5-Bromo-3,4-difluoro-N-(2-hydroxy-éthyl)-2-(4-iodo-2-méthyl-phénylamino)-benzamide ;
le N-(2,3-Dihydroxy-propyl)-3,4-difluoro-2-(4-iodo-2-méthyl-phénylamino)-benzamide ;
le 5-Bromo-3,4-difluoro-2-(4-iodo-2-méthyl-phénylamino)-N-(2-pipéridin-1-yl-éthyl)-benzamide ;
le 3,4-Difluoro-N-(2-hydroxy-éthyl)-2-(4-iodo-2-méthyl-phénylamino)-benzamide ;
le N-(2,3-Dihydroxy-propyl)-4-fluoro-2-(4-iodo-2-méthyl-phénylamino)-benzamide ;
le 3,4-Difluoro-N-(3-hydroxy-propyl)-2-(4-iodo-2-méthyl-phénylamino)-benzamide ;
le 5-Bromo-3,4-difluoro-2-(4-iodo-2-méthyl-phénylamino)-N-(2-pyrrolidin-1-yl-éthyl)-benzamide ;
le 5-Bromo-3,4-difluoro-2-(4-iodo-2-méthyl-phénylamino)-N-(2-pyridin-4-yl-éthyl)-benzamide ;
le 4-Fluoro-N-(2-hydroxy-éthyl)-2-(4-iodo-2-méthyl-phénylamino)-benzamide ;
le 5-Bromo-N-(3-diméthylamino-propyl)-3,4-difluoro-2-(4-iodo-2-méthyl-phénylamino)-benzamide ;
le 5-Bromo-3,4-difluoro-2-(4-iodo-2-méthyl-phénylamino)-N-(2-morpholin-4-yl-éthyl)-benzamide ;
le 3,4-Difluoro-2-(4-iodo-2-méthyl-phénylamino)-N-(2-morpholin-4-yl-éthyl)-benzamide ;
le 3,4-Difluoro-2-(4-iodo-2-méthyl-phénylamino)-N-(2-pyrrolidin-1-yl-éthyl)-benzamide ;
le 3,4-Difluoro-2-(4-iodo-2-méthyl-phénylamino)-N-(2-pyridin-4-yl-éthyl)-benzamide ;
le N-(3-Diméthylamino-propyl)-3,4-difluoro-2-(4-iodo-2-méthyl-phénylamino)-benzamide ;
le N-Benzyl-4-fluoro-2-(4-iodo-2-méthyl-phénylamino)-benzamide ;
le 2-(4-Bromo-2-méthyl-phénylamino)-3,4-difluoro-N-(2-hydroxy-éthyl)-benzamide ;
le 4-Fluoro-2-(4-iodo-2-méthyl-phénylamino)-N-(2-morpholin-4-yl-éthyl)-benzamide ;
le 4-Fluoro-2-(4-iodo-2-méthyl-phénylamino)-N-(3-pipéridin-1-yl-propyl)-benzamide ;
le 3,4-Difluoro-2-(4-iodo-2-méthyl-phénylamino)-N-(3-pipéridin-1-yl-propyl)-benzamide ;
le 4-Fluoro-2-(4-iodo-2-méthyl-phénylamino)-N-(2-thiophén-2-yl-éthyl)-benzamide ;
le 4-Fluoro-2-(4-iodo-2-méthyl-phénylamino)-N-(2-pyrrolidin-1-yl-éthyl)-benzamide ;
le 2-(4-Bromo-2-méthyl-phénylamino)-3,4-difluoro-N-(2-morpholin-4-yl-éthyl)-benzamide ;
le 5-Bromo-3,4-difluoro-2-(4-iodo-2-méthyl-phénylamino)-N-pyridin-4-ylméthyl-benzamide ;
le 3,4-Difluoro-2-(4-iodo-2-méthyl-phénylamino)-N-pyridin-4-ylméthyl-benzamide ;
le 2-(4-Bromo-2-méthyl-phénylamino)-N-(3-diméthylaminopropyl)-3,4-difluoro-benzamide ;
le 4-Fluoro-2-(4-iodo-2-méthyl-phénylamino)-N-pyridin-4-ylméthyl-benzamide ;
le 4-Fluoro-2-(4-iodo-2-méthyl-phénylamino)-N-(2-pyridin-4-yl-éthyl)-benzamide ;
le 2-(4-Bromo-2-méthyl-phénylamino)-3,4-difluoro-N-(2-pyridin-4-yl-éthyl)-benzamide ;
le 2-(4-Bromo-2-méthyl-phénylamino)-3,4-difluoro-N-(3-hydroxy-propyl)-benzamide ;
le 2-(4-Bromo-2-méthyl-phénylamino)-3,4-difluoro-N-(2-pyrrolidin-1-yl-éthyl)-benzamide ;
le 4-Fluoro-2-(4-iodo-2-méthyl-phénylamino)-N-phénéthyl-benzamide ;
le 2-(4-Bromo-2-méthyl-phénylamino)-3,4-difluoro-N-(2-thiophén-2-yl-éthyl)-benzamide ;
le 2-(4-Bromo-2-méthyl-phénylamino)-3,4-difluoro-N-pyridin-4-ylméthyl-benzamide ;
le 2-(4-Bromo-2-méthyl-phénylamino)-3,4-difluoro-N-phénéthyl-benzamide ;
le 2-(4-Bromo-2-méthyl-phénylamino)-3,4-difluoro-N-(2-pipéridin-1-yl-éthyl)-benzamide ;
le 5-Chloro-N-{3-[4-(2-hydroxy-éthyl)-pipérazin-1-yl]-propyl}-2-(4-iodo-2-méthyl-phénylamino)-benzamide ;
le 5-Fluoro-N-{3-[4-(2-hydroxy-éthyl)-pipérazin-1-yl]-propyl}-2-(4-iodo-2-méthyl-phénylamino)-benzamide ;
le 2-(4-lodo-2-méthyl-phénylamino)-5-nitro-N-pyridin-4-ylméthyl-benzamide ;
le 5-Bromo-N-{3-[4-(2-hydroxy-éthyl)-pipérazin-1-yl]-propyl}-2-(4-iodo-2-méthyl-phénylamino)-benzamide ;
le 5-Chloro-N-(2-diéthylamino-éthyl)-2-(4-iodo-2-méthyl-phénylamino)-benzamide ;
le 5-Chloro-2-(4-iodo-2-méthyl-phénylamino)-N-(2-pipéridin-1-yl-éthyl)-benzamide ;
le (3-Hydroxy-pyrrolidin-1-yl)-[2-(4-iodo-2-méthyl-phénylamino)-5-nitro-phényle] ;
le 5-Chloro-2-(4-iodo-2-méthyl-phénylamino)-N-(2-pyrrolidin-1-yl-éthyl)-benzamide ;
le 5-Bromo-N-(2-diéthylamino-éthyl)-2-(4-iodo-2-méthyl-phénylamino)-benzamide ;
le N-{2-[Bis-(2-hydroxy-éthyl)-amino]-éthyl}-5-chloro-2-(4-iodo-2-méthyl-phénylamino)-benzamide ;
le N-{2-[Bis-(2-hydroxy-éthyl)-amino]-éthyl}-5-bromo-2-(4-iodo-2-méthyl-phénylamino)-benzamide ;
le N-{3-[4-(2-Hydroxy-éthyl)-pipérazin-1-yl]-propyl}-2-(4-iodo-2-méthyl-phénylamino)-benzamide ;
le 5-Fluoro-2-(4-iodo-2-méthyl-phénylamino)-N-pyridin-4-ylméthyl-benzamide ;
le 5-Bromo-2-(4-iodo-2-éthyl-phénylamino)-N-(2-pyrrolidin-1-yl-éthyl)-benzamide ;
le 5-Bromo-2-(4-iodo-2-méthyl-phénylamino)-N-(2-pipéridin-1-yl-éthyl)-benzamide ;
le 5-Fluoro-2-(4-iodo-2-méthyl-phénylamino)-N-(2-pyrrolidin-1-yl-éthyl)-benzamide ;
le 5-Chloro-N-(3-diméthylamino-propyl)-2-(4-iodo-2-méthyl-phénylamino)-benzamide ;
le N-{2-[Bis-(2-hydroxy-éthyl)-amino]-éthyl}-5-fluoro-2-(4-iodo-2-méthyl-phénylamino)-benzamide ;
le 5-Chloro-N-(3-hydroxy-propyl)-2-(4-iodo-2-méthyl-phénylamino)-benzamide ;
le 5-Chloro-N-(3-diéthylamino-2-hydroxy-propyl)-2-(4-iodo-2-méthyl-phénylamino)-benzamide ;
le 5-Fluoro-2-(4-iodo-2-méthyl-phénylamino)-N-(2-pipéridin-1-yl-éthyl)-benzamide ;
le 5-Bromo-N-(3-hydroxy-propyl)-2-(4-iodo-2-méthyl-phénylamino)-benzamide ;
le 5-Bromo-2-(4-iodo-2-méthyl-phénylamino)-N-(3-pipéridin-1-yl-propyl)-benzamide ;
le N-{2-[Bis-(2-hydroxy-éthyl)-amino]-éthyl)-2-(4-iodo-2-méthyl-phénylamino)-5-nitro-benzamide ;
le 5-Chloro-2-(4-iodo-2-méthyl-phénylamino)-N-(2-morpholin-4-yl-éthyl)-benzamide ;
le 5-Chloro-N-(3-diéthylamino-propyl)-2-(4-iodo-2-méthyl-phénylamino)-benzamide ;
le 5-Chloro-N-(2-diisopropylamino-éthyl)-2-(4-iodo-2-méthyl-phénylamino)-benzamide ;
le 5-Chloro-2-(4-iodo-2-méthyl-phénylamino)-N-(3-pipéridin-1-yl-propyl)-benzamide ;
le 2-(4-lodo-2-méthyl-phénylamino)-5-nitro-N-(2-pipéridin-1-yl-éthyl)-benzamide ;
le 5-Bromo-2-(4-iodo-2-méthyl-phénylamino)-N-(2-pipérazin-1-yl-éthyl)-benzamide ;
le N-(2-Diéthylamino-éthyl)-5-fluoro-2-(4-iodo-2-méthyl-phénylamino)-benzamide ;
le 5-Bromo-N-(3-diméthylamino-propyl)-2-(4-iodo-2-méthyl-phénylamino)-benzamide ;
le N-(3-Hydroxy-propyl)-2-(4-iodo-2-méthyl-phénylamino)-5-nitro-benzamide ;
le 5-Fluoro-N-(3-hydroxy-propyl)-2-(4-iodo-2-méthyl-phénylamino)-benzamide ;
le N-(3-Diéthylamino-propyl)-5-fluoro-2-(4-iodo-2-méthyl-phénylamino)-benzamide ;
le N-(3-Diéthylamino-propyl)-2-(4-iodo-2-méthyl-phénylamino)-5-nitro-benzamide ;
le 5-Bromo-2-(4-iodo-2-méthyl-phénylamino)-N-(2-morpholin-4-yl-éthyl)-benzamide ;
le 2-(4-Iodo-2-méthyl-phénylamino)-5-nitro-N-(3-pipéridin-1-yl-propyl)-benzamide ;
le [5-Fluoro-2-(4-iodo-2-méthyl-phénylamino)-phényl]-(3-hydroxy-pyrrolidin-1-yl)-méthanone ;
le 5-Bromo-N-(2-diisopropylamino-éthyl)-2-(4-iodo-2-méthyl-phénylamino)-benzamide ;
le 5-Fluoro-2-(4-iodo-2-méthyl-phénylamino)-N-(2-morpholin-4-yl-éthyl)-benzamide ;
le 5-Fluoro-2-(4-iodo-2-méthyl-phénylamino)-N-(3-pipéridin-1-yl-propyl)-benzamide ;
le [5-Fluoro-2-(4-iodo-2-méthyl-phénylamino)-phényl]-[4-(2-hydroxyéthyl)-pipérazin-1-yl-méthanone ;
le N-(3-Diéthylamino-2-hydroxy-propyl)-5-fluoro-2-(4-iodo-2-méthyl-phénylamino)-benzamide ;
le N-Cyclopropyl-5-fluoro-2-(4-iodo-2-méthyl-phénylamino)-benzamide ;
le 5-Chloro-N-(2-hydroxy-éthyl)-2-(4-iodo-2-méthyl-phénylamino)-benzamide ;
le 5-Fluoro-N-(2-hydroxy-éthyl)-2-(4-iodo-2-méthyl-phénylamino)-benzamide ;
le N-Benzyloxy-5-fluoro-2-(4-iodo-2-méthyl-phénylamino)-benzamide ;
le N-Benzyloxy-5-bromo-2-(4-iodo-2 -méthyl-phénylamino)-benzamide ;
le 2-(4-Iodo-2-méthyl-phénylamino)-5-nitro-N-(4-sulfamoyl-benzyl)-benzamide ;
le 5-Bromo-N-(2-hydroxy-éthyl)-2-(4-iodo-2-méthyl-phénylamino)-benzamide ;
le N-(2-Hydroxy-éthyl)-5-iodo-2-(4-iodo-2-méthyl-phénylamino)-benzamide ;
le N-(2 -Hydroxy-éthyl)-2-(4-iodo-2-éthyl-phénylamino)-5-nitro-benzamide ;
le 2-(4-lodo-2-méthyl-phénylamino)-N-méthyl-5-nitro-N-phényl-benzamide ;
le 5-Chloro-N-cyclopropyl-2-(4-iodo-2-méthyl-phénylamino)-benzamide ;
le 5-Fluoro-2-(4-iodo-2-méthyl-phénylamino)-N-méthyl-N-phényl-benzamide ;
le N-Allyl-5-fluoro-2-(4-iodo-2-méthyl-phénylamino)-benzamide;
le N-Benzyloxy-5-iodo-2-(4-iodo-2-méthyl-phénylamino)-benzamide ;
le 5-Fluoro-2-(4-iodo-2-méthyl-phénylamino)-N-(4-sulfamoyl-benzyl)-benzamide
le N-Allyl-5-chloro-2-(4-iodo-2-méthyl-phénylamino)-benzamide ;
le N-Cyclopropyl-2-(4-iodo-2-méthyl-phénylamino)-5-nitro-benzamide ;
le 5-Bromo-N-cyclopropyl-2-(4-iodo-2-méthyl-phénylamino)-benzamide ;
le 5-Chloro-2-(4-iodo-2-méthyl-phénylamino)-N-méthyl-N-phényl-benzamide ;
le 5-Iodo-2-(4-iodo-2-méthyl-phénylamino)-N-(4-sulfamoyl-benzyl)-benzamide ;
le 5-Bromo-2-(4-iodo-2-méthyl-phénylamino)-N-(4-sulfamoyl-benzyl)-benzamide ;
le N-Allyl-2-(4-iodo-2-méthyl-phénylamino)-5-nitro-benzamide ;
le 2-(4-Iodo-2-méthyl-phénylamino)-5-nitro-N-(4-sulfamoyl-benzyl)-benzamide ;
le N-Allyl-5-bromo-2-(4-iodo-2-méthyl-phénylamino)-benzamide ;
le 5-Fluoro-2-(4-iodo-2-méthyl-phénylamino)-N-(3-méthyl-benzyl)-benzamide ;
le N-Cyclopropyl-5-iodo-2-(4-iodo-2-méthyl-phénylamino)-benzamide ;
le 5-Bromo-2-(4-iodo-2-méthyl-phénylamino)-N-méthyl-N-phényl-benzamide ;
le N-Benzyloxy-2-(4-iodo-2-méthyl-phénylamino)-5-nitro-benzamide ;
le N-Cyclohexyl-5-iodo-2-(4-iodo-2-méthyl-phénylamino)-benzamide ;
le N-Allyl-5-iodo-2-(4-iodo-2-méthyl-phénylamino)-benzamide ;
le 5-lodo-2-(4-iodo-2-méthyl-phénylamino)-N-(3-méthyl-benzyl)-benzamide ;
le 2-(4-)odo-2-méthyl-phénylamino)-N-(3-méthy)-benzyl)-5-nitro-benzamide ;
le 5-lodo-2-(4-iodo-2-méthyl-phénylamino)-N-méthyl-N-phényl-benzamide ;
le N-Cyclohexyl-5-fluoro-2-(4-iodo-2-méthyl-phénylamino)-benzamide ;
le 5-Chloro-N-cyclohexyl-2-(4-iodo-2-méthyl-phénylamino)-benzamide ; le 5-Bromo-2-(4-iodo-2-méthyl-phénylamino)-N-(3-méthyl-benzyl)-benzamide ;
le 5-Bromo-N-cyclohexyl-2-(4-iodo-2-méthyl-phénylamino)-benzamide ; le 5-Chloro-2-(4-iodo-2-méthyl-phénylamino)-N-(3-méthyl-benzyl)-benzamide ;
le N-Cyclohexyl-2-(4-iodo-2-méthyl-phénylamino)-5-nitro-benzamide ;
le N-Benzyloxy-5-bromo-2-(4-iodo-2-méthyl-phénylamino)-benzamide ;
le N-Benzyloxy-5-fluoro-2-(4-iodo-2-méthyl-phénylamino)-benzamide ;
le 5-Chloro-N-(2-hydroxy-éthyl)-2-(4-iodo-2-méthyl-phénylamino)-benzamide ;
le 5-Bromo-N-(2-hydroxy-éthyl)-2-(4-iodo-2-méthyl-phénylamino)-benzamide ;
le 2-(4-lodo-2-méthyl-phénylamino)-N-méthyl-5-nitro-N-phényl-benzamide ;
le 5-Chloro-2-(4-iodo-2-méthyl-phénylamino)-N-méthyl-N-phényl-benzamide ;
le N-(2-Hydroxy-éthyl)-5-iodo-2-(4-iodo-2-méthyl-phénylamino)-benzamide ;
le 5-Chloro-N-cyclopropyl-2-(4-iodo-2-méthyl-phénylamino)-benzamide ;
le N-Allyl-5-chloro-2-(4-iodo-2-méthyl-phénylamino)-benzamide ;
le 5-Fluoro-2-(4-iodo-2-méthyl-phénylamino)-N-méthyl-N-phényl-benzamide ;
le N-(2-Hydroxy-éthyl)-2-(4-iodo-2-méthyl-phénylamino)-5-nitro-benzamide ;
le 5-Fluoro-N-(2-hydroxy-éthyl)-2-(4-iodo-2-méthyl-phénylamino)-benzamide ;
le 5-Bromo-N-cyclopropyl-2-(4-iodo-2-méthyl-phénylamino)-benzamide ;
le N-Cyclopropyl-5-fluoro-2-(4-iodo-2-méthyl-phénylamino)-benzamide ;
le 5-Fluoro-2-(4-iodo-2-méthyl-phénylamino)-N-(4-sulfamoyl-benzyl)-benzamide ;
le N-Cyclopropyl-2-(4-iodo-2-méthyl-phénylamino)-5-nitro-benzamide ;
le N-Allyl-5-fluoro-2-(4-iodo-2-méthyl-phénylamino)-benzamide ;
le N-Benzyloxy-5-iodo-2-(4-iodo-2-méthyl-phénylamino)-benzamide ;
le N-Allyl-5-bromo-2-(4-iodo-2-méthyl-phénylamino)-benzamide ;
le 5-Bromo-2-(4-iodo-2-méthyl-phénylamino)-N-(4-sulfamoyl-benzyl)-benzamide ;
le 5-Bromo-2-(4-iodo-2-méthyl-phénylamino)-N-méthyl-N-phényl-benzamide ;
le N-Allyl-2-(4-iodo-2-méthyl-phénylamino)-5-nitro-benzamide ;
l'Alcool 4-fluoro-2-(4-iodo-2-méthyl-phénylamino)-benzylique ;
le [5-Chloro-2-(4-iodo-2-méthyl-phénylamino)-phényl]-méthanol ;
le [2-(4-Iodo-2-méthyl-phénylamino)-5-nitro-phényl]-méthanol ;
le [5-Bromo-2-(4-iodo-2-méthyl-phénylamino)-phényl]-méthanol ; et
le N-Allyl-2-(4-iodo-2-méthyl-phénylamino)-5-nitro-benzamide.

4. Combinaison anticancéreuse qui comprend un inhibiteur mitotique et un inhibiteur MEK, dans laquelle l'inhibiteur MEK est un composé phénylamine de Formule II : 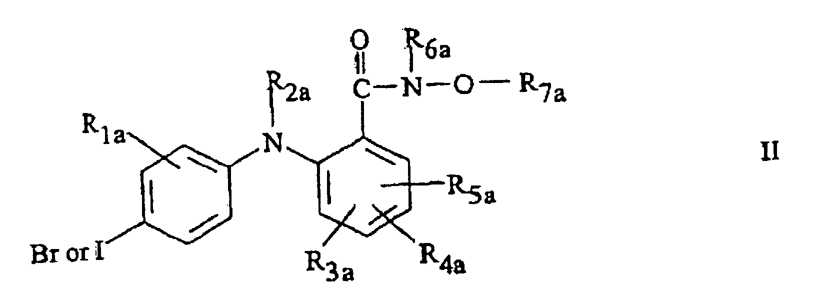 dans laquelle :
R₁ₐ représente un atome d'hydrogène, un groupe hydroxy, un groupe alkyle en (C₁-C₈), un groupe alcoxy en (C₁-C₈), un groupe halogéno, un groupe trifluorométhyle, ou CN ;
R₂ₐ représente un atome d'hydrogène ;
R₃ₐ, R₄ₐ, et R₅ₐ représentent indépendamment un atome d'hydrogène, un groupe hydroxy, un groupe halogéno, un groupe trifluorométhyle, un groupe alkyle en (C₁-C₈), un groupe alcoxy en (C₁-C₈), un groupe nitro, CN, ou (O ou NH)ₘ-(CH₂)ₙ-R₉ₐ,
où R₉ₐ représente un atome d'hydrogène, un groupe hydroxy, CO₂H ou NR₁₀ₐR₁₁ₐ.
n est compris entre 0 et 4 ;
m vaut 0 ou 1 ;
R₁₀ₐ et R₁₁ₐ représentent indépendamment un atome d'hydrogène ou un groupe alkyle en (C₁-C₈), ou bien pris conjointement avec l'atome d'azote auquel ils sont attachés peuvent compléter un noyau cyclique de 3 à 10 membres contenant, éventuellement, un, deux ou trois hétéroatomes supplémentaires choisis parmi O, S, NH, ou un bien groupe N-(alkyle en (C₁-C₈)) ;
R₆ₐ représente un atome d'hydrogène, un groupe alkyle en (C₁-C₈), un groupe (CO)-(alkyle en (C₁-C₈)), un groupe aryle, un groupe aralkyle, ou un groupe cycloalkyle en (C₃-C₁₀) ;
R₇ₐ représente un atome d'hydrogène, un groupe alkyle en (C₁-C₈), un groupe alcényle en (C₂-C₈), un groupe alcynyle en (C₂-C₈), un groupe cycloalkyle en (C₃-C₁₀) ou un groupe cycloalkyle en (C₃-C₁₀) (contenant, éventuellement, un hétéroatome choisi parmi O, S, ou NR₉ₐ) ;
et dans laquelle l'un quelconque des groupes alkyle, alcényle, aryle, hétéroaryle, hétérocyclique et alcynyle précédents peut être non substitué ou substitué par un groupe halogéno, un groupe hydroxy, un groupe alcoxy en (C₁-C₆), un groupe amino, un groupe nitro, un groupe (alkyle en (C₁-C₄))amino, un groupe di(alkyle en (C₁-C₄))amino, un groupe cycloalkyle en (C₃-C₆), un groupe phényle, un groupe phénoxy, un groupe hétéroaryle en (C₃-C₅) ou un radical hétérocyclique, ou bien un groupe hétéroaryloxy en (C₃-C₅) ou un oxy-radical hétérocyclique ; ou R₆ₐ et R₇ₐ pris conjointement avec l'atome d'azote auquel ils sont attachés peuvent compléter un noyau cyclique de 5 à 10 membres contenant, éventuellement, un, deux ou trois hétéroatomes supplémentaires choisis parmi O, S, ou NR₁₀ₐR₁₁ₐ ; ou un sel pharmaceutiquement acceptable de ceux-ci.

5. Combinaison anticancéreuse comprenant un inhibiteur mitotique et un inhibiteur MEK 1 ou MEK 2 sélectif choisi parmi :
le 4-Fluoro-N-hydroxy-2-(4-iodo-2-méthyl-phénylamino)-benzamide ;
le 4-Fluoro-2-(4-iodo-2-méthyl-phénylamino)-N-(méthoxy)-benzamide ;
le 4-Fluoro-2-(4-iodo-2-méthyl-phénylamino)-N-(prop-2-ynyloxy)-benzamide ;
le 4-Fluoro-2-(4-iodo-2-méthyl-phénylamino)-N-(2-phénoxyéthoxy)-benzamide ;
le 4-Fluoro-2-(4-iodo-2-méthyl-phénylamino)-N-(2-thiénylméthoxy)-benzamide ;
le 4-Fluoro-2-(4-iodo-2-méthyl-phénylamino)-N-(prop-2-ényloxy)-benzamide ;
le 4-Fluoro-2-(4-iodo-2-méthyl-phénylamino)-N-(cyclopropylméthoxy-benzamide ;
le 4-Fluoro-2-(4-iodo-2-méthyl-phénylamino)-N-(cyclopentoxy)-benzamide ;
le 3,4-Difluoro-2-(4-iodo-2-méthyl-phénylamino)-N-(3-furylméthoxy)-benzamide ;
le 3,4-Difluoro-2-(4-iodo-2-méthyl-phénylamino)-N-éthoxy-benzamide ;
le 3,4-Difluoro-2-(4-iodo-2-méthyl-phénylamino)-N-(but-2-ényloxy)-benzamide ;
le 3,4-Difluoro-2-(4-iodo-2-méthyl-phénylamino)-N-(cyclopropylméthoxy)-benzamide ;
le 3,4-Difluoro-2-(4-iodo-2-méthyl-phénylamino)-N-(1-méthylprop-2-ynyloxy)-benzamide ;
le 3,4-Difluoro-2-(4-iodo-2-méthyl-phénylamino)-N-(3-phénylprop-2-ynyloxy)-benzamide ;
le 3,4-Difluoro-2-(4-iodo-2- méthyl-phénylamino)-N-(3-méthyl-5-phénylpent-2-én-4-ynyloxy)-benzamide ;
le 3,4-Difluoro-2-(4-iodo-2-méthyl-phénylamino)-N-(prop-2-ynyloxy)-benzamide ;
le 3,4-Difluoro-2-(4-iodo-2-méthyl-phénylamino)-N-(propoxy)-benzamide ;
le 3,4-Difluoro-2-(4-iodo-2-méthyl-phénylamino)-N-(cyclobutyloxy)-benzamide ;
le 3,4-Difluoro-2-(4-iodo-2-méthyl-phénylamino)-N-(2-thiénylméthoxy)-benzamide ;
le 3,4-Difluoro-2-(4-iodo-2-méthyl-phénylamino)-N-(2-méthyl-prop-2-ényloxy)-benzamide ;
le 3,4-Difluoro-2-(4-iodo-2-méthyl-phénylamino)-N-(2-phénoxyéthoxy)-benzamide ;
le 3,4-Difluoro-2-(4-iodo-2-méthyl-phénylamino)-N-(but-2-ényloxy)-benzamide ;
le 3,4-Difluoro-2-(4-iodo-2-méthyl-phénylamino)-N-(but-3-ynyloxy)-benzamide ;
le 3,4-Difluoro-2-(4-iodo-2-méthyl-phénylamino)-N-(cyclopentyloxy)-benzamide ;
le 3,4-Difluoro-2-(4-iodo-2-méthyl-phénylamino)-N-(3-(2-fluorophényl)-prop-2-ynyloxy)-benzamide ;
le 5-Bromo-3,4-difluoro-N-hydroxy-2-(4-iodo-2-méthyl-phénylamino)-benzamide ;
le 5-Bromo-3,4-difluoro-2-(4-iodo-2-méthyl-phénylamino)-N-(n-propoxy)-benzamide ;
le 5-Bromo-3,4-difluoro-N-(furan-3-ylméthoxy)-2-(4-iodo-2-méthyl-phénylamino)-benzamide ;
le 5-Bromo-N-(but-2-ényloxy)-3,4-difluoro-2-(4-iodo-2-méthyl-phénylamino)-benzamide ;
le 5-Bromo-N-butoxy-3,4-difluoro-2-(4-iodo-2-méthyl-phénylamino)-benzamide ;
le 5-Bromo-3,4-difluoro-2-(4-iodo-2-méthyl-phénylamino)-N-(3-méthyl-but-2-ényloxy)-benzamide ;
le 5-Bromo-3,4-difluoro-2-(4-iodo-2-méthyl-phénylamino)-N-(3-méthyl-pent-2-én-4-ynyloxy)-benzamide ;
le 5-Bromo-3,4-difluoro-2-(4-iodo-2-méthyl-benzyl)-N-[5-(3-méthoxy-phényl)-3-méthyl-pent-2-én-4-ynyloxy]-benzamide ;
le 5-Bromo-3,4-difluoro-2-(4-iodo-2-méthyl-phénylamino)-N-prop-2-ynyloxy)-benzamide ;
le 5-Bromo-3,4-difluoro-2-(4-iodo-2-méthyl-phénylamino)-N-[3-(3-méthoxy-phényl)-prop-2-ynyloxy]-benzamide ;
le 5-Bromo-3,4-difluoro-2-(4-iodo-2-méthyl-phénylamino)-N-(thiopen-2-ylméthoxy)-benzamide ;
le 5-Bromo-3,4-difluoro-2-(4-iodo-2-méthyl-phénylamino)-N-(pyridin-3-ylméthoxy)-benzamide ;
le 5-Bromo-3-4-difluoro-2-(4-iodo-2-méthyl-phénylamino)-N-(3-(2-fluorophényl)-prop-2-ynyloxy)-benzamide ;
le 5-Bromo-3,4-difluoro-2-(4-iodo-2-méthyl-phénylamino)-N-(éthoxy)-benzamide ;
le 5-Bromo-3,4-difluoro-2-(4-iodo-2-méthyl-phénylamino)-N-(cyclopropylméthoxy)-benzamide ;
le 5-Bromo-3,4-difluoro-2-(4-iodo-2-méthyl-phénylamino)-N-(isopropoxy)-benzamide ;
le 5-Bromo-3,4-difluoro-2-(4-iodo-2-méthyl-phénylamino)-N-(but-3-ynyloxy)-benzamide ;
le 5-Chloro-N-hydroxy-2-(4-iodo-2-méthyl-phénylamino)-benzamide ;
le 5-Chloro-2-(4-iodo-2-méthyl-phénylamino)-N-(tétrahydro-pyran-2-yloxy)-benzamide ;
le 5-Chloro-2-(4-iodo-2-méthyl-phénylamino)-N-méthoxy-benzamide ;
le 4-Bromo-2-(4-iodo-2-méthyl-phénylamino)-N-phénylméthoxy-benzamide ;
le 4-Fluoro-2-(4-iodo-2-méthyl-phénylamino)-N-phénylméthoxy-benzamide ;
le 5-Fluoro-N-hydroxy-2-(4-iodo-2-méthyl-phénylamino)-benzamide ;
le 5-lodo-2-(4-iodo-2-méthyl-phénylamino)-N-phényl méthoxy-benzamide ;
le 5-Fluoro-2-(4-iodo-2-méthyl-phénylamino)-N-(tétrahydropyran-2-yloxy)-benzamide ;
le 3,4-Difluoro-2-(4-bromo-2-méthyl-phénylamino)-N-(3-phénylprop-2-ynyloxy)-benzamide ;
le 3,4-Difluoro-2-(4-bromo-2-méthyl-phénylamino)-N-(3-furylméthoxy)-benzamide ;
le 3,4-Difluoro-2-(4-bromo-2-méthyl-phénylamino)-N-(2-thiénylméthoxy)-benzamide ;
le 3,4-Difluoro-2-(4-bromo-2-méthyl-phénylamino)-N-(but-3-ynyloxy)-benzamide ;
le 3,4-Difluoro-2-(4-bromo-2-méthyl-phénylamino)-N-(2-méthyl-prop-2-ényloxy)-benzamide ;
le 3,4-Difluoro-2-(4-bromo-2-méthyl-phénylamino)-N-(but-2-ényloxy)-benzamide ;
le 3,4-Difluoro-2-(4-bromo-2-méthyl-phénylamino)-N-(méthoxy)-benzamide ;
le 3,4-Difluoro-2-(4-bromo-2-méthyl-phénylamino)-N-(éthoxy)-benzamide ;
le 3,4-Difluoro-2-(4-bromo-2-méthyl-phénylamino)-N-(cyclobutoxy)-benzamide ;
le 3,4-Difluoro-2-(4-bromo-2-méthyl-phénylamino)-N-(isopropoxy)-benzamide ;
le 3,4-Difluoro-2-(4-bromo-2-méthyl-phénylamino)-N-(2-phénoxyéthoxy)-benzamide ;
le 3,4-Difluoro-2-(4-bromo-2-méthyl-phénylamino)-N-(cyclopropyl-méthoxy)-benzamide ;
le 3,4-Difluoro-2-(4-bromo-2-méthyl-phénylamino)-N-(n-propoxy)-benzamide ;
le 3,4-Difluoro-2-(4-bromo-2-méthyl-phénylamino)-N-(1-méthyl-prop-2-ynyloxy)-benzamide ;
le 3,4-Difluoro-2-(4-bromo-2-méthyl-phénylamino)-N-(3-(3-fluorophényl)-prop-2-ynyloxy)-benzamide ;
le 3,4-Difluoro-2-(4-bromo-2-méthyl-phénylamino)-N-(4,4-diméthylpent-2-ynyloxy)-benzamide ;
le 3,4-Difluoro-2-(4-bromo-2-méthyl-phénylamino)-N-(cyclopentoxy)-benzamide ;
le 3,4,5-Trifluoro-N-hydroxy-2-(4-iodo-2-méthyl-phénylamino)-benzamide ;
le 5-Chloro-3,4-difluoro-N-hydroxy-2-(4-iodo-2-méthyl-phénylamino)-benzamide ;
le 5-Bromo-3,4-difluoro-2-(2-fluoro-4-iodo-phénylamino)-N-hydroxy-benzamide ;
le N-Hydroxy-2-(4-iodo-2-méthyl-phénylamino)-4-nitro-benzamide ;
le 3,4,5-Trifluoro-2-(2-fluoro-4-iodo-phénylamino)-N-hydroxy-benzamide ;
le 5-Chloro-3,4-difluoro-2-(2-fluoro-4-iodo-phénylamino)-N-hydroxy-benzamide ;
le 5-Bromo-2-(2-chloro-4-iodo-phénylamino)-3,4-difluoro-N-hydroxy-benzamide ;
le 2-(2-Fluoro-4-iodo-phénylamino)-N-hydroxy-4-nitro-benzamide ;
le 2-(2-Chloro-4-iodo-phénylamino)-3,4,5-trifluoro-N-hydroxy-benzamide ;
le 5-Chloro-2-(2-chloro-4-iodo-phénylamino)-3,4-difluoro-N-hydroxy-benzamide ;
le 5-Bromo-2-(2-bromo-4-iodo-phénylamino)-3,4-difluoro-N-hydroxy-benzamide ;
le 2-(2-Chloro-4-iodo-phénylamino)-N-hydroxy-4-méthyl-benzamide ;
le 2-(2-Bromo-4-iodo-phénylamino)-3,4,5-trifluoro-N-hydroxy-benzamide ;
le 2-(2-Bromo-4-iodo-phénylamino)-5-chloro-3,4-difluoro-N-hydroxy-benzamide ;
le 2-(2-Bromo-4-iodo-phénylamino)-N-hydroxy-4-nitro-benzamide ;
le 4-Fluoro-2-(2-fluoro-4-iodo-phénylamino)-N-hydroxy-benzamide ;
le 3,4-Difluoro-2-(2-fluoro-4-iodo-phénylamino)-N-hydroxy-benzamide ;
le 2-(2-Chloro-4-iodo-phénylamino)-4-fluoro-N-hydroxy-benzamide ;
le 2-(2-Chloro-4-iodo-phénylamino)-3,4-difluoro-N-hydroxy-benzamide ;
le 2-(2-Bromo-4-iodo-phénylamino)-4-fluoro-N-hydroxy-benzamide ;
le 2-(2-Bromo-4-iodo-phénylamino)-3,4-difluoro-N-hydroxy-benzamide ;
le N-Cyclopropylméthoxy-3,4,5-trifluoro-2-(4-iodo-2-méthyl-phénylamino)-benzamide ;
le 5-Chloro-N-cyclopropylméthoxy-3,4-difluoro-2-(4-iodo-2-méthyl-phénylamino)-benzamide ;
le 5-Bromo-N-cyclopropylméthoxy-3,4-difluoro-2-(2-fluoro-4-iodo-phénylamino)-benzamide ;
le N-Cyclopropylméthoxy-2-(4-iodo-2-méthyl-phénylamino)-4-nitro-benzamide ;
le N-Cyclopropylméthoxy-3,4,5-trifluoro-2-(2-fluoro-4-iodo-phénylamino)-benzamide ;
le 5-Chloro-N-cyclopropylméthoxy-3,4-difluoro-2-(2-fluoro-4-iodo-phénylamino)-benzamide ;
le 5-Bromo-2-(2-chloro-4-iodo-phénylamino)-N-cyclopropylméthoxy-3,4-difluoro-benzamide ;
le N-Cyclopropylméthoxy-2-(2-fluoro-4-iodo-phénylamino)-4-nitro-benzamide ;
le 2-(2-Chloro-4-iodo-phénylamino)-N-cyclopropylméthoxy-3,4,5-trifluoro-benzamide ;
le 5-Chloro-2-(2-chloro-4-iodo-phénylamino)-N-cyclopropylméthoxy-3,4-difluoro-benzamide ;
le 5-Bromo-2-(2-bromo-4-iodo-phénylamino)-N-éthoxy-3,4-difluoro-benzamide ;
le 2-(2-Chloro-4-iodo-phénylamino)-N-éthoxy-4-nitro-benzamide ;
le 2-(2-Bromo-4-iodo-phénylamino)-N-cyclopropylméthoxy-3,4,5-trifluoro-benzamide ;
le 2-(2-Bromo-4-iodo-phénylamino)-5-chloro-N-cyclopropylméthoxy-3,4-difluoro-benzamide ;
le 2-(2-Bromo-4-iodo-phénylamino)-N-cyclopropylméthoxy-4-nitro-benzamide ;
le N-Cyclopropylméthoxy-4-fluoro-2-(2-fluoro-4-iodo-phénylamino)-benzamide ;
le N-Cyclopropylméthoxy-3,4-difluoro-2-(2-fluoro-4-iodo-phénylamino)-benzamide ;
le 2-(2-Chloro-4-iodo-phénylamino)-N-cyclopropylméthoxy-4-fluoro-benzamide ;
le 2-(2-Chloro-4-iodo-phénylamino)-N-cyclopropylméthoxy-3,4-difluoro-benzamide ;
le 2-(2-Bromo-4-iodo-phénylamino)-N-cyclopropylméthoxy-4-fluoro-benzamide ; ou
le 2-(2-Bromo-4-iodo-phénylamino)-N-cyclopropylméthoxy-3,4-difluoro-benzamide.

6. Combinaison selon la revendication 1, dans laquelle l'inhibiteur MEK est un inhibiteur MEK1 ou MEK2 choisi parmi :
le 2-(2-chloro-4-iodophénylamino)-N-cyclopropylméthoxy-3,4-difluoro-benzamide (PD 184352) ;
le 2-(2-méthyl-4-iodophénylamino)-N-hydroxy-4-fluorobenzamide (PD 170611) ;
le 2-(2-méthyl-4-iodophénylamino)-N-hydroxy-3,4-difluoro-5-bromobenzamide (PD 171984) ;
le 2-(2-méthyl-4-iodophénylamino)-N-cyclopropylméthoxy-3,4-difluoro-5-bromobenzamide (PD 177168) ;
le 2-(2-méthyl-4-iodophénylamino)-N-cyclobutylméthoxy-3,4-difluoro-5-bromobenzamide (PD 180841) ;
le 2-(2-chloro-4-iodophénylamino)-N-cyclopropylméthoxy-3,4-difluoro-5-bromobenzamide (PD 184161) ;
le 2-(2-chloro-4-iodophénylamino)-N-hydroxy-3,4-difluoro-5-bromo-benzamide (PD 184386) ;
le 2-(2-chloro-4-iodophénylamino)-N-cyclobutylméthoxy-3,4-difluoro-benzamide (PD 185625) ;
le 2-(2-chloro-4-iodophénylamino)-N-hydroxy-4-fluorobenzamide (PD 185848) ;
le 2-(2-méthyl-4-iodophénylamino)-N-hydroxy-3,4-difluorobenzamide (PD 188563) ;
le 2-(2-méthyl-4-iodophénylamino)-N-cyclopropylméthoxy-3,4,5-trifluoro-benzamide (PD 198306) ; et
le 2-(2-chloro-4-iodophénylamino)-N-cyclopropylméthoxy-4-fluoro-benzamide (PD 203311).

7. Combinaison anticancéreuse comprenant le paclitaxel et l'inhibiteur MEK 2-(2-chloro-4-iodophénylamino)-N-cyclopropylméthoxy-3,4-difluorobenzamide (PD 184352).

8. Utilisation d'une combinaison anticancéreuse comprenant un inhibiteur mitotique et un inhibiteur MEK selon les revendications 1 à 7 pour la fabrication de produits pharmaceutiques destinés au traitement d'un cancer.

9. Utilisation selon la revendication 8, pour la fabrication de deux produits pharmaceutiques ne pouvant pas être administrés simultanément.

10. Utilisation selon la revendication 8, dans laquelle l'inhibiteur MEK est une phénylamine de Formule I.

11. Utilisation selon la revendication 8, dans laquelle l'inhibiteur MEK est une phénylamine de Formule II.

12. Utilisation selon la revendication 8, dans laquelle l'inhibiteur MEK utilisé en combinaison avec un inhibiteur mitotique est un inhibiteur MEK 1 ou MEK 2 sélectif.

13. Utilisation selon la revendication 11, dans laquelle l'inhibiteur MEK est un composé choisi parmi :
le 2-(2-chloro-4-iodophénylamino)-N-cyclopropylméthoxy-3,4-difluoro-benzamide (PD 184352) ;
le 2-(2-méthyl-4-iodophénylamino)-N-hydroxy-4-fluorobenzamide (PD 170611);
le 2-(2-méthyl-4-iodophénylamino)-N-hydroxy-3,4-difluoro-5-bromo-benzamide (PD 171984) ;
le 2-(2-méthyl-4-iodophénylamino)-N-cyclopropylméthoxy-3,4-difluoro-5-bromobenzamide (PD 177168) ;
le 2-(2-méthyl-4-iodophénylamino)-N-cyclobutylméthoxy-3,4-difluoro-5-bromobenzamide (PD 180841) ;
le 2-(2-chloro-4-iodophénylamino)-N-cyclopropylméthoxy-3,4-difluoro-5-bromobenzamide (PD 184161) ;
le 2-(2-chloro-4-iodophénylamino)-N-hydroxy-3,4-difluoro-5-bromo-benzamide (PD 184386) ;
le 2-(2-chloro-4-iodophénylamino)-N-cyclobutylméthoxy-3,4-difluoro-benzamide (PD 185625) ;
le 2-(2-chloro-4-iodophénylamino)-N-hydroxy-4-fluorobenzamide (PD 185848) ;
le 2-(2-méthyl-4-iodophénylamino)-N-hydroxy-3,4-difluorobenzamide (PD 188563) ;
le 2-(2-méthyl-4-iodophénylamino)-N-cyclopropylméthoxy-3,4,5-trifluoro-benzamide(PD 198306) ; et
le 2-(2-chloro-4-iodophénylamino)-N-cyclopropylméthoxy-4-fluoro-benzamide (PD 203311).

14. Utilisation selon la revendication 8, dans laquelle l'inhibiteur mitotique est choisi parmi le paclitaxel, le docétaxel, la vincristine, la vinblastine, la vinorelbine et la vinflunine.

15. Utilisation selon la revendication 14, dans laquelle l'inhibiteur mitotique est le paclitaxel.

16. Utilisation selon la revendication 14, dans laquelle l'inhibiteur mitotique est le docétaxel.

17. Utilisation selon la revendication 14, dans laquelle l'inhibiteur mitotique est la vincristine.

18. Utilisation selon la revendication 14, dans laquelle l'inhibiteur mitotique est la vinblastine.

19. Utilisation selon la revendication 14, dans laquelle l'inhibiteur mitotique est la vinorelbine.

20. Utilisation selon la revendication 14, dans laquelle l'inhibiteur mitotique est la vinflunine.

21. Utilisation selon la revendication 14, dans laquelle l'inhibiteur MEK est le 2-(2-chloro-4-iodophénylamino)-N-cyclopropylméthoxy-3,4-difluorobenzamide (PD 184352).

22. Utilisation selon la revendication 13, dans laquelle l'inhibiteur mitotique est le paclitaxel, le docétaxel ou la vincristine.
